# EUROPEAN PATENT SPECIFICATION

(11) **EP 4 157 834 B1**
(45) Date of publication and mention of the grant of the patent: **03.07.2024**
(21) Application number: 21732200.7
(22) Date of filing: 27.05.2021
(51) Int. Cl.: C07D 413/12, A61K 31/422, A61K 31/4245, A61K 31/4025, A61P 35/00, A61P 25/00, A61P 25/28, A61P 3/00, A61P 3/10, A61P 9/00, A61P 9/10, A61P 11/00

(54) **N-(1-CYANO-PYRROLIDIN-3-YL)-5-(3-(TRIFLUOROMETHYL)PHENYL)OXAZOLE-2-CARBOXAMIDE DERIVATIVES AND THE CORRESPONDING OXADIAZOLE DERIVATIVES AS USP30 INHIBITORS FOR THE TREATMENT OF MITOCHONDRIAL DYSFUNCTION**
N-(1-CYANO-PYRROLIDIN-3-YL)-5-(3-(TRIFLUOROMETHYL)PHENYL)OXAZOLE-2-CARBOXAMID-DERIVATE UND DIE ENTSPRECHENDEN OXADIAZOL-DERIVATE ALS USP30 INHIBITOREN ZUR BEHANDLUNG VON MITOCHONDRIALER DYSFUNKTION
DÉRIVÉS DE N-(1-CYANO-PYRROLIDIN-3-YL)-5-(3-(TRIFLUOROMÉTHYL)PHÉNYL)OXAZOLE-2-CARBOXAMIDE ET LES DÉRIVÉS DE OXADIAZOLE CORRESPONDANTS EN TANT QU'INHIBITEURS D'USP30 POUR LE TRAITEMENT DE DYSFONCTIONNEMENT MITOCHONDRIAL

(30) Priority: 28.05.2020 GB 202008051; 21.10.2020 GB 202016709
(43) Date of publication of application: 05.04.2023
(73) Proprietor: Mission Therapeutics Limited, Cambridge CB22 3AT (GB)
(72) Inventor: LUCKHURST, Christopher Andrew, Cambridge CB22 3AT (GB); KEMP, Mark Ian, Cambridge CB22 3AT (GB); THOMPSON, Paul William, Cambridge CB22 3AT (GB)
(74) Representative: TLIP Limited
(86) International application number: PCT/EP2021/064166
(87) International publication number: WO 2021/239863

(56) References cited:
- WO-A1-2016/156816
- WO-A1-2020/212350
- WO-A1-2021/043870

## Description

### FIELD OF THE INVENTION

The present invention relates to a class of N-cyanopyrrolidines with activity as inhibitors of the deubiquitylating enzyme ubiquitin C-terminal hydrolase 30, also known as ubiquitin specific peptidase 30 (USP30), the compounds for use in methods of medical treatment, processes for the preparation thereof and composition containing said inhibitors. These inhibitors have utility in a variety of therapeutic areas, including conditions involving mitochondrial dysfunction, cancer and fibrosis.

### BACKGROUND OF THE INVENTION

Ubiquitin is a small protein consisting of 76 amino acids that is important for the regulation of protein function in the cell. Ubiquitylation and deubiquitylation are enzymatically mediated processes by which ubiquitin is covalently bound or cleaved from a target protein by deubiquitylating enzymes (DUBs), of which there are approximately 100 DUBs in human cells, divided into sub-families based on sequence homology. The USP family are characterised by their common Cys and His boxes which contain Cys and His residues critical for their DUB activities. The ubiquitylation and deubiquitylation processes have been implicated in the regulation of many cellular functions including cell cycle progression, apoptosis, modification of cell surface receptors, regulation of DNA transcription and DNA repair. Thus, the ubiquitin system has been implicated in the pathogenesis of numerous disease states including inflammation, viral infection, metabolic dysfunction, CNS disorders, and oncogenesis.

Ubiquitin is a master regulator of mitochondrial dynamics. Mitochondria are dynamic organelles whose biogenesis, fusion and fission events are regulated by the post-translational regulation via ubiquitylation of many key factors such as mitofusins. In humans, USP30 is a 517 amino acid protein which is found in the mitochondrial outer membrane (Nakamura et al, 2008, Mol Biol 19:1903-11). It is the sole deubiquitylating enzyme bearing a mitochondrial addressing signal and has been shown to deubiquitylate a number of mitochondrial proteins. It has been demonstrated that USP30 opposes parkin-mediated mitophagy and that reduction of USP30 activity can rescue parkin-mediated defects in mitophagy (Bingol et al, 2015, Nature 510:370-5; Gersch et al, 2017, Nat Struct Mol Biol 24(11): 920-930; Cunningham et al, 2015, Nat Cell Biol 17(2): 160-169). USP30 inactivation can also increase mitochondrial protein import, potentially through ubiquitylation of TOM proteins (Jacoupy et al, 2019, Sci Rep 9(1): 11829). A small proportion of USP30 has been localized to peroxisomes, which are generated through fusion of mitochondrial and ER vesicles, with USP30 potentially antagonizing the Pex2/pexophagy pathway (Riccio et al, 2019, J Cell Biol 218(3): 798-807). The E3 Ub ligase March5 and the deubiquitinase USP30 associate with the translocase and regulate mitochondrial import, and while March5 opposes mitochondrial import and directs degradation of substrates, USP30 deubiquitinates substrates to promote their import (Phu et al, 2020, Molecular Cell 77, 1107-1123). Mitochondrial dysfunction can be defined as diminished mitochondrial content (mitophagy or mitochondrial biogenesis), as a decrease in mitochondrial activity and oxidative phosphorylation, but also as modulation of reactive oxygen species (ROS) generation. Hence a role for mitochondrial dysfunctions in a very large number of aging processes and pathologies.

For example, Parkinson's disease affects around 10 million people worldwide (Parkinson's Disease Foundation) and is characterised by the loss of dopaminergic neurons in the substantia nigra. The exact mechanisms underlying PD are unclear; however mitochondrial dysfunction is increasingly appreciated as a key determinant of dopaminergic neuronal susceptibility in PD and is a feature of both familial and sporadic disease, as well as in toxin-induced Parkinsonism. Parkin is one of a number of proteins that have been implicated with early onset PD. While most PD cases are linked to defects in alpha-synuclein, 10% of Parkinson's cases are linked to specific genetic defects, one of which is in the ubiquitin E3 ligase parkin. Parkin and the protein kinase PTEN-induced putative kinase 1 (PINK1) collaborate to ubiquitylate mitochondrial membrane proteins of damaged mitochondria resulting in mitophagy. Dysregulation of mitophagy results in increased oxidative stress, which has been described as a characteristic of PD. Inhibition of USP30 could therefore be a potential strategy for the treatment of PD. For example, PD patients with parkin mutations leading to reduced activity could be therapeutically compensated by inhibition of USP30.

It has been reported that depletion of USP30 enhances mitophagic clearance of mitochondria and also enhances parkin-induced cell death. USP30 has also been shown to regulate BAX/BAK-dependent apoptosis independently of parkin overexpression. Depletion of USP30 sensitises cancer cells to BH-3 mimetics such as ABT-737, without the need for parkin overexpression. Thus, an anti-apoptotic role has been demonstrated for USP30 and USP30 is therefore a potential target for anti-cancer therapy.

The ubiquitin-proteasome system has gained interest as a target for the treatment of cancer following the approval of the proteasome inhibitor bortezomib (Velcade^{®}) for the treatment of multiple myeloma. Extended treatment with bortezomib is limited by its associated toxicity and drug resistance. However, therapeutic strategies that target specific aspects of the ubiquitin-proteasome pathway upstream of the proteasome, such as DUBs, are predicted to be better tolerated (Bedford et al, 2011, Nature Rev 10:29-46).

Fibrotic diseases, including renal, hepatic and pulmonary fibrosis, are a leading cause of morbidity and mortality and can affect all tissues and organ systems. Fibrosis is considered to be the result of acute or chronic stress on the tissue or organ, characterized by extracellular matrix deposition, reduction of vascular/tubule/duct/airway patency and impairment of function ultimately resulting in organ failure. Many fibrotic conditions are promoted by lifestyle or environmental factors; however, a proportion of fibrotic conditions can be initiated through genetic triggers or indeed are considered idiopathic (i.e. without a known cause). Certain fibrotic disease, such as idiopathic pulmonary fibrosis (IPF), can be treated with non-specific kinase inhibitor (nintedanib) or drugs without a well-characterized mechanism of action (pirfenidone). Other treatments for organ fibrosis, such as kidney or liver fibrosis, alleviate pressure on the organ itself (e.g. beta blockers for cirrhosis, angiotensin receptor blockers for chronic kidney disease). Attention to lifestyle factors, such as glucose and diet control, may also influence the course and severity of disease.

Mitochondrial dysfunction has been implicated in a number of fibrotic diseases, with oxidative stress downstream of dysfunction being the key pathogenic mediator, alongside decreased ATP production. In preclinical models, disruption of the mitophagy pathway (through mutation or knockout of either parkin or PINK1) exacerbates lung fibrosis and kidney fibrosis, with evidence of increased oxidative stress.

Kurita et al, 2017, Respiratory Research 18:114, discloses that accumulation of profibrotic myofibroblasts is a crucial process for fibrotic remodelling in IPF. Recent findings are said to show participation of autophagy/mitophagy, part of the lysosomal degradation machinery, in IPF pathogenesis, and that mitophagy has been implicated in myofibroblast differentiation through regulating mitochondrial reactive oxygen species (ROS)-mediated platelet-derived growth factor receptor (PDGFR) activation. Kurita's results suggested that pirfenidone induces PARK2-mediated mitophagy and also inhibits lung fibrosis development in the setting of insufficient mitophagy, which may at least partly explain the anti-fibrotic mechanisms for IPF treatment.

Williams et al, 2015, Pharmacol Res. December; 102: 264-269, discuss the role of PINK1-Parkin-mediated autophagy in protecting against alcohol and acetaminophen-induced liver injury by removing damaged mitochondria via mitophagy. It is suggested that pharmacological stabilization of USP8 or inactivation of USP15 and USP30 may be potential therapeutic targets for upregulating Parkin-induced mitophagy and in turn protect against drug-induced liver injury. However, it is noted that the DUBs are regulated both transcriptionally and post-translationally, which may make drug development for targeting these specific enzymes challenging, and in addition, phosphorylated ubiquitin was shown to be resistant to DUBs. The authors conclude that upregulating PINK1 stabilization or kinase activity may be a more effective target than inhibiting DUBs.

Williams et al, 2015, Biomolecules 5, 2619-2642, and Williams et al, 2015, Am J Physiol Gastrointest Liver Physiol 309: G324-G340, review mechanisms involved in regulation of mitochondrial homeostasis in the liver and how these mechanisms may protect against alcohol-induced liver disease.

Luciani et al, 2020, Nat. Commun. 11, 970, reports deregulation of mitochondrial network in terminally differentiated cells contributes to a broad spectrum of disorders, including methylmalonic acidemia (MMA). MMA is one of the most common inherited metabolic disorders, due to deficiency of the mitochondrial methylmalonyl-coenzyme A mutase (MMUT). MMUT deficiency induces metabolic and mitochondrial alterations that are exacerbated by anomalies in PINK1/Parkin-mediated mitophagy, causing the accumulation of dysfunctional mitochondria that trigger epithelial stress and ultimately cell damage. A link is suggested between primary MMUT deficiency, diseased mitochondria, mitophagy dysfunction and epithelial stress, and potential therapeutic perspectives for MMA is provided.

Kluge et al, Bioorganic & Medicinal Chemistry Letters, 2018, 28 2655-2659, reports that selective inhibitors of USP30 accelerate mitophagy.

Series of derivatives of N-cyano-substituted heterocycles are disclosed as deubiquitylating enzyme inhibitors in PCT applications WO 2016/046530 (US 15/513125, US 15/894025, US 16/448066), WO 2016/156816 (US 15/558632, US 16/297937, US 16/419558, US 16/419747, US 16/788446), WO 2017/009650 (US 15/738900), WO 2017/093718 (US 15/776149), WO 2017/103614 (US 15/781615), WO 2017/149313 (US 16/078518), WO 2017/109488 (US 16/060299), WO 2017/141036 (US 16/070936), WO 2017/163078 (US 16/087515), WO 2017/158381 (US 16/080229), WO 2017/158388 (US 16/080506), WO 2018/065768 (US 16/336685), WO 2018/060742 (US 16/336202), WO 2018/060689 (US 16/334836), WO 2018/060691 (US 16/336363), WO 2018/220355 (US 16/615040), WO 2018/234755 (US 16/615709), WO 2020/212350, WO 2020/212351 and WO 2021/043870, each of which are expressly incorporated herein by reference. PCT application WO 2019/171042 (US 16/977019), which is expressly incorporated herein by reference, discloses the use of N-cyanopyrrolidines as inhibitors of USP30 for the treatment of fibrotic diseases.

Falgueyret et al, 2001, J.Med.Chem. 44, 94-104, and PCT application WO 01/77073 refer to cyanopyrrolidines as inhibitors of Cathepsins K and L, with potential utility in treating osteoporosis and other bone-resorption related conditions. PCT application WO 2015/179190 refers to N-acylethanolamine hydrolysing acid amidase inhibitors, with potential utility in treating ulcerative colitis and Crohn's disease. PCT application WO 2013/030218 refers to quinazolin-4-one compounds as inhibitors of ubiquitin specific proteases, such as USP7, with potential utility in treating cancer, neurodegenerative diseases, inflammatory disorders and viral infections. PCT applications WO 2015/017502 and WO 2016/019237 refer to inhibitors of Bruton's tyrosine kinase with potential utility in treating disease such as autoimmune disease, inflammatory disease and cancer. PCT applications WO 2009/026197, WO 2009/129365, WO 2009/129370, and WO 2009/129371, refer to cyanopyrrolidines as inhibitors of Cathepsin C with potential utility in treating COPD. United States patent application US 2008/0300268 refers to polyaromatic compounds as inhibitors of tyrosine kinase receptor PDGFR. PCT applications WO 2019/222468, WO 2019/071073, WO 2020/036940 and WO 2020/072964, Rusilowicz-Jones et al, 2020, bioRxiv 2020.04.16.044206 (20 April 2020), and Tsefou et al, bioRxiv 2021.02.02.429344 (2 February 2021), refer to cyanamide-containing compounds as USP30 inhibitors. Yue et al, 2014, Cell Research, 24, 482-496, refers to a diterpenoid derivative 15-oxospiramilactone as a USP30 inhibitor that induced mitochondrial fusion.

PCT application WO 2015/183987 refers to pharmaceutical compositions comprising deubiquitinase inhibitors and human serum albumin in methods of treating cancer, fibrosis, an autoimmune disease or condition, an inflammatory disease or condition, a neurodegenerative disease or condition or an infection. It is noted that deubiquitinases, including UCHL5/UCH37, USP4, USP9X, USP11 and USP15, are said to have been implicated in the regulation of the TGF-beta signalling pathway, the disruption of which gives rise to neurodegenerative and fibrotic diseases, autoimmune dysfunction and cancer.

PCT application WO 2006/067165 refers to a method for treating fibrotic diseases using indolinone kinase inhibitors. PCT application WO 2007/119214 refers to a method for treating early stage pulmonary fibrosis using an endothelin receptor antagonist. PCT application WO 2012/170290 refers to a method for treating fibrotic diseases using THC acids. PCT application WO 2018/213150 refers to sulfonamide USP30 inhibitors with potential utility in the treatment of conditions involving mitochondrial defects. Larson-Casey et al, 2016, Immunity 44, 582-596, concerns macrophage Akt1 kinase-mediated mitophagy, apoptosis resistance and pulmonary fibrosis. Tang et al, 2015, Kidney Diseases 1, 71-79, reviews the potential role of mitophagy in renal pathophysiology.

There exists a need for safe, alternative, and/or improved methods and compositions for the treatment or prevention of conditions involving mitochondrial dysfunction, cancer and fibrosis, and the various symptoms and conditions associated therewith. While not wishing to be bound by any particular theory or mechanism, it is believed that the compounds of the present invention act to inhibit the enzyme USP30, which in turn upregulates Parkin-induced mitophagy.

Acute Kidney Injury (AKI) is defined as an abrupt decrease in kidney function occurring over 7 days or less, with severity of injury staged based on increased serum creatinine (SCr) and decreased urine output as described in the Kidney Disease Improving Global Outcomes (KDIGO) guidelines. AKI occurs in about 13.3 million people per year, 85% of whom live in the developing world and it is thought to contribute to about 1.7 million deaths every year (Mehta et al, 2015, Lancet 385(9987): 2616-2643). AKI more than likely results in permanent kidney damage (i.e., chronic kidney disease; CKD) and may also result in damage to non-renal organs. AKI is a significant public health concern particularly when considering the absolute number of patients developing incident CKD, progressive CKD, end-stage renal disease and cardiovascular events. AKI has been found to be prevalent in patients hospitalised by COVID-19 and is strongly associated with hospital mortality, with mitochondrial damage and dysfunction reported as a potential pathophysiological mechanism and therapeutic target (Kellum et al, Nephrol Dial Transplant (2020) 35: 1652-1662).

AKI and CKD are viewed as a continuum on the same disease spectrum (Chawla et al, 2017, Nat Rev Nephrol 13(4): 241-257). Patients undergoing coronary artery bypass graft (CABG) are at high risk for kidney injury. There is an obvious unmet medical need in the development of medicinal products for the treatment and/or prevention of AKI.

The kidney is a site of high metabolic demand, with high mitophagy rates demonstrated in vivo (McWilliams et al, 2018, Cell Metab 27(2): 439-449 e435). Renal Proximal Tubule Epithelial Cells (RPTECs), a cell type with significant ATP requirement for solute/ion exchange, are rich in mitochondria and are the primary effector cells of Acute Kidney Injury (AKI) in the kidney. Mitochondrial dysfunction has been implicated in AKI/CKD mechanisms, both through multiple lines of evidence from preclinical AKI and CKD models and also through data demonstrating abnormal mitochondrial phenotypes in patient biopsies (Emma et al, 2016, Nat Rev Nephrol 12(5): 267-280; Eirin et al, 2017, Handb Exp Pharmacol 240: 229-250). Furthermore, Primary mitochondrial disease often manifest in renal symptoms, such as focal segmental glomerulosclerosis (Kawakami et al, 2015, J Am Soc Nephrol 26(5): 1040-1052) in patients with MELAS/MIDD, and also primary tubular pathologies in patients with Coenzyme Q deficiencies. Mutations in mtDNA can cause maternally inherited tubulointerstitial disease (Connor et al, 2017, PLoS Genet 13(3): e1006620).

Regarding mitochondrial quality control in renal injury (Tang et al, 2018, Autophagy 14(5): 880-897) demonstrated that renal injury was exacerbated following ischemic AKI in both PINK1 KO and PARK2 KO mice, suggesting that PINK1/PARKIN-mediated mitophagy plays a protective role following IRI in the kidney. In addition, parkin/PINK1 mitophagy protects against cisplatin induced kidney injury (Wang et al, 2018, Cell Death Dis 9(11): 1113). Limited models of CKD are available for mitophagy investigation, supportive evidence for mitochondrial quality control in fibrosis comes from studies on fibrotic lung conditions such as COPD and IPF. Parkin knockout animals show exacerbated lung fibrosis in response to bleomycin (Kobayashi et al, 2016, J Immunol, 197:504-516). Similarly, airway epithelial cells from parkin knockout (KO) animals show exacerbated fibrotic and senescent responses to cigarette smoke (Araya et al, 2019, Autophagy 15(3): 510-526).

Preclinical models are available to study potential novel therapeutics, through their ability to model fibrosis pathology (e.g. collagen deposition) consistent with the human condition. Preclinical models can be toxin-mediated (e.g. bleomycin for lung and skin fibrosis), surgical (e.g. ischemia/reperfusion injury model and unilateral ureter obstruction model for acute tubulointerstitial fibrosis), and genetic (e.g. diabetic (db/db) mice for diabetic nephropathy). For example, both examples previously given for indicated IPF treatments (nintedanib and pirfenidone) show efficacy in the bleomycin lung fibrosis model.

Accordingly, there is a need for compounds that are inhibitors of USP30 for the treatment or prevention of conditions where inhibition of USP30 is indicated. In particular, there exists a need for USP30 inhibitors that have suitable and/or improved properties in order to maximise efficacy against the target disease.

### SUMMARY OF THE INVENTION

The present invention is directed to a compound of formula (I), which is selected from formula (I)(i) and formula (I)(ii): 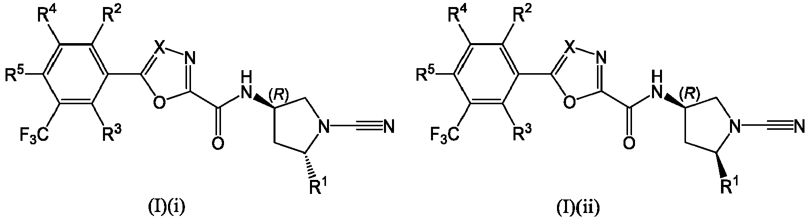 a tautomer thereof , or a pharmaceutically acceptable salt of said compound or tautomer, wherein:
X is CH or N;
R¹ is selected from (C₁-C₄)alkyl, (C₁-C₄)fluoroalkyl, CH₂OCH₃, CH₂SO₂CH₃ and CH₂-N-linked triazole;
R² is selected from hydrogen, halogen, (C₁-C₄)alkoxy and cyclopropoxy; and
R³, R⁴ and R⁵ are each independently selected from hydrogen and halogen.

The present invention is also directed to the compounds of formula (I) for use in methods of medical treatment, particularly in the treatment of conditions involving mitochondrial dysfunction, cancer and fibrosis, and also processes for the preparation thereof and pharmaceutical compositions containing said compounds.

### DETAILED DESCRIPTION OF THE INVENTION

The present invention is directed to USP30 inhibitors that have suitable and/or improved properties in order to maximise efficacy against the target disease. Such properties include, for example, potency, selectivity, physicochemical properties, ADME (absorption, distribution, metabolism and excretion) properties, including PK (pharmacokinetic) profile, and safety profile.

It is generally desirable to maximise the potency of a drug molecule against the target enzyme in relevant assays in order to lower the effective/efficacious dosage that is to be administered to patients. Compounds of the invention may be tested for USP30 affinity using the in vitro biochemical fluorescence polarization (FP) assay described herein.

USP30 is a transmembrane protein located in the outer membrane of mitochondria, which are energy-producing organelles present inside cells. Therefore, being able to demonstrate cellular activity in vitro is advantageous, as this is one of a number of components that may indicate a greater ability to engage the target in its physiological setting, i.e. where the USP30 inhibitor compound is able to penetrate cells. The USP30 cellular western blot (WB) assay aims to test the activity of compounds against USP30 in cells using an irreversible activity probe to monitor USP30 activity. Analogously to the cellular western blot assay, target engagement assessment (ex vivo) may be carried out in either brain or kidney tissue samples from compound-dosed animals.

To extend target binding knowledge to downstream pharmacodynamics, assessment of TOM20 (an outer mitochondrial membrane protein) ubiquitylation may be made.

In general, it is important for a drug to be as selective as possible for its desired target enzyme; additional activities give rise to the possibility of side effects. The exact physiological role of many DUBs has yet to be fully determined, however, irrespective of whatever role these DUBs may or may not play, it is a sound medicinal chemistry precept to ensure that any drug has selectivity over related mechanistic targets of unknown physiological function. Representative examples of DUB enzymes for which the compounds of the present invention may be screened against are UCHL1, UCHL3, UCHL5, YOD1, SENP2, SENP6, TRABID, BAP1, Cezanne, MINDY2/FAM63B, OTU1, OTUD3, OTUD5, OTUD6A, OTUD6B, OTUB1/UBCH5B, OTUB2, CYLD, VCPIP, AMSH-LP, JOSD1, JOSD2, USP1/UAF1, USP2, USP4, USP5, USP6, USP7, USP8, USP9x, USP10, USP11, USP12/UAF1, USP13, USP14, USP15, USP16, USP19, USP20, USP21, USP22, USP24, USP25, USP28, USP32, USP34, USP35, USP36, USP45, USP46/UAF1, USP47 and USP48. Preferably, compounds of the invention have good selectivity for USP30 over one or more of these DUB enzymes.

Aside from selectivity over other DUB enzymes, it is important for a drug to have low affinity for other targets, and pharmacological profiling may be performed against panels of targets to assess the potential for, and to minimise, potential off-target effects. Examples of targets for which the compounds of the present invention may be screened against are those of the industry standard Eurofins-Cerep SafetyScreen44 panel, which includes 44 targets as a representative selection of GPCR receptors, transporters, ion channels, nuclear receptors, and kinase and non-kinase enzymes. Preferably, compounds of the invention have insignificant affinity against targets of this screening panel. Further examples of targets for which the compounds of the present invention may be screened against are kinases of the Thermo Fisher SelectScreen kinase profiling panel, which includes 39 targets as a representative selection of kinase enzymes. Preferably, compounds of the invention have insignificant affinity against targets of this screening panel. Additionally, examples of a particular enzyme class for which the compounds of the present invention may be screened against are the cathepsins (e.g. cathepsin A, B, C, H, K, L, L2, S, V and Z). Preferably, compounds of the invention have good selectivity for USP30 over one or more of these enzymes.

There is also a need for compounds that have favourable pharmacokinetic properties such that they are suitable for oral administration. An orally administered drug should have good bioavailability; that is an ability to readily cross the gastrointestinal (GI) tract and not be subject to extensive metabolism as it passes from the GI tract into the systemic circulation. Once a drug is in the systemic circulation the rate of metabolism is also important in determining the time of residence of the drug in the body.

Thus, it is clearly favourable for drug molecules to have the properties of being readily able to cross the GI tract and being only slowly metabolised in the body. The Caco-2 assay is a widely accepted model for predicting the ability of a given molecule to cross the GI tract. The majority of metabolism of drug molecules generally occurs in the liver, and in vitro assays using whole cell hepatocytes (animal or human) are widely accepted methods for measuring the susceptibility of a given molecule towards metabolism in the liver. Such assays aim to predict in vivo clearance from the hepatocyte calculated clearance value.

Compounds which have good Caco-2 flux and are stable towards hepatocytes are predicted to have good oral bioavailability (good absorption across the GI tract and minimal extraction of compound as it passes through the liver) and a long residence time in the body that is sufficient for the drug to be efficacious.

The solubility of a compound is an important factor in achieving a desired concentration of drug in systemic circulation for the anticipated pharmacological response. Low aqueous solubility is a problem encountered with formulation development of new chemical entities and to be absorbed a drug must be present in the form of solution at the site of absorption. The kinetic solubility of a compound may be measured using a turbidimetric solubility assay, the data from which may also be used in conjunction with Caco-2 permeability data to predict dose dependent human intestinal absorption.

Other parameters that may be measured using standard assays that are indicative of a compound's exposure profile include, for example plasma stability (half-life measurement), blood AUC, Cₘₐₓ, Cₘᵢₙ and Tₘₐₓ values.

The treatment of CNS disorders, including Alzheimer's disease, Parkinson's disease, and other disorders described herein, requires drug molecules to target the brain, which requires adequate penetration of the blood brain barrier. There is, therefore, a need for USP30 inhibitors that possess effective blood brain penetration properties and provide suitable residence time in the brain to be efficacious. The probability that a compound can cross the blood brain barrier may be measured by an in vitro flux assay utilizing a MDR1-MDCK cell monolayer (Madin-Darby Canine Kidney cells transfected with MDR-1 resulting in overexpression of the human efflux transporter P-glycoprotein). Additionally, exposure may also be measured directly in brain and plasma using in vivo animal models.

There is also a need for compounds that have a favourable safety profile, which may be measured by a variety of standard in vitro and in vivo methods. A cell toxicity counter-screen may be used to assay the anti-proliferative/cytotoxic effect in a particular cell line (e.g. HCT116) by fluorometric detection of rezasurin (alamarBlue^{™}) to resofurin in response to mitochondrial activity.

Toxicology and safety studies may also be conducted to identify potential target organs for adverse effects and define the Therapeutic Index to set the initial starting doses in clinical trials. Regulatory requirements generally require studies to be conducted in at least two laboratory animal species, one rodent (rat or mouse) and one nonrodent (rabbit, dog, non-human primate, or other suitable species).

The bacterial reverse mutation assay (Ames Test) may be used to evaluate the mutagenic properties of compounds of the invention, commonly by using the bacterial strain Salmonella typhimurium, which is mutant for the biosynthesis of the amino acid histidine.

The micronucleus assay may be used to determine if a compound is genotoxic by evaluating the presence of micronuclei. Micronuclei may contain chromosome fragments produced from DNA breakage (clastogens) or whole chromosomes produced by disruption of the mitotic apparatus (aneugens).

The hERG predictor assay provides valuable information about the possible binding of test compounds to the potassium channel and potential QT prolongation on echocardiogram. Inhibition of the hERG current causes QT interval prolongation resulting in potentially fatal ventricular tachyarrhythmia (Torsades de Pointes). Typically, assay data may be generated from an automated patch-clamp assay platform.

The present invention is therefore directed to USP30 inhibitors that have suitable and/or improved properties in order to maximise efficacy against the target disease. Such properties include, for example, potency, selectivity, physicochemical properties, ADME (absorption, distribution, metabolism and excretion) properties, including PK (pharmacokinetic) profile, and safety profile.

Compounds of the present invention have been found to demonstrate one or more of the above identified properties that are both significant and unexpected. For instance, Examples 1 to 12 of the present invention are highly potent for USP30, as measured in the biochemical assay described herein. All of these Examples (1 to 12) of the present invention are significantly more selective for USP30 over other DUBs and cathepsins. The significant and unexpected properties of the compounds of the present invention make them particularly suitable for use in the treatment and/or prevention of diseases linked to USP30 activity.

According to a first aspect, the present invention provides a compound of formula (I), which is selected from formula (I)(i) and formula (I)(ii): 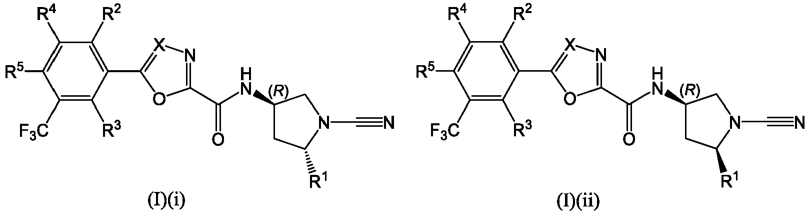 a tautomer thereof , or a pharmaceutically acceptable salt of said compound or tautomer, wherein:
X is CH or N;
R¹ is selected from (C₁-C₄)alkyl, (C₁-C₄)fluoroalkyl, CH₂OCH₃, CH₂SO₂CH₃ and CH₂-N-linked triazole;
R² is selected from hydrogen, halogen, (C₁-C₄)alkoxy and cyclopropoxy; and
R³, R⁴ and R⁵ are each independently selected from hydrogen and halogen.

The compound of formula (I) exists as a single stereoisomer with the absolute stereochemistry shown.

Alkyl and alkoxy groups may be straight or branched and contain 1 to 4 carbon atoms. Examples of alkyl include methyl, ethyl, n-propyl, isopropyl, n-butyl, isobutyl, and sec-butyl. Examples of alkoxy include methoxy, ethoxy, n-propoxy, isopropoxy and t-butoxy.

Halogen means fluorine, chlorine, bromine or iodine, in particular, fluorine or chlorine. Fluoroalkyl groups may contain one or more fluorine substituents. Examples are fluoromethyl, difluoromethyl and trifluoromethyl. The N-linked triazole may be 1,2,3-triazole or 1,2,4-triazole and is preferably, in all embodiments of the invention, a 1,2,3-triazol-1-yl ring.

Unless otherwise indicated, the term substituted means substituted by one or more defined groups. In the case where groups may be selected from more than one alternative, the selected groups may be the same or different. The term 'independently' means that where more than one substituent is selected from more than one possible substituent, those substituents may be the same or different.

Preferred embodiments of the compound of formula (I) are defined below.

Preferably, R¹ is selected from methyl, CH₂F, CHF₂, CF₃, CH₂OCH₃, CH₂SO₂CH₃ and CH₂-N-linked triazole.

More preferably, R¹ is selected from methyl, CH₂OCH₃, CH₂SO₂CH₃ and CH₂-N-linked triazole. Most preferably, R¹ is CH₂OCH₃.

Preferably, R² is selected from hydrogen, chlorine, fluorine, methoxy and cyclopropoxy.

More preferably, R² is selected from hydrogen, chlorine, methoxy and cyclopropoxy.

Most preferably, R² is hydrogen.

Preferably, R³, R⁴ and R⁵ are each independently selected from hydrogen, chlorine and fluorine. Most preferably, R³, R⁴ and R⁵ are each hydrogen.

According to one aspect of the invention, the compound of formula (I) has the formula (I)(i):

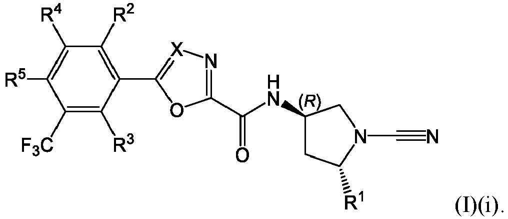

According to another aspect of the invention, the compound of formula (I) has the formula (I)(ii):

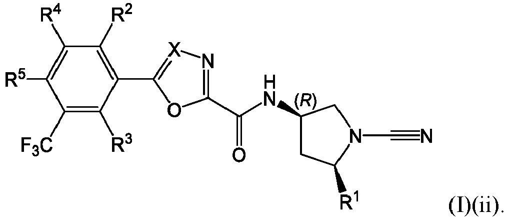

According to a first preferred aspect, the invention provides a compound of formula (I)(i) wherein X is CH; providing the compound of formula (IA)(i): 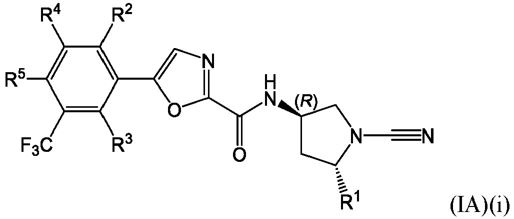 a tautomer thereof, or a pharmaceutically acceptable salt of said compound or tautomer, wherein:
R¹ is selected from (C₁-C₄)alkyl, (C₁-C₄)fluoroalkyl, CH₂OCH₃, CH₂SO₂CH₃ and CH₂-N-linked triazole;
R² is selected from hydrogen, halogen, (C₁-C₄)alkoxy and cyclopropoxy; and
R³, R⁴ and R⁵ are each independently selected from hydrogen and halogen.

Preferred embodiments of the compound of formula (IA)(i) are defined below.

Preferably, R¹ is selected from methyl, CH₂F, CHF₂, CF₃, CH₂OCH₃, CH₂SO₂CH₃ and CH₂-N-linked triazole.

More preferably, R¹ is selected from methyl, CH₂OCH₃, CH₂SO₂CH₃ and CH₂-N-linked triazole. Most preferably, R¹ is CH₂OCH₃.

Preferably, R² is selected from hydrogen, chlorine, fluorine, methoxy and cyclopropoxy.

More preferably, R² is selected from hydrogen, chlorine, methoxy and cyclopropoxy.

Yet more preferably, R² is selected from hydrogen and cyclopropoxy.

Most preferably, R² is hydrogen.

Preferably, R³, R⁴ and R⁵ are each independently selected from hydrogen, chlorine and fluorine.

Most preferably, R³, R⁴ and R⁵ are each hydrogen.

According to a second preferred aspect, the invention provides a compound of formula (I)(i) wherein X is N; providing the compound of formula (IB)(i): 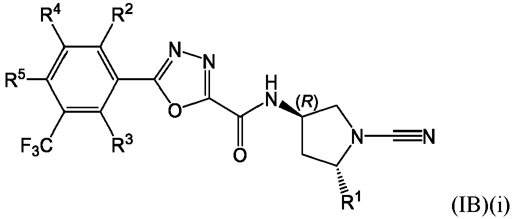 a tautomer thereof, or a pharmaceutically acceptable salt of said compound or tautomer, wherein:
R¹ is selected from (C₁-C₄)alkyl, (C₁-C₄)fluoroalkyl, CH₂OCH₃, CH₂SO₂CH₃ and CH₂-N-linked triazole;
R² is selected from hydrogen, halogen, (C₁-C₄)alkoxy and cyclopropoxy; and
R³, R⁴ and R⁵ are each independently selected from hydrogen and halogen.

Preferred embodiments of the compound of formula (IB)(i) are defined below.

Preferably, R¹ is selected from methyl, CH₂F, CHF₂, CF₃, CH₂OCH₃, CH₂SO₂CH₃ and CH₂-N-linked triazole.

More preferably, R¹ is selected from methyl, CH₂OCH₃, CH₂SO₂CH₃ and CH₂-N-linked triazole.

Most preferably, R¹ is selected from methyl and CH₂OCH₃.

Preferably, R² is selected from hydrogen, chlorine, fluorine, methoxy and cyclopropoxy.

More preferably, R² is selected from hydrogen, chlorine, methoxy and cyclopropoxy.

Most preferably, R² is hydrogen.

Preferably, R³, R⁴ and R⁵ are each independently selected from hydrogen, chlorine and fluorine.

Most preferably, R³, R⁴ and R⁵ are each hydrogen.

Preferred compounds of formula (I)(i) of the present invention are selected from:
N-((3*R*,5*S*)-1-cyano-5-(methoxymethyl)-pyrrolidin-3-yl)-5-(3-(trifluoromethyl)phenyl)oxazole-2-carboxamide;
N-((3*R*,5*R*)-1-cyano-5-methylpyrrolidin-3-yl)-5-(3-(trifluoromethyl)phenyl)oxazole-2-carboxamide; N-((3*R*,5*S*)-1-cyano-5-((methylsulfonyl)methyl)pyrrolidin-3-yl)-5-(3-(trifluoromethyl)phenyl)-oxazole-2-carboxamide;
N-((3*R*,5*S*)-5-((1H-1,2,3-triazol-1-yl)methyl)-1-cyanopyrrolidin-3-yl)-5-(3-(trifluoromethyl)phenyl) oxazole-2-carboxamide;
N-((3*R*,5*S*)-1-cyano-5-(methoxymethyl)pyrrolidin-3-yl)-5-(2-cyclopropoxy-5-(trifluoromethyl)phenyl)-1,3,4-oxadiazole-2-carboxamide;
N-((3*R*,5*R*)-1-cyano-5-methylpyrrolidin-3-yl)-5-(2-cyclopropoxy-5-(trifluoromethyl)phenyl)-1,3,4-oxadiazole-2-carboxamide;
N-((3R,SR)-1-cyano-5-methylpyrrolidin-3-yl)-5-(3-(trifluoromethyl)phenyl)-1,3,4-oxadiazole-2-carboxamide;
N-((3*R*,5*S*)-1-cyano-5-(methoxymethyl)pyrrolidin-3-yl)-5-(3-(trifluoromethyl)phenyl)-1,3,4-oxadiazole-2-carboxamide;
N-((3*R*,5*S*)-1-cyano-5-(methoxymethyl)pyrrolidin-3-yl)-5-(2-cyclopropoxy-5-(trifluoromethyl)phenyl)-oxazole-2-carboxamide;
N-((3*R*,5*S*)-1-cyano-5-(methoxymethyl)pyrrolidin-3-yl)-5-(2-methoxy-5-(trifluoromethyl)phenyl)-1,3,4-oxadiazole-2-carboxamide; and
5-(2-chloro-5-(trifluoromethyl)phenyl)-N-((3*R*,5*S*)-1-cyano-5-(methoxymethyl)pyrrolidin-3-yl)-1,3,4-oxadiazole-2-carboxamide;
or a pharmaceutically acceptable salt thereof.

According to a third preferred aspect, the invention provides a compound of formula (I)(ii) wherein X is CH; providing the compound of formula (IA)(ii): 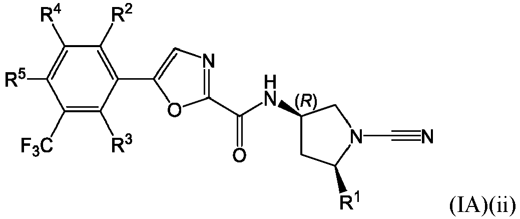 a tautomer thereof, or a pharmaceutically acceptable salt of said compound or tautomer, wherein:
R¹ is selected from (C₁-C₄)alkyl, (C₁-C₄)fluoroalkyl, CH₂OCH₃, CH₂SO₂CH₃ and CH₂-N-linked triazole;
R² is selected from hydrogen, halogen, (C₁-C₄)alkoxy and cyclopropoxy; and
R³, R⁴ and R⁵ are each independently selected from hydrogen and halogen.

Preferred embodiments of the compound of formula (IA)(ii) are defined below.

Preferably, R¹ is selected from methyl, CH₂F, CHF₂, CF₃, CH₂OCH₃, CH₂SO₂CH₃ and CH₂-N-linked triazole.

More preferably, R¹ is selected from methyl, CH₂OCH₃, CH₂SO₂CH₃ and CH₂-N-linked triazole. Most preferably, R¹ is CH₂OCH₃.

Preferably, R² is selected from hydrogen, chlorine, fluorine, methoxy and cyclopropoxy.

More preferably, R² is selected from hydrogen, chlorine, methoxy and cyclopropoxy.

Yet more preferably, R² is selected from hydrogen and cyclopropoxy.

Most preferably, R² is hydrogen.

Preferably, R³, R⁴ and R⁵ are each independently selected from hydrogen, chlorine and fluorine.

Most preferably, R³, R⁴ and R⁵ are each hydrogen.

According to a fourth preferred aspect, the invention provides a compound of formula (I)(ii) wherein X is N; providing the compound of formula (IB)(ii): 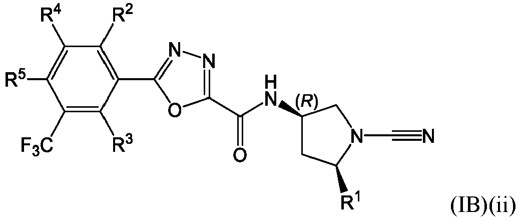 a tautomer thereof, or a pharmaceutically acceptable salt of said compound or tautomer, wherein:
R¹ is selected from (C₁-C₄)alkyl, (C₁-C₄)fluoroalkyl, CH₂OCH₃, CH₂SO₂CH₃ and CH₂-N-linked triazole;
R² is selected from hydrogen, halogen, (C₁-C₄)alkoxy and cyclopropoxy; and
R³, R⁴ and R⁵ are each independently selected from hydrogen and halogen.

Preferred embodiments of the compound of formula (IB)(ii) are defined below.

Preferably, R¹ is selected from methyl, CH₂F, CHF₂, CF₃, CH₂OCH₃, CH₂SO₂CH₃ and CH₂-N-linked triazole.

More preferably, R¹ is selected from methyl, CH₂OCH₃, CH₂SO₂CH₃ and CH₂-N-linked triazole. Most preferably, R¹ is selected from methyl and CH₂OCH₃.

Preferably, R² is selected from hydrogen, chlorine, fluorine, methoxy and cyclopropoxy.

More preferably, R² is selected from hydrogen, chlorine, methoxy and cyclopropoxy.

Most preferably, R² is hydrogen.

Preferably, R³, R⁴ and R⁵ are each independently selected from hydrogen, chlorine and fluorine.

Most preferably, R³, R⁴ and R⁵ are each hydrogen.

Preferred compounds of formula (I)(ii) of the present invention are selected from:
N-((3*R*,5*R*)-1-cyano-5-(methoxymethyl)-pyrrolidin-3-yl)-5-(3-(trifluoromethyl)phenyl)oxazole-2-carboxamide;
N-((3*R*,5*S*)-1-cyano-5-methylpyrrolidin-3-yl)-5-(3-(trifluoromethyl)phenyl)oxazole-2-carboxamide; N-((3*R*,5*R*)-1-cyano-5-((methylsulfonyl)methyl)pyrrolidin-3-yl)-5-(3-(trifluoromethyl)phenyl)-oxazole-2-carboxamide;
N-((3*R*,5*R*)-5-((1H-1,2,3-triazol-1-yl)methyl)-1-cyanopyrrolidin-3-yl)-5-(3-(trifluoromethyl)phenyl) oxazole-2-carboxamide;
N-((3*R*,5*R*)-1-cyano-5-(methoxymethyl)pyrrolidin-3-yl)-5-(2-cyclopropoxy-5-(trifluoromethyl)phenyl)-1,3,4-oxadiazole-2-carboxamide;
N-((3*R*,5*S*)-1-cyano-5-methylpyrrolidin-3-yl)-5-(2-cyclopropoxy-5-(trifluoromethyl)phenyl)-1,3,4-oxadiazole-2-carboxamide;
N-((3*R*,5*S*)-1-cyano-5-methylpyrrolidin-3-yl)-5-(3-(trifluoromethyl)phenyl)-1,3,4-oxadiazole-2-carboxamide;
N-((3*R*,5*R*)-1-cyano-5-(methoxymethyl)pyrrolidin-3-yl)-5-(3-(trifluoromethyl)phenyl)-1,3,4-oxadiazole-2-carboxamide;
N-((3*R*,5*R*)-1-cyano-5 -(methoxymethyl)pyrrolidin-3 -yl)-5 -(2-cyclopropoxy-5 - (trifluoromethyl)phenyl)-oxazole-2-carboxamide;
N-((3*R*,5*R*)-1-cyano-5-(methoxymethyl)pyrrolidin-3-yl)-5-(2-methoxy-5-(trifluoromethyl)phenyl)-1,3,4-oxadiazole-2-carboxamide; and
5-(2-chloro-5-(trifluoromethyl)phenyl)-N-((3*R*,5*R*)-1-cyano-5-(methoxymethyl)pyrrolidin-3-yl)-1,3,4-oxadiazole-2-carboxamide;
or a pharmaceutically acceptable salt thereof.

Pharmaceutical acceptable salts of the compounds of formula (I) include the acid addition and base salts (including di-salts) thereof.

Suitable acid addition salts are formed from acids which form non-toxic salts. Examples include the acetate, aspartate, benzoate, besylate, bicarbonate/carbonate, bisulfate, camsylate, citrate, edisylate, esylate, fumarate, gluceptate, gluconate, glucuronate, hibenzate, hydrochloride/chloride, hydrobromide/bromide, hydroiodide/iodide, hydrogen phosphate, isethionate, D- and L-lactate, malate, maleate, malonate, mesylate, methylsulfate, 2-napsylate, nicotinate, nitrate, orotate, palmate, phosphate, saccharate, stearate, succinate sulfate, D-and L-tartrate, and tosylate salts.

Suitable base salts are formed from bases which form non-toxic salts. Examples include the aluminium, ammonium, arginine, benzathine, calcium, choline, diethylamine, diolamine, glycine, lysine, magnesium, meglumine, olamine, potassium, sodium, tromethamine and zinc salts.

For a review on suitable salts, see Stahl and Wermuth, Handbook of Pharmaceutical Salts: Properties, Selection, and Use, Wiley-VCH, Weinheim, Germany (2002).

A pharmaceutical acceptable salt of a compound of formula (I) may be readily prepared by mixing together solutions of the compound of formula (I) and the desired acid or base, as appropriate. The salt may precipitate from solution and be collected by filtration or may be recovered by evaporation of the solvent.

Pharmaceutical acceptable solvates in accordance with the invention include hydrates and solvates wherein the solvent of crystallization may be isotopically substituted, e.g. DzO, acetone-d₆, DMSO-d₆.

Also, within the scope of the invention are clathrates, drug-host inclusion complexes wherein, in contrast to the aforementioned solvates, the drug and host are present in non-stoichiometric amounts. For a review of such complexes, see J. Pharm Sci, 64 (8), 1269-1288 by Haleblian (August 1975). Hereinafter all references to compounds of formula (I) include references to salts thereof and to solvates and clathrates of compounds of formula (I) and salts thereof.

The invention includes all polymorphs of the compounds of formula (I) as hereinbefore defined. So-called "prodrugs" of the compounds of formula (I) are for instance

certain derivatives of compounds of formula (I) which have little or no pharmacological activity themselves can, when metabolised upon administration into or onto the body, give rise to compounds of formula (I) having the desired activity. Such derivatives are referred to as "prodrugs".

Prodrugs in accordance with the invention can, for example, be produced by replacing appropriate functionalities present in the compounds of formula (I) with certain moieties known to those skilled in the art as "pro-moieties" as described, for example, in "Design of Prodrugs" by H Bundgaard (Elsevier, 1985).

Finally, certain compounds of formula (I) may themselves act as prodrugs of other compounds of formula (I).

Certain derivatives of compounds of formula (I) which contain a nitrogen atom may also form the corresponding *N*-oxide.

Included within the scope of the present invention are all tautomeric forms of the compounds of formula (I).

Conventional techniques for the preparation/isolation of individual enantiomers include chiral synthesis from a suitable optically pure precursor or resolution of the racemate (or the racemate of a salt or derivative) using, for example, chiral high performance liquid chromatography (HPLC). Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person. Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of propan-2-ol, typically from 2% to 20%, and from 0 to 5% by volume of an alkylamine, typically 0.1% diethylamine. Concentration of the eluate affords the enriched mixture. The present invention includes all crystal forms of the compounds of formula (I) including racemates and racemic mixtures (conglomerates) thereof. Stereoisomeric conglomerates may be separated by conventional techniques known to those skilled in the art - see, for example, "Stereochemistry of Organic Compounds" by E. L. Eliel and S. H. Wilen (Wiley, New York, 1994).

The compounds of formula (I) contain two chiral centres at the carbon atoms of the pyrrolidine ring that are substituted by R¹ and the amide and said stereocentres could exist in either the (*R*) or (*S*) configuration. The designation of the absolute configuration (*R*) and (*S*) for stereoisomers in accordance with IUPAC nomenclature is dependent on the nature of the substituents and application of the sequence-rule procedure. The compounds of formula (I) could, therefore, exist in four stereoisomeric forms.

The compounds of formula (I) of the present invention exist as a single stereoisomer. The pyrrolidine carbon atom of the amide substituent exists as the (*R*)-stereocentre, whereas the designation of the pyrrolidine carbon atom of the R¹ group is dependent on the nature of the substituent. The compound of formula (I) is isolated as a single stereoisomer and may exist with a stereoisomeric excess of at least 60%, preferably at least 80%, more preferably at least 90%, more preferably at least 95%, for example 96%, 97%, 98%, 99%, or 100%.

Additional chiral centres may exist in the compounds of formula (I) within the R¹ substituent itself. Included within the scope of the present invention are all such stereoisomeric forms of the compounds of formula (I).

The present invention also includes all pharmaceutically acceptable isotopic variations of a compound of formula (I). An isotopic variation is defined as one in which at least one atom is replaced by an atom having the same atomic number, but an atomic mass different from the atomic mass usually found in nature.

Examples of isotopes suitable for inclusion in the compounds of the invention include isotopes of hydrogen, such as ²H and ³H, carbon, such as ¹³C and ¹⁴C, nitrogen, such as ¹⁵N, oxygen, such as ¹⁷O and ¹⁸O, phosphorus, such as ³²P, sulfur, such as ³⁵S, fluorine, such as ¹⁸F, and chlorine, such as ³⁶Cl.

Substitution of the compounds of the invention with isotopes such as deuterium may afford certain therapeutic advantages resulting from greater metabolic stability, for example, increased in vivo half-life or reduced dosage requirements, and hence may be preferred in some circumstances.

Certain isotopic variations of the compounds of formula (I), for example, those incorporating a radioactive isotope, are useful in drug and/or substrate tissue distribution studies. The radioactive isotopes tritium, and ¹⁴C, are particularly useful for this purpose in view of their ease of incorporation and ready means of detection.

Isotopic variations of the compounds of formula (I) can generally be prepared by conventional techniques known to those skilled in the art or by processes analogous to those described in the accompanying Examples and Preparations using appropriate isotopic variations of suitable reagents.

The compounds of formula (I) are inhibitors of the deubiquitylating enzyme USP30.

According to a further aspect, the present invention provides a compound of formula (I) as defined herein, a tautomer thereof, or a pharmaceutically acceptable salt of said compound or tautomer for use as a medicament.

According to a further aspect, the present invention provides a method of treatment or prevention of a disorder or condition where inhibition of USP30 is known, or can be shown, to produce a beneficial effect, in a mammal, comprising administering to said mammal a therapeutically effective amount of a compound of formula (I) as defined herein, a tautomer thereof, or a pharmaceutically acceptable salt of said compound or tautomer.

According to a further aspect, the present invention provides the use of a compound of formula (I) as defined herein, a tautomer thereof, or a pharmaceutically acceptable salt of said compound or tautomer, in the manufacture of a medicament for the treatment or prevention of a disorder or condition where inhibition of USP30 is known, or can be shown, to produce a beneficial effect. The manufacture of a medicament may include, inter alia, the chemical synthesis of the compound of formula (I) or a salt thereof, or the preparation of a composition or formulation comprising the compound or salt, or the packaging of any medicament comprising the compound.

According to a further aspect, the present invention provides the compounds for use in a method of inhibition of USP30 in a patient comprising administering to the patient a therapeutically effective amount of a compound of formula (I) as defined herein, a tautomer thereof, or a pharmaceutically acceptable salt of said compound or tautomer.

The disorder or condition benefiting from USP30 activity is selected from a condition involving mitochondrial dysfunction, cancer and fibrosis.

In one preferred embodiment of all aspects of the invention, the disorder or condition benefiting from USP30 activity is a condition involving mitochondrial dysfunction.

Mitochondrial dysfunctions result from defects of the mitochondria, which are specialized compartments present in every cell of the body except red blood cells. When mitochondria fail, less and less energy is generated within the cell and cell injury or even cell death will follow. If this process is repeated throughout the body the life of the subject in whom this is happening is severely compromised. Diseases of the mitochondria appear most often in organs that are very energy demanding such as the brain, heart, liver, skeletal muscles, kidney and the endocrine and respiratory system.

The condition involving mitochondrial dysfunction may be selected from a condition involving a mitophagy defect, a condition involving a mutation in mitochondrial DNA, a condition involving mitochondrial oxidative stress, a condition involving a defect in mitochondrial membrane potential, mitochondrial biogenesis, a condition involving a defect in mitochondrial shape or morphology, and a condition involving a lysosomal storage defect.

In particular, the condition involving mitochondrial dysfunction may be selected from a neurodegenerative disease; multiple sclerosis (MS); mitochondrial encephalopathy, lactic acidosis and stroke-like episodes (MELAS) syndrome; materially-inherited diabetes and deafness (MIDD); Leber's hereditary optic neuropathy (LHON); cancer (including, for example, breast, ovarian, prostate, lung, kidney, gastric, colon, testicular, head and neck, pancreas, brain, melanoma, bone or other cancers of tissue organs and cancers of the blood cells, such as lymphoma and leukaemia, multiple myeloma, metastatic carcinoma, osteosarcoma, chondosarcoma, Ewing's sarcoma, nasopharyngeal carcinoma, colorectal cancer, and non-small cell lung carcinoma); neuropathy, ataxia, retinitis pigmentosa, maternally inherited Leigh syndrome (NARP-MILS); Danon disease; diabetes; diabetic nephropathy; metabolic disorders; heart failure; ischemic heart disease leading to myocardial infarction; psychiatric diseases, for example schizophrenia; multiple sulfatase deficiency (MSD); mucolipidosis II (ML II); mucolipidosis III (ML III); mucolipidosis IV (ML IV); GMl-gangliosidosis (GM1); neuronal ceroid-lipofuscinoses (NCL1); Alpers disease; Barth syndrome; beta-oxidation defects; carnitine-acyl-carnitine deficiency; carnitine deficiency; creatine deficiency syndromes; co-enzyme Q10 deficiency; complex I deficiency; complex II deficiency; complex III deficiency; complex IV deficiency; complex V deficiency; COX deficiency; chronic progressive external ophthalmoplegia syndrome (CPEO); CPT I deficiency; CPT II deficiency; glutaric aciduria type II; Kearns-Sayre syndrome; lactic acidosis; long-chain acyl-CoA dehydrogenase deficiency (LCHAD); Leigh disease or syndrome; Leigh syndrome French Canadian (LSFC) variant; lethal infantile cardiomyopathy (LIC); Luft disease; medium-chain acyl-CoA dehydrogenase deficiency (MCAD); myoclonic epilepsy and ragged-red fiber (MERRF) syndrome; mitochondrial cytopathy; mitochondrial recessive ataxia syndrome; mitochondrial DNA depletion syndrome; myoneurogastointestinal disorder and encephalopathy; Pearson syndrome; pyruvate dehydrogenase deficiency; pyruvate carboxylase deficiency; POLG mutations; medium/short-chain 3-hydroxyacyl-CoA dehydrogenase (M/SCHAD) deficiency; very long-chain acyl-CoA dehydrogenase (VLCAD) deficiency; peroxisomal disorders; methylmalonic acidemia; mevalonate kinase deficiency; age-dependent decline in cognitive function and muscle strength; and cognitive impairment associated with neurodegenerative and neuropsychiatric disorders.

The condition involving mitochondrial dysfunction may be a CNS disorder, for example a neurodegenerative disease.

Neurodegenerative diseases include, but are not limited to, Parkinson's disease, Alzheimer's disease, amyotrophic lateral sclerosis (ALS), Huntington's disease, ischemia, stroke, dementia with Lewy bodies, multiple system atrophy (MSA), progressive supranuclear palsy (PSP), corticobasal degeneration (CBD), and frontotemporal dementia.

In particular, the compounds of the invention may be useful in the treatment or prevention of Parkinson's disease, including, but not limited to, PD related to mutations in α-synuclein, parkin, PINK1, GBA, and LRRK2, and autosomal recessive juvenile Parkinson's disease (AR-JP) or early onset Parkinson's disease (EOPD), where parkin or PINK1 is mutated, truncated or deleted.

In particular, the compounds of the invention may be useful in treatment of cognitive impairment associated with neurodegenerative and neuropsychiatric disorders, including, for example, cognitive impairment associated with Alzheimer's disease and Parkinson's disease, preclinical or prodromal forms of AD and PD, Huntington's disease, dementia with lewy body disease, cognitive impairment associated with schizophrenia, mood disorders, bipolar and major depressive disorders.

The compounds of the invention or pharmaceutical compositions thereof as described herein may be combined with one or more additional agents when used for the treatment or prevention of conditions involving mitochondrial dysfunction. The compounds may be combined with one or more additional agents selected from levodopa, a dopamine agonist, a monoamino oxygenase (MAO) B inhibitor, a catechol O-methyltransferase (COMT) inhibitor, an anticholinergic, riluzole, amantadine, a cholinesterase inhibitor, memantine, tetrabenazine, an antipsychotic, diazepam, clonazepam, an antidepressant, and an anti-convulsant. The compounds may be combined with agents which reduce/remove pathogenic protein aggregates in neurodegenerative diseases, such as agents which reduce/remove alpha-synuclein in Parkinson's disease, multiple system atrophy or dementia with Lewy bodies; agents which reduce/remove Tau in Alzheimer's disease or progressive supranuclear palsy; agents which reduce/remove TDP-43 in ALS or frontotemporal dementia.

In another preferred embodiment of all aspects of the invention, the disorder or condition benefiting from USP30 activity is cancer. The cancer may be linked to mitochondrial dysfunction. Preferred cancers include, for example, breast, ovarian, prostate, lung, kidney, gastric, colon, testicular, head and neck, pancreas, brain, melanoma, bone or other cancers of tissue organs and cancers of the blood cells, such as lymphoma and leukaemia, multiple myeloma, metastatic carcinoma, osteosarcoma, chondosarcoma, Ewing's sarcoma, nasopharyngeal carcinoma, colorectal cancer, colorectal cancer, and non-small cell lung carcinoma.

In particular, the compounds of the invention may be useful in the treatment or prevention of cancer where apoptotic pathways are dysregulated and more particularly where proteins of the BCL-2 family are mutated, or over or under expressed.

Fibrosis refers to the accumulation of extracellular matrix constituents that occurs following trauma, inflammation, tissue repair, immunological reactions, cellular hyperplasia, and neoplasia. Fibrotic disorders that may be treated by the compounds and compositions of the present invention include, inter alia, fibrosis/fibrotic disorders associated with major organ diseases, for example, interstitial lung disease (ILD), liver cirrhosis, non-alcoholic fatty liver disease (NAFLD) and non-alcoholic steatohepatitis (NASH) (hepatic fibrosis), kidney disease (renal fibrosis), acute kidney injury (AKI), acute kidney disease (AKD), chronic kidney disease (CKD), delayed kidney graft function, heart or vascular disease (cardiac fibrosis) and diseases of the eye; fibroproliferative disorders, for example, systemic and local scleroderma, keloids and hypertrophic scars, atherosclerosis, restenosis, and Dupuytren's contracture; scarring associated with trauma, for example, surgical complications, chemotherapeutics drug-induced fibrosis (e.g. bleomycin-induced fibrosis), radiation-induced fibrosis, accidental injury and burns); retroperitoneal fibrosis (Ormond's disease); and peritoneal fibrosis/peritoneal scarring in patients receiving peritoneal dialysis, usually following renal transplantation. See, for example, Wynn et al, 2004, Nat Rev Immunol. August; 4(8): 583-594. The present invention therefore relates to the compounds for use in methods of treatment or prevention, and compounds and compositions used in said methods, of fibrosis/fibrotic disorders of and/or associated with the major organs, including for example, the lung, liver, kidney, heart, skin, eye, gastrointestinal tract, peritoneum and bone marrow, and other diseases/disorders herein described.

The compounds may be combined with agents which are used as treatments for kidney disease, including anti-diabetic agents, cardiovascular disease agents, and novel agents targeting disease relevant pathways such as oxidative stress (including, but not limited to, the nrf2/keap-1 pathway) and anti-apoptotic pathways (including, but not limited to, anti p53 agents).

Interstitial lung disease (ILD) includes disorders in which pulmonary inflammation and fibrosis are the final common pathways of pathology, for example, sarcoidosis, silicosis, drug reactions, infections and collagen vascular diseases, such as rheumatoid arthritis and systemic sclerosis (scleroderma). The fibrotic disorder of the lung includes, for example, pulmonary fibrosis, idiopathic pulmonary fibrosis (IPF), usual interstitial pneumonitis (UIP), interstitial lung disease, cryptogenic fibrosing alveolitis (CFA), bronchiolitis obliterans, and bronchiectasis.

Idiopathic pulmonary fibrosis (IPF) is the most common type of ILD and has no known cause.

The compounds may be combined with agents which are treatments for IPF and potentially for ILD, including nintedanib and pirfenidone.

Liver cirrhosis has similar causes to ILD and includes, for example, cirrhosis associated with viral hepatitis, schistosomiasis and chronic alcoholism.

Kidney disease may be associated with diabetes, which can damage and scar the kidneys leading to a progressive loss of function, and also hypertensive diseases. Kidney fibrosis may occur at any stage of kidney disease, from acute kidney disease (AKD) post injury and chronic kidney disease (CKD), such as incident CKD and progressive CKD, through to end-stage renal disease (ESRD). Kidney fibrosis can develop as a result of cardiovascular disease such as hypertension or diabetes, both of which place immense strain on kidney function which promotes a fibrotic response. However, kidney fibrosis can also be idiopathic (without a known cause), and certain genetic mitochondrial diseases also present kidney fibrosis manifestations and associated symptoms.

Heart disease may result in scar tissue that can impair the ability of the heart to pump.

Diseases of the eye include, for example, macular degeneration and retinal and vitreal retinopathy, which can impair vision.

In a preferred embodiment, the present invention is directed to the treatment or prevention of idiopathic pulmonary fibrosis (IPF).

In another preferred embodiment, the present invention is directed to the treatment or prevention of kidney fibrosis.

In another preferred embodiment, the present invention is directed to the treatment or prevention of acute kidney injury (AKI), especially in high risk patients. Examples include post-surgical AKI, for example organ transplantation, such as due to ischemia reperfusion injury, delayed graft function; oncology, such as AKI due to chemotherapy; contrast medium-induced nephropathy, such as direct-tubular cytotoxicity, hemodynamic ischemia and osmotic effects; acute interstitial nephritis, such as due to drugs or infection; AKI due to obstruction such as kidney stones; and COVID-19-induced AKI. A particular high risk patient sub-group are those undergoing cardiac surgery, for example, coronary artery bypass graft and/or valve surgery. There are established static risk factors for AKI such as age 65 years or over, insulin dependent diabetes, CKD (adults with an estimated glomerular filtration rate [eGFR] less than 60 ml/min/1.73 m2 are at particular risk), heart failure, liver disease, history of AKI.

In another preferred embodiment, the present invention is directed to the treatment or prevention of acute kidney disease (AKD) or chronic kidney disease (CKD) stemming from such AKI, including for example, tubulointerstitial fibrosis and diabetic nephropathy.

In another preferred embodiment, the present invention is directed to the treatment or prevention of liver diseases, including but not limited to NAFLD, NASH, liver cirrhosis, portal hypertension, acute liver failure, and hepatocellular carcinoma. Liver disease such as NAFLD and NASH may be associated with various metabolic conditions such as metabolic syndrome and Type II diabetes, which also would increase risk for various diabetes associated pathologies, including diabetic retinopathy and peripiheral neuropathies.

The compounds of the invention or pharmaceutical compositions thereof as described herein may be combined with one or more additional agents when used for the treatment or prevention of conditions involving liver disease and metabolic dysfunction, including metformin, sulfonylureas, DPP-4 inhibitors, GLP-1 agonists, PPAR agonists, SGLT2 inhibitors, angiotensin-converting enzyme (ACE) inhibitors and angiotensin II receptor blockers (ARBs).

Leigh syndrome is a rare inherited neurometabolic disorder that affects the central nervous system. This progressive disorder begins in infants between the ages of three months and two years. Rarely, it occurs in teenagers and adults. Leigh syndrome can be caused by mutations in nuclear DNA encoding for mitochondrial proteins, mutations in mitochondrial DNA (maternally inherited Leigh syndrome - MILS), or by deficiencies of an enzyme called pyruvate dehydrogenase located on the short arm of the X Chromosome (X-linked Leigh syndrome). Symptoms of Leigh syndrome usually progress rapidly. The earliest signs may be poor sucking ability, and the loss of head control and motor skills. These symptoms may be accompanied by loss of appetite, vomiting, irritability, continuous crying, and seizures. As the disorder progresses, symptoms may also include generalized weakness, lack of muscle tone, and episodes of lactic acidosis, which can lead to impairment of respiratory and kidney function.

In maternally inherited Leigh syndrome (MILS), genetic mutations in mitochondrial DNA (at a high proportion of >90%) interfere with the energy sources that run cells in an area of the brain that plays a role in motor movements. Genetic mutations in mitochondrial DNA result in a chronic lack of energy in these cells, which in turn affects the central nervous system and causes progressive degeneration of motor functions. When the genetic mutations in mitochondrial DNA that causes MILS are less abundant (less than 90%), the condition is known as neuropathy ataxia and retinitis pigmentosa (NARP). There is also a form of Leigh's disease (called X-linked Leigh's disease) which is the result of mutations in a gene that produces another group of substances that are important for cell metabolism. A further variant of Leigh syndrome exists which is called French Canadian variant, characterized by mutations in a gene called LRPPRC. Similar neurological symptoms are expressed as those for Leigh syndrome, although Liver Steatosis is commonly also observed in the French Canadian variant.

In a preferred embodiment, the present invention is directed to the treatment or prevention of Leigh syndrome or disease, including for example, X-linked Leigh's disease, Leigh syndrome French Canadian variant, and/or the symptoms associated with Leigh's disease.

The compounds may be combined with novel agents which may be used as treatments for mitochondrial disease, including, but not limited to, nicotinamide riboside.

References to 'treatment' includes means to ameliorate, alleviate symptoms, eliminate the causation of the symptoms either on a temporary or permanent basis. The compounds of the invention are useful in the treatment of the diseases disclosed herein in humans and other mammals.

In another embodiment, the invention encompasses prophylactic therapy of the diseases disclosed herein and includes means to prevent or slow the appearance of symptoms of the named disorder or condition. The compounds of the invention are useful in the prevention of the diseases disclosed herein in humans and other mammals.

A patient in need of treatment or prevention may, for example, be a human or other mammal suffering from the condition or at risk of suffering from the condition.

According to a further aspect, the present invention provides a pharmaceutical composition comprising a compound of formula (I) as defined herein, or a pharmaceutically acceptable salt of said compound or tautomer, together with a pharmaceutically acceptable diluent or carrier.

Pharmaceutical compositions of the invention comprise any of the compounds of the invention combined with any pharmaceutically acceptable carrier, adjuvant or vehicle. Examples of pharmaceutically acceptable carriers are known to those skilled in the art and include, but are not limited to, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms. The compositions may be in the form of, for example, tablets, capsules, powders, granules, elixirs, lozenges, suppositories, syrups and liquid preparations including suspensions and solutions. The term "pharmaceutical composition" in the context of this invention means a composition comprising an active agent and comprising additionally one or more pharmaceutically acceptable carriers. The composition may further contain ingredients selected from, for example, diluents, adjuvants, excipients, vehicles, preserving agents, fillers, disintegrating agents, wetting agents, emulsifying agents, suspending agents, sweetening agents, flavouring agents, perfuming agents, antibacterial agents, antifungal agents, lubricating agents and dispersing agents, depending on the nature of the mode of administration and dosage forms.

The compounds of the invention or pharmaceutical compositions thereof, as described herein, may be used alone or combined with one or more additional pharmaceutical agents. The compounds may be combined with an additional anti-tumour therapeutic agent, for example, chemotherapeutic drugs or inhibitors of other regulatory proteins. In one embodiment, the additional anti-tumour therapeutic agent is a BH-3 mimetic. In a further embodiment, BH-3 mimetics may be selected from but not limited to one or more of ABT-737, ABT-199, ABT-263, and Obatoclax. In a further embodiment, the additional anti-tumour agent is a chemotherapeutic agent. Chemotherapeutic agents may be selected from but not limited to, olaparib, mitomycin C, cisplatin, carboplatin, oxaliplatin, ionizing radiation (IR), camptothecin, irinotecan, topotecan, temozolomide, taxanes, 5-fluoropyrimidines, gemcitabine, and doxorubicin.

For the treatment or prevention of fibrotic disorders, for example, the compounds of the invention or pharmaceutical compositions thereof, as described herein, may be used alone or combined with one or more additional pharmaceutical agents selected from the group consisting of anticholinergic agents, beta-2 mimetics, steroids, PDE-IV inhibitors, p38 MAP kinase inhibitors, NK1 antagonists, LTD4 antagonists, EGFR inhibitors and endothelin antagonists.

In particular, the compounds of the invention or pharmaceutical compositions thereof, as described herein, may be used alone or combined with one or more additional pharmaceutical agents selected from the group consisting of general immunosuppressive drugs, such as a corticosteroid, immunosuppressive or cytotoxic agents, or antifibrotics, such as pirfenidone or a non-specific kinase inhibitor (e.g. nintedanib).

The pharmaceutical compositions of the invention may be administered in any suitably effective manner, such as oral, parenteral, topical, inhaled, intranasal, rectal, intravaginal, ocular and aural. Pharmaceutical compositions suitable for the delivery of compounds of the present invention and methods for their preparation will be readily apparent to those skilled in the art. Such compositions and methods for their preparation may be found, for example, in "Remington's Pharmaceutical Sciences", 19th Edition (Mack Publishing Company, 1995).

### Oral Administration

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as tablets, capsules containing particulates, liquids, or powders, lozenges (including liquid-filled), chews, multi-and nano-particulates, gels, films (including muco- adhesive), ovules, sprays, and liquid formulations.

Liquid formulations include suspensions, solutions, syrups, and elixirs. Such formulations may be employed as fillers in soft or hard capsules and typically comprise a carrier, for example, water, ethanol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

A typical tablet may be prepared using standard processes known to a formulation chemist, for example, by direct compression, granulation (dry, wet, or melt), melt congealing, or extrusion. The tablet formulation may comprise one or more layers and may be coated or uncoated.

Examples of excipients suitable for oral administration include carriers, for example, cellulose, calcium carbonate, dibasic calcium phosphate, mannitol and sodium citrate, granulation binders, for example, polyvinylpyrrolidine, hydroxypropylcellulose, hydroxypropylmethylcellulose and gelatin, disintegrants, for example, sodium starch glycolat and silicates, lubricating agents, for example, magnesium stearate and stearic acid, wetting agents, for example, sodium lauryl sulfate, preservatives, anti-oxidants, flavours and colourants.

Solid formulations for oral administration may be formulated to be immediate and/or modified release. Modified release formulations include delayed-, sustained-, pulsed-, controlled dual-, targeted and programmed release. Details of suitable modified release technologies such as high energy dispersions, osmotic and coated particles are to be found in Verma et al, Pharmaceutical Technology On-line, 25 (2), 1-14 (2001). Other modified release formulations are described in US Patent No. 6,106,864.

### Parenteral Administration

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by suitable processing, for example, the use of high energy spray-dried dispersions and/or by the use of appropriate formulation techniques, such as the use of solubility-enhancing agents.

Formulations for parenteral administration may be formulated to be immediate and/or modified release. Modified release formulations include delayed, sustained, pulsed, controlled dual, targeted and programmed release.

Pharmaceutical compositions of the present invention also include compositions and methods known in the art for bypassing the blood brain barrier or can be injected directly into the brain. Suitable areas for injection include the cerebral cortex, cerebellum, midbrain, brainstem, hypothalamus, spinal cord and ventricular tissue, and areas of the PNS including the carotid body and the adrenal medulla.

### Dosage

The magnitude of an effective dose of a compound will, of course, vary with the nature of the severity of the condition to be treated and the route of administration. The selection of appropriate dosages is within the remit of the physician. The daily dose range is about 10µg to about 100 mg per kg body weight of a human and non-human animal and in general may be around 10µg to 30mg per kg body weight per dose. The above dose may be given from one to three times per day.

For example, oral administration may require a total daily dose of from 5mg to 1000mg, such as from 5 to 500mg, while an intravenous dose may only require from 0.01 to 30 mg/kg body weight, such as from 0.1 to 10 mg/kg, more preferably from 0.1 to 1 mg/kg body weight. The total daily dose may be administered in single or divided doses.

The skilled person will also appreciate that, in the treatment or prevention of certain conditions, compounds of the invention may be taken as a single dose on an "as required" basis (i.e. as needed or desired).

### Synthetic methodologies

Compounds of formula (I) may be prepared using methods as described below in the general reaction schemes and the representative examples. Where appropriate, the individual transformations within a scheme may be completed in a different order. The invention is illustrated by the following non-limiting examples in which the following abbreviations and definitions are used. Compounds were characterised by liquid chromatography-mass spectroscopy (LCMS) or ¹H NMR or both.

According to a further aspect, the present invention provides a process for the preparation of a compound of formula (I)(i) comprising reacting a compound of formula (IV), where Y is OH with an amine of formula (V)(i), where PG is a protecting group, such as BOC or CBZ, to give an amide of formula (III)(i) (Scheme 1). The amide-coupling reaction can be performed using standard methodology, for example by reaction using a coupling reagent such as DCC, HATU, HBTU, EDC or via a mixed anhydride. Alternatively, the acid (IV), where Y is OH, can be converted into the acid chloride (IV), where Y is Cl, using SOCl₂, PCl₃, or PCl₅, which can then be reacted with the amine (V)(i), preferably in a suitable solvent in the presence of a suitable base. Alternatively, the compound (IV), where Y forms the ester, can be reacted directly with the amine (V)(i), preferably in a suitable solvent.

The compound of formula (III)(i) may be deprotected using standard methods to give amine (II)(i) which may then be reacted with cyanogen bromide to give the corresponding compound of formula (I)(i).

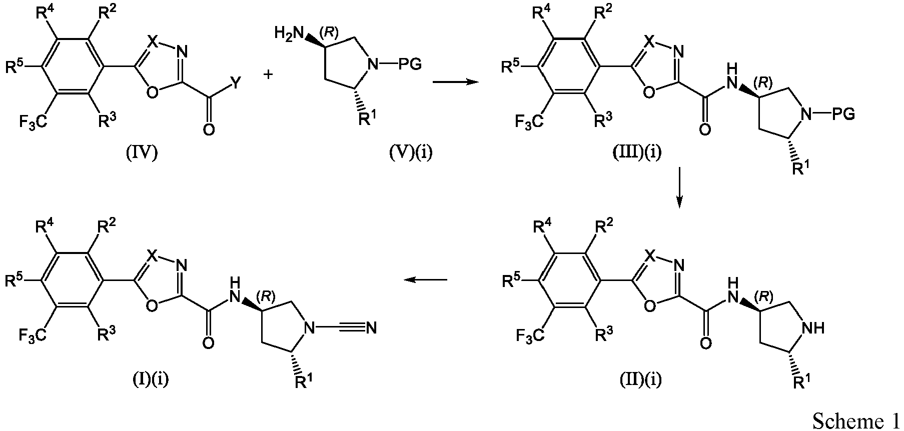

In a further aspect, the present invention provides a compound, which is selected from formulae (II)(i) and (III)(i): 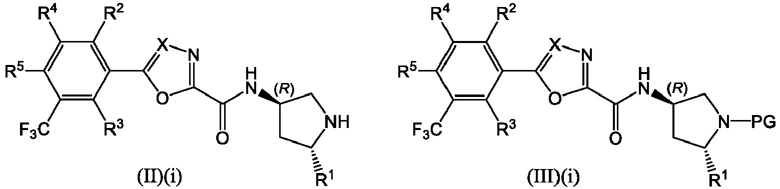 wherein PG is a protecting group, preferably BOC or CBZ, and X, R¹, R², R³, R⁴ and R⁵ are as defined herein for the compound of formula (I) and preferred embodiments thereof, a tautomer thereof, or a salt of said compound or tautomer. When X is CH, the compounds of formulae (II)(i), (III)(i) and (IV) are (IIA)(i), (IIIA)(i) and (IVA), respectively. When X is N, the compounds of formulae (II)(i), (III)(i) and (IV) are (IIB)(i), (IIIB)(i) and (IVB)(i), respectively.

According to a further aspect, the present invention provides a process for the preparation of a compound of formula (I)(ii) comprising reacting a compound of formula (IV), where Y is OH with an amine of formula (V)(ii), where PG is a protecting group, such as BOC or CBZ, to give an amide of formula (III)(ii) (Scheme 2). The amide-coupling reaction can be performed using standard methodology, for example by reaction using a coupling reagent such as DCC, HATU, HBTU, EDC or via a mixed anhydride. Alternatively, the acid (IV), where Y is OH, can be converted into the acid chloride (IV), where Y is Cl, using SOCh, PCl₃, or PCl₅, which can then be reacted with the amine (V)(ii), preferably in a suitable solvent in the presence of a suitable base. Alternatively, the compound (IV), where Y forms the ester, can be reacted directly with the amine (V)(ii), preferably in a suitable solvent.

The compound of formula (III)(ii) may be deprotected using standard methods to give amine (II)(ii) which may then be reacted with cyanogen bromide to give the corresponding compound of formula (I)(ii).

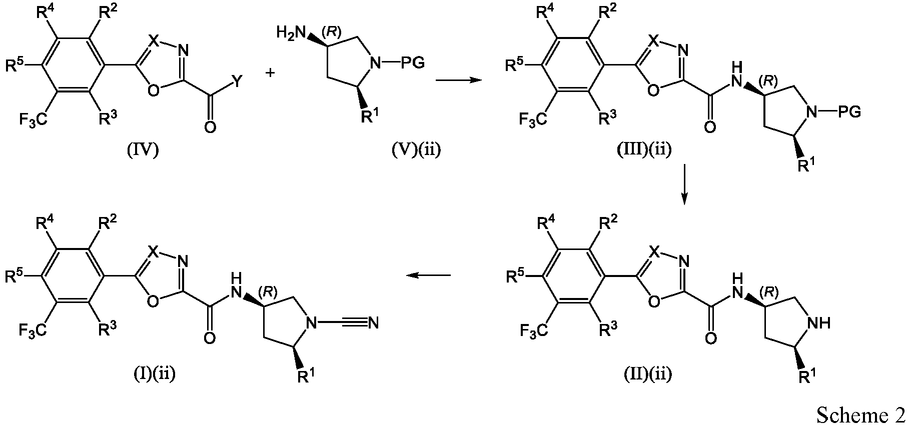

In a further aspect, the present invention provides a compound, which is selected from formulae (II)(ii) and (III)(ii): 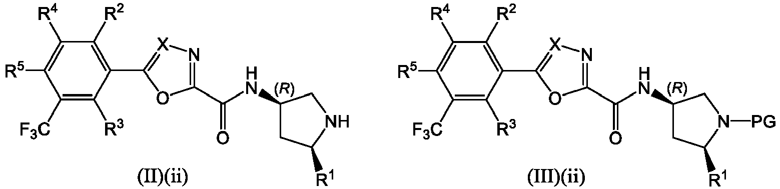 wherein PG is a protecting group, preferably BOC or CBZ, and X, R¹, R², R³, R⁴ and R⁵ are as defined herein for the compound of formula (I) and preferred embodiments thereof, a tautomer thereof, or a salt of said compound or tautomer. When X is CH, the compounds of formulae (II)(ii), (III)(ii) and (IV) are (IIA)(ii), (IIIA)(ii) and (IVA), respectively. When X is N, the compounds of formulae (II)(ii), (III)(ii) and (IV) are (IIB)(ii), (IIIB)(ii) and (IVB)(ii), respectively.

Protecting groups are preferably selected from tert-butyloxycarbonyl (BOC), benzyloxycarbonyl (Cbz), p-methoxybenzyl carbonyl (MeOZ), 9-fluorenylmethyloxycarbonyl (Fmoc), acetyl (Ac), benzoyl (Bz), benzyl (Bn), carbamate, p-methoxybenzyl (PMB), 3,4-dimethoxybenzyl (DMPM), p-methoxyphenyl (PMP), tosyl (Ts), trichloroethoxycarbonyl (Troc), 4-nitrobenzenesulfonyl (Nosyl) and 2-nitrophenylsulfenyl (Nps). Most preferred are BOC and Cbz.

### Abbreviations

| | | | |
|---|---|---|---|
| br s | broad singlet (NMR signal) | MeOH | methanol |
| CO | carbon monoxide | min | minute(s) |
| d | doublet (NMR signal) | N₂ | nitrogen |
| dba | dibenzylacetone | NMP | *N*-methylpyrrolidone |
| DCM | dichloromethane | *rac* | racemic |
| DMF | *N*,*N*-dimethylformamide | rt | room temperature |
| DMSO | dimethylsulfoxide | s | singlet (NMR signal) |
| ES | electrospray | SFC | Supercritical fluid chromatogaphy |
| EtOAc | ethyl acetate | SOCl₂ | thionyl chloride |
| h | hour(s) | TBD | 1,5,7-triazabicyclo[4.4.0]dec-5-ene |
| H₂ | hydrogen | TEA | triethylamine |
| m | multiplet (NMR signal) | TFA | trifluoroacetic acid |
| MsCl | methanesulfonyl chloride | THF | tetrahydrofuran |
| MeCN | acetonitrile | TsCl | 4-toluenesulfonyl chloride |
| | | vol | volumes |
| HPLC | High performance liquid chromatography | LCMS | Liquid chromatography - mass spectrometry |

LCMS / HPLC / SFC Methods

### Method C

| | | | |
|---|---|---|---|
| Mobile phase | (A) | 2 mM Ammonium acetate & 0.1% formic acid in water | |
| | (B) | 0.1% Formic acid in acetonitrile | |
| Instrument | | Waters ACQUITY H Class with PDA and SQ Detector | |
| Column | | BEH C18 (50 mm x 2.1 mm) 1.7 µm | |
| Flow rate | | 0.550 mL/min | |
| Column oven temperature | | Ambient | |
| Run time | | 3.0 min | |

| Gradient | | | |
|---|---|---|---|
| TIME (min) | Flow Rate (mL/min) | %A | %B |
| 0.01 | 0.55 | 98 | 2 |
| 0.30 | 0.55 | 98 | 2 |
| 0.60 | 0.55 | 50 | 50 |
| 1.10 | 0.55 | 25 | 75 |
| 2.00 | 0.60 | 0 | 100 |
| 2.70 | 0.60 | 0 | 100 |
| 2.71 | 0.55 | 98 | 2 |
| 3.00 | 0.55 | 98 | 2 |

### Method C1

| | | | |
|---|---|---|---|
| Mobile phase | (A) | 2 mM Ammonium acetate & 0.1% formic acid in water | |
| | (B) | 0.1% Formic acid in acetonitrile | |
| Instrument | | Waters ACQUITY UPLC H Class with PDA and SQ detector | |
| Column | | BEH C18 (50 mm x 2.1 mm) 1.7 µm | |
| Flow rate | | 0.550 mL/min | |
| Column oven temperature | | Ambient | |
| Run time | | 2.0 min | |

| Gradient | | | |
|---|---|---|---|
| TIME (min) | Flow Rate (mL/min) | %A | %B |
| 0.00 | 0.55 | 95 | 5 |
| 0.60 | 0.60 | 30 | 70 |
| 0.80 | 0.65 | 10 | 90 |
| 1.10 | 0.65 | 0 | 100 |
| 1.70 | 0.65 | 0 | 100 |
| 1.71 | 0.55 | 95 | 5 |
| 2.00 | 0.55 | 95 | 5 |

### Method H

| | | | |
|---|---|---|---|
| Mobile phase | (A) | 5 mM Ammonium bicarbonate in water | |
| | (B) | 100% Acetonitrile | |
| Instrument | | Shimadzu Nexera UFLC with 2020 single quadrupole mass detector | |
| Column | | Waters X-Bridge C18 (50 x 4.6 mm) 3.5 µm | |
| Column oven temperature | | Ambient | |
| Flow rate | | 1.0 mL/min | |
| Run time | | 8.0 min | |

| Gradient | | | |
|---|---|---|---|
| TIME (min) | Flow Rate (mL/min) | %A | %B |
| 0.01 | 1.0 | 95 | 5 |
| 3.50 | 1.0 | 10 | 90 |
| 4.50 | 1.0 | 5 | 95 |
| 6.00 | 1.0 | 5 | 95 |
| 6.01 | 1.0 | 95 | 5 |
| 8.00 | 1.0 | 95 | 5 |

### Method H1

| | | | |
|---|---|---|---|
| Mobile phase | (A) | 5 mM Ammonium bicarbonate in water | |
| | (B) | 100% Acetonitrile | |
| Instrument | | Shimadzu Nexera UFLC with 2020 single quadrupole mass detector | |
| Column | | Waters X-Bridge C18 (50 x 4.6 mm) 3.5 µm | |
| Column oven temperature | | Ambient | |
| Flow rate | | 1.0 mL/min | |
| Run time | | 6.0 min | |

| Gradient | | | |
|---|---|---|---|
| TIME (min) | Flow Rate (mL/min) | %A | %B |
| 0.01 | 1.0 | 95 | 5 |
| 2.80 | 1.0 | 15 | 85 |
| 3.50 | 1.0 | 5 | 95 |
| 5.00 | 1.0 | 5 | 95 |
| 5.01 | 1.0 | 95 | 5 |
| 6.00 | 1.0 | 95 | 5 |

### Method H3

| | | | |
|---|---|---|---|
| Mobile phase | (A) | 5 mM Ammonium bicarbonate in water | |
| | (B) | 100% Acetonitrile | |
| Instrument | | Shimadzu Nexera High Presure UHPLC and LCMS-2020 | |
| Column | | X-Bridge C18 (50 x 4.6 mm) 3.5 µm | |
| Column oven temperature | | Ambient | |
| Flow rate | | 1.0 mL/min | |
| Run time | | 6.0 min | |

| Gradient | | | |
|---|---|---|---|
| TIME (min) | Flow Rate (mL/min) | %A | %B |
| 0.01 | 1.0 | 95 | 5 |
| 2.80 | 1.0 | 15 | 85 |
| 3.50 | 1.0 | 5 | 95 |
| 5.00 | 1.0 | 5 | 95 |
| 5.01 | 1.0 | 95 | 5 |
| 6.00 | 1.0 | 95 | 5 |

### Method J

| | | | |
|---|---|---|---|
| Mobile phase | (A) | 2 mM Ammonium acetate & 0.1% formic acid in water | |
| | (B) | 0.1% Formic acid in acetonitrile | |
| Instrument | | Waters ACQUITY H Class with PDA and SQ detector | |
| Column | | BEH C18 (50 mm x 2.1 mm) 1.7 µm | |
| Flow rate | | 0.450 mL/min | |
| Column oven temperature | | Ambient | |
| Run time | | 8.0 min | |

| Gradient | | | |
|---|---|---|---|
| TIME (min) | Flow Rate (mL/min) | %A | %B |
| 0.01 | 0.45 | 98 | 2 |
| 0.50 | 0.45 | 98 | 2 |
| 5.00 | 0.45 | 10 | 90 |
| 6.00 | 0.45 | 5 | 95 |
| 7.00 | 0.45 | 5 | 95 |
| 7.01 | 0.45 | 98 | 2 |
| 8.00 | 0.45 | 98 | 2 |

### Method M

| | | | |
|---|---|---|---|
| Mobile phase | (A) | 10 mM Ammonium acetate in water | |
| | (B) | 100 % Acetonitrile | |
| Instrument | | Agilent 1290 Infinity RRLC attached with Agilent 6120 Mass detector and Diode array Detector | |
| Column | | Waters XSelect, Phenyl-Hexyl (250 mm x 4.6 mm) 5 µm | |
| Flow rate | | 1.0 mL/min | |
| Column oven temperature | | Ambient | |
| Run time | | 35.0 min | |

| Gradient | | | |
|---|---|---|---|
| TIME (min) | Flow Rate (mL/min) | %A | %B |
| 0.00 | 1.0 | 90 | 10 |
| 10.0 | 1.0 | 60 | 40 |
| 20.0 | 1.0 | 30 | 70 |
| 30.0 | 1.0 | 0 | 100 |
| 33.0 | 1.0 | 0 | 100 |
| 33.01 | 1.0 | 90 | 10 |
| 35.0 | 1.0 | 90 | 10 |

### Method 13

| | | | |
|---|---|---|---|
| Mobile phase | (A) | 0.1% v/v (30% v/v aqueous ammonia) in water | |
| | (B) | 0.1% v/v (30% v/v aqueous ammonia) in acetonitrile | |
| Instrument | | Waters e2695 HPLC with PDA detector | |
| Column | | X-bridge C8 (250 x 4.6 mm), 5 µm | |
| Column oven temperature | | 25 °C | |
| Flow rate | | 1.0 mL/min | |
| Run time | | 40.0 min | |

| Gradient | | | |
|---|---|---|---|
| TIME (min) | Flow Rate (mL/min) | %A | %B |
| 0.00 | 1.0 | 95 | 5 |
| 5.00 | 1.0 | 95 | 5 |
| 10.00 | 1.0 | 70 | 30 |
| 15.00 | 1.0 | 70 | 30 |
| 25.00 | 1.0 | 40 | 60 |
| 30.00 | 1.0 | 10 | 90 |
| 35.00 | 1.0 | 10 | 90 |
| 35.01 | 1.0 | 95 | 5 |
| 40.00 | 1.0 | 95 | 5 |

### Method Y3 Method used for analytical chiral SFC

| | | | |
|---|---|---|---|
| Mobile Phase | (A) | Liquid carbon dioxide | |
| | (B) | 0.1% Diethylamine in propan-2-ol : acetonitrile (50:50) | |
| Instrument | | Waters SFC investigator and PDA detector | |
| Column | | Chiralcel OJ-H (250 x 4.6 mm), 5 µm | |
| Flow rate | | 4.0 mL/min | |
| Run time | | 7.0 min | |
| Gradient | | | |

| TIME (min) | Flow Rate (mL/min) | %B start | %B end |
|---|---|---|---|
| 0 to 5 | 4.0 | 5 | 5 |
| 5 to 7 | 4.0 | 50 | 50 |

### Method Y4 Method used for analytical chiral SFC

| Mobile Phase | (A) | Liquid carbon dioxide | |
|---|---|---|---|
| | (B) | 0.1% Diethylamine in propan-2-ol : acetonitrile (50:50) | |
| Instrument | | Waters SFC investigator and PDA detector | |
| Column | | Chiralpak IH (250 × 4.6 mm), 5 µm | |
| Flow rate | | 4.0 mL/min | |
| Run time | | 10.0 min | |
| Gradient | | | |

| TIME (min) | Flow Rate (mL/min) | %B start | %B end |
|---|---|---|---|
| 0 to 5 | 4.0 | 5 | 5 |
| 5 to 10 | 4.0 | 50 | 50 |

### Method Y5 Method used for analytical chiral SFC

| Mobile Phase | (A) | Liquid carbon dioxide | |
|---|---|---|---|
| | (B) | 0.1% Diethylamine in propan-2-ol : acetonitrile (50:50) | |
| Instrument | | Waters SFC investigator and PDA detector | |
| Column | | Chiralpak AD-H (250 x 4.6 mm), 5 µm | |
| Flow rate | | 4.0 mL/min | |
| Run time | | 10.0 min | |
| Gradient | | | |

| TIME (min) | Flow Rate (mL/min) | %B start | %B end |
|---|---|---|---|
| 0 to 5 | 4.0 | 5 | 5 |
| 5 to 10 | 4.0 | 50 | 50 |

### Method Y8 Method used for analytical chiral SFC

| Mobile Phase | (A) | Liquid carbon dioxide | |
|---|---|---|---|
| | (B) | 0.1% Diethylamine in propan-2-ol : acetonitrile (50:50) | |
| Instrument | | Waters SFC investigator and PDA detector | |
| Column | | Chiralpak IC (250 x 4.6 mm), 5 µm | |
| Flow rate | | 4.0 mL/min | |
| Run time | | 10.0 min | |
| Gradient | | | |

| TIME (min) | Flow Rate (mL/min) | %B start | %B end |
|---|---|---|---|
| 0 to 5 | 4.0 | 5 | 5 |
| 5 to 10 | 4.0 | 50 | 50 |

### Method Y9 Method used for analytical chiral SFC

| Mobile Phase | (A) | Liquid carbon dioxide | |
|---|---|---|---|
| | (B) | 0.1% Diethylamine in propan-2-ol : acetonitrile (50:50) | |
| Instrument | | Waters SFC investigator and PDA detector | |
| Column | | Chiralpak IB-N (250 x 4.6 mm), 5 µm | |
| Flow rate | | 4.0 mL/min | |
| Run time | | 10.0 min | |
| Gradient | | | |

| TIME (min) | Flow Rate (mL/min) | %B start | %B end |
|---|---|---|---|
| 0 to 5 | 4.0 | 5 | 5 |
| 5 to 10 | 4.0 | 50 | 50 |

### Method Y10 Method used for analytical chiral HPLC

| Mobile Phase | (A) | 0.1% Diethylamine in n-hexane | |
|---|---|---|---|
| | (B) | 0.1% Diethylamine in propan-2-ol : acetonitrile (70-30) | |
| Instrument | | Agilent 1260 Series HPLC and PDA detector | |
| Column | | Chiralpak IH (250 × 4.6 mm), 5 µm | |
| Flow rate | | 1.0 mL/min | |
| Run time | | 25.0 min | |
| Gradient | | | |

| TIME (min) | Flow Rate (mL/min) | %A | %B |
|---|---|---|---|
| 0.01 | 1.0 | 80 | 20 |
| 5.00 | 1.0 | 50 | 50 |
| 10.00 | 1.0 | 30 | 70 |
| 20.00 | 1.0 | 30 | 70 |
| 20.01 | 1.0 | 80 | 20 |
| 25.00 | 1.0 | 80 | 20 |

### Method Y13

| Mobile Phase | (A) | Liquid carbon dioxide | |
|---|---|---|---|
| | (B) | 0.1% Diethylamine in propan-2-ol : acetonitrile (50:50) | |
| Instrument | | Waters SFC Investigator and PDA detector | |
| Column | | Chiralpak IC (250 x 4.6 mm), 5 µm | |
| Flow rate | | 4.0 mL/min | |
| Run time | | 8.0 min | |
| Gradient | | | |

| TIME (min) | Flow Rate (mL/min) | %B | %B |
|---|---|---|---|
| 0 to 5 | 4.0 | 5 | 50 |
| 5 to 8 | 4.0 | 50 | 50 |

### Synthesis of Intermediates

### Intermediate A

### Ethyl 5-(3-(trifluoromethyl)phenyl) oxazole-2-carboxylate

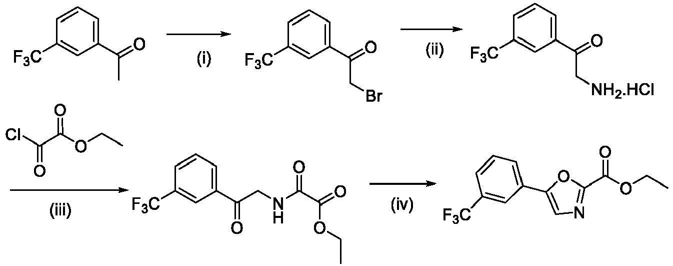

(i) Br₂, DCM, 0 °C to rt, 5 h; (ii) NaN(CHO)₂, MeCN, 80 °C, 1 h, then MeOH, *conc.* HCl, 80 °C, 2 h; (iii) K₂CO₃, DCM, 0 °C to rt, 2 h; (iv) POCl₃, 105 °C, 5 h.

### Step (i)

### 2-Bromo-1-(3-(trifluoromethyl)phenyl)ethan-1-one

This reaction was performed in duplicate. To a stirred solution of 1-(3-(trifluoromethyl)phenyl)ethan-1-one (CAS 349-76-8, from Combi-blocks, 100.0 g, 531.49 mmol) in DCM (9000 mL) was added a solution of bromine (84.93 g, 27.39 mL, 531.49 mmol) in DCM (1000 mL) dropwise at 0 °C. The mixture was slowly warmed to rt and stirred for 5 h. The mixtures from both reactions were combined and poured into saturated NaHCO₃ solution (8000 mL) and extracted with DCM (4 × 5000 mL). The combined organic phases were dried over Na₂SO₄ and concentrated under reduced pressure. The residue were suspended in MeOH (1000 mL), stirred at -78 °C for 30 min to form a precipitate, which was filtered off and collected with suction to afford 2-bromo-1-(3-(trifluoromethyl)phenyl)ethan-1-one (280.0 g, 1048.49 mmol, 98% yield). ¹H NMR (400 MHz, DMSO-d₆) δ ppm: 8.29 - 8.31 (m, 2H), 8.07 (d, *J* = 8.0 Hz, 1H), 7.83 (t, *J* = 7.6 Hz, 1H), 5.06 (s, 2H).

### Step (ii)

### 2-Amino-1-(3-(trifluoromethyl)phenyl)ethan-1-one hydrochloride

To a stirred solution of 2-bromo-1-(3-(trifluoromethyl)phenyl)ethan-1-one (276.0 g, 1033.51 mmol) in MeCN (2750 mL) was added sodium diformylamide (147.32 g, 1550.27 mmol). The reaction mixture was heated at 80 °C for 1 h then concentrated under reduced pressure. The residue was diluted with MeOH (900 mL) and *conc.* HCl (680 mL). The mixture was then heated at 80 °C for 2 h, and allowed to cool to rt and the mixture was concentrated under reduced pressure. The residue was stirred with isopropyl alcohol (1800 mL) to form a precipitate which was filtered off and collected with suction, washed with isopropyl alcohol (200 mL) and dried under reduced pressure to afford 2-amino-1-(3-(trifluoromethyl)phenyl)ethan-1-one hydrochloride (223.0 g, 930.48 mmol, 90% yield).

LCMS: Method H, 2.59 min, MS: ES+ 204.0.

### Step (iii)

### Ethyl 2-oxo-2-((2-oxo-2-(3-(trifluoromethyl)phenyl) ethyl)amino)acetate

To a stirred solution of 2-amino-1-(3-(trifluoromethyl)phenyl)ethan-1-one HCl salt (285.0 g, 1189.18 mmol) in DCM (3000 mL) was added K₂CO₃ (410.26 g, 2972.95 mmol) at 0 °C. Ethyl oxalyl chloride (243.48 g, 199.25 mL, 1783.77 mmol) was added dropwise at 0 °C over a time period of 2 h. The mixture was allowed to warm to rt and stirred for 2 h, then poured into ice-cold water (2000 mL) and extracted with DCM (2 × 1000 mL). The combined organic phases were dried over Na₂SO₄ and concentrated under reduced pressure to afford ethyl 2-oxo-2-((2-oxo-2-(3-(trifluoromethyl)phenyl)ethyl)amino)acetate (120.0 g, 395.72 mmol, 33% yield). This material was used directly in the next step without purification.

LCMS: Method H, 3.07 min, MS: ES- 301.9.

### Step (iv)

### Ethyl 5-(3-(trifluoromethyl)phenyl)oxazole-2-carboxylate

A stirred solution of ethyl 2-oxo-2-((2-oxo-2-(3-(trifluoromethyl)phenyl)ethyl)-amino)acetate (120.0 g, 395.72 mmol) in POCl₃ (600 mL, 5 vol) was heated at 105 °C for 5 h. The reaction mixture was cooled to rt, then slowly poured on to crushed ice (5 kg), the mixture was basified with solid NaHCO₃ and extracted with ethyl acetate (3 × 4000 mL). The combined organic phases were dried over Na₂SO₄ and concentrated under reduced pressure. The residue was suspended in MeOH (1500 mL) and stirred at -78 °C for 0.5 h to form a precipitate, which was filtered and collected with suction to afford ethyl 5-(3-(trifluoromethyl)phenyl)oxazole-2-carboxylate (85 g, 298.01 mmol, 75.30% yield).

LCMS: Method H, 3.61 min, MS: ES+ 286.15; ¹H NMR (400 MHz, DMSO-*d₆*) δ ppm: 8.22 (s, 1H), 8.17 (s, 1H), 8.13 (d, *J* = 7.6 Hz, 1H), 7.80 - 7.86 (m, 2H), 4.40 - 4.45 (m, 2H), 1.37 (t, *J* = 7.2 Hz, 3H).

### Intermediate B

### Ethyl 5-(2-cyclopropoxy-5-(trifluoromethyl)phenyl)-1,3,4-oxadiazole-2-carboxylate

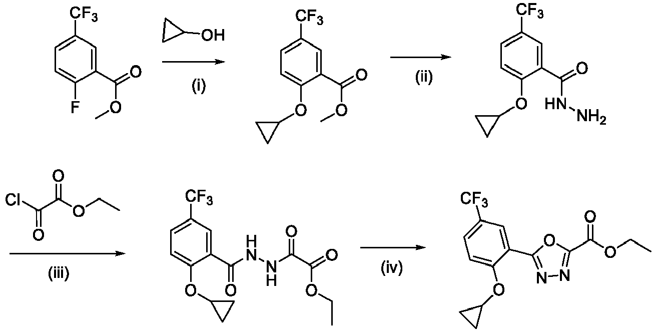

(i) NaH, THF, 0 °C, 3 h; (ii) NH₂NH₂.H₂O, MeOH, 0 °C to rt, 16 h; (iii) K₂CO₃, DCM, 0 °C to rt, 2 h; (iv) TsCl, TEA, DCM, 0 °C to rt, 2 h.

### Step (i)

### Methyl 2-cyclopropoxy-5-(trifluoromethyl)benzoate

This reaction was performed in triplicate. To a stirred solution of cyclopropanol (CAS 16545-68-9, from Synthonix, 0.52 g, 9.0 mmol) in THF (8 mL) was added NaH (60% in oil, 0.36 g, 9.0 mmol) in portions at 0 °C and stirred for 30 min. A solution of methyl-2-fluoro-5-(trifluoromethyl)benzoate (CAS 556112-92-6, from Oakwood, 1.0 g, 4.50 mmol) in THF (2 mL) was added dropwise and the mixture was stirred at 0 °C for 3 h. The three reaction mixtures were combined and poured into ice-cold water (200 mL) and extracted with EtOAc (2 x 200 mL). The combined organic phases were dried over Na₂SO₄ and concentrated under reduced pressure to afford methyl 2-cyclopropoxy-5-(trifluoromethyl)benzoate (2.1 g, 8.07 mmol, 60% yield). This material was directly used in the next step.

LCMS: Method H, 3.92 min, MS: ES+ 261.0.

### Step (ii)

### 2-Cyclopropoxy-5-(trifluoromethyl)benzohydrazide

To a stirred solution of methyl 2-cyclopropoxy-5-(trifluoromethyl)benzoate (2.1 g, 8.07 mmol) in MeOH (20 mL) was added hydrazine hydrate (99%, 1.0 g, 20.19 mmol) dropwise at 0 °C. The mixture was slowly warmed to rt and stirred for 16 h, then concentrated under reduced pressure to yield 2-cyclopropoxy-5-(trifluoromethyl)benzohydrazide (2.0 g, 7.68 mmol, 95% yield). This material was directly used in the next step.

¹H NMR (400 MHz, DMSO-*d₆*) *δ* ppm 9.21 (s, 1H), 7.82 - 7.86 (m, 2H), 7.60 (d, *J* = 8.4 Hz, 1H), 4.56 (br s, 2H), 4.00 - 4.08 (m, 1H), 0.73 - 0.91 (m, 4H).

### Step (iii)

### Ethyl 2-(2-(2-cyclopropoxy-5-(trifluoromethyl)benzoyl)hydrazineyl)-2-oxoacetate

To a stirred solution of 2-cyclopropoxy-5-(trifluoromethyl)benzohydrazide (2.0 g, 7.68 mmol) in DCM (20 mL) was added K₂CO₃ (3.18 g, 23.07 mmol) at 0 °C. Ethyl oxalyl chloride (1.56 g, 11.53 mmol) was added dropwise at 0 °C. The mixture was allowed to warm to rt, stirred for 2 h, then poured into water (100 mL) and extracted with EtOAc (2 × 100 mL). The combined organic phases were dried over Na₂SO₄ and concentrated under reduced pressure to afford ethyl 2-(2-(2-cyclopropoxy-5-(trifluoromethyl)benzoyl)hydrazineyl)-2-oxoacetate (3.0 g, quantitative yield). This crude material was directly used in the next step.

LCMS: Method H, 2.32 min, MS: ES- 359.0.

### Step (iv)

### Ethyl 5-(2-cyclopropoxy-5-(trifluoromethyl)phenyl)-1,3, 4-oxadiazole-2-carboxylate

To a stirred solution of ethyl 2-(2-(2-cyclopropoxy-5-(trifluoromethyl)benzoyl)hydrazineyl)-2-oxoacetate (3.0 g, 8.33 mmol) in DCM (30 mL) was added TEA (1.68 g, 2.3 mL, 16.66 mmol) at rt. Tosyl chloride (1.58 g, 8.33 mmol) was added at 0 °C. The mixture was allowed to warm to rt, stirred for 2 h, then poured into water (100 mL) and extracted with EtOAc (2 × 100 mL). The combined organic phases were dried over Na₂SO₄ and concentrated under reduced pressure. The residue was purified by flash column chromatography (silica gel, 15% EtOAc in *n*-hexanes) to afford ethyl 5-(2-cyclopropoxy-5-(trifluoromethyl)phenyl)-1,3,4-oxadiazole-2-carboxylate (1.2 g, 3.51 mmol, 46% yield over two steps).

LCMS: Method H, 3.67 min, MS: ES+ 343.1.

### Intermediate C

### Ethyl 5-(3-(trifluoromethyl)phenyl)-1,3, 4-oxadiazole-2-carboxylate

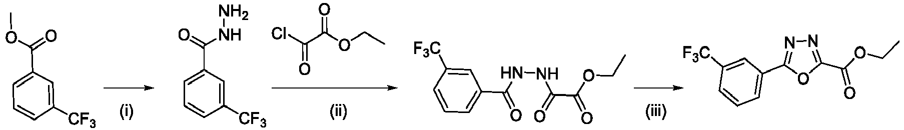

(i) NH₂NH₂.H₂O, MeOH, 0 °C to rt, 16 h; (ii) K₂CO₃, DCM, 0 °C to rt, 2 h; (iii) TsCl, TEA, DCM, 0 °C to rt, 2 h.

### Step (i)

### 3-(Trifluoromethyl)benzohydrazide

To a stirred solution of methyl 3-(trifluoromethyl)benzoate (CAS 2557-13-3, from Combi-blocks, 3.0 g, 14.70 mmol) in MeOH (30 mL) was added hydrazine hydrate (99%, 1.83 g, 36.76 mmol) dropwise at 0 °C. The mixture was slowly warmed to rt and stirred for 16 h, then concentrated under reduced pressure. The residue was poured into water (300 mL) and extracted with EtOAc (2 × 300 mL). The combined organic phases were dried over Na₂SO₄ and concentrated under reduced pressure to yield 3-(trifluoromethyl)benzohydrazide (3.4 g, quantitative yield).

LCMS: Method C, 1.33 min, MS: ES+ 204.9.

### Step (ii)

### Ethyl 2-oxo-2-(2-(3-(trifluoromethyl)benzoyl)hydrazineyl)acetate

To a stirred solution of 3-(trifluoromethyl)benzohydrazide (3.4 g, 16.66 mmol) in DCM (40 mL) was added K₂CO₃ (6.89 g, 49.98 mmol) at rt. Ethyl oxalyl chloride (3.41 g, 2.79 mL, 24.99 mmol) was added dropwise at 0 °C. The mixture was allowed to warm to rt, stirred for 2 h, then poured into water (500 mL) and extracted with EtOAc (2 × 400 mL). The combined organic phases were dried over Na₂SO₄ and concentrated under reduced pressure to afford ethyl 2-oxo-2-(2-(3-(trifluoromethyl)benzoyl)hydrazineyl)acetate (6.0 g, quantitative yield).

LCMS: Method C, 1.43 min, MS: ES+ 305.0.

### Step (iii)

### Ethyl 5-(3-(trifluoromethyl)phenyl)-1,3,4-oxadiazole-2-carboxylate

To a stirred solution of ethyl 2-oxo-2-(2-(3-(trifluoromethyl)benzoyl)hydrazineyl)acetate (6.0 g, 19.73 mmol) in DCM (60 mL) was added TEA (3.98 g, 5.5 mL, 39.46 mmol) and tosyl chloride (3.76 g, 19.73 mmol) at 0 °C. The mixture was allowed to warm to rt, stirred for 2 h, then poured into water (500 mL) and extracted with DCM (2 × 300 mL). The combined organic phases were dried over Na₂SO₄ and concentrated under reduced pressure. The residue was purified by flash column chromatography (silica gel, 15% EtOAc in n-hexanes) to afford ethyl 5-(3-(trifluoromethyl)phenyl)-1,3,4-oxadiazole-2-carboxylate (2.0 g, 6.99 mmol, 47% yield over three steps).

LCMS: Method C, 1.71 min, MS: ES+ 286.8.

### Intermediate D

### Ethyl-5-(2-cyclopropoxy-5-(trifluoromethyl)phenyl)oxazole-2-carboxylate

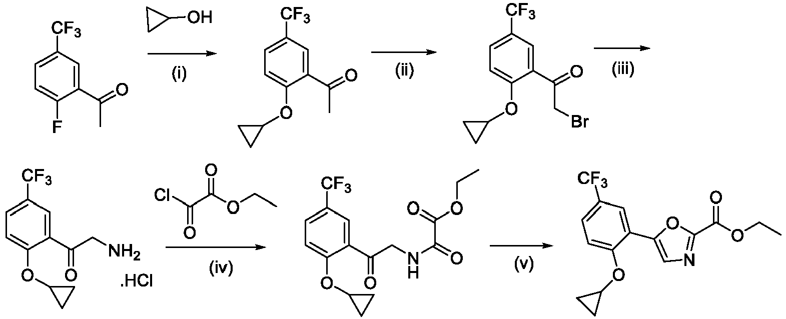

(i) NaH, THF, 0 °C, 2 h; (ii) phenyl trimethylammonium tribromide, rt, 1.5 h; (iii) NaN(CHO)₂, MeCN, 80 °C, 1.5 h, then MeOH, *conc.* HCl, 80 °C, 1.5 h; (iv) K₂CO₃, DCM, 0 °C, 1 h; (v) POCl₃, 100 °C, 2 h.

### Step (i)

### 1-(2-Cyclopropoxy-5-(trifluoromethyl)phenyl)ethan-1-one

To a stirred solution of cyclopropanol (CAS 16545-68-9, from Synthonix, 0.56 g, 9.70 mmol) in THF (8 mL) was added NaH (60% in mineral oil, 0.23 g, 9.70 mmol) at 0 °C in portions. A solution of 1-(2-fluoro-5-(triffuoromethyl)phenyl)ethan-1-one (CAS 202664-53-7, from Combi-blocks, 1.0 g, 4.85 mmol) in THF (2 mL) was added dropwise at 0 °C. The mixture was stirred at 0 °C for 2 h, then poured into water (100 mL) and extracted with EtOAc (2 x 100 mL). The combined organic phases were dried over Na₂SO₄ and concentrated under reduced pressure. The residue was purified by flash column chromatography (silica gel, 6.9 % EtOAc in n-hexanes) to yield 1-(2-cyclopropoxy-5-(trifluoromethyl)phenyl)ethan-1-one (0.95 g, 3.89 mmol, 80% yield).

¹H NMR (400 MHz, CDCl₃) δ ppm: 8.04 (d, *J* = 2.0 Hz, 1H), 7.74 - 7.76 (m, 1H), 7.48 (d, *J* = 8.8 Hz, 1H), 3.90 - 3.95 (m, 1H), 2.61 (s, 3H), 0.87 - 0.98 (m, 4H).

### Step (ii)

### 2-Bromo-1-(2-cyclopropoxy-5-(trifluoromethyl)phenyl)ethan-1-one

To a stirred solution of 1-(2-cyclopropoxy-5-(trifluoromethyl)phenyl)ethan-1-one (1.60 g, 6.56 mmol) in THF (10 mL) was added phenyl trimethylammonium tribromide (2.71 g, 7.21 mmol) in portions at rt. The mixture was stirred at rt for 1.5 h, then poured into water (100 mL) and extracted with EtOAc (2 x 150 mL). The combined organic phases were dried over Na₂SO₄ and concentrated under reduced pressure. The residue was purified by flash column chromatography (silica gel, 1.8% EtOAc in n-hexanes) to yield 2-bromo-1-(2-cyclopropoxy-5-(trifluoromethyl)phenyl)ethan-1-one (1.58 g, 4.91 mmol, 74% yield). ¹H NMR (400 MHz, CDCl₃) δ ppm: 8.13 - 8.16 (m, 1H), 7.82 (t, *J* = 8.8 Hz, 1H), 7.52 (d, *J* = 7.2 Hz, 1H), 4.52 (s, 2H), 3.97 - 4.00 (m, 1H), 0.97 - 0.99 (m, 4H).

### Step (iii)

### 2-Amino-1-(2-cyclopropoxy-5-(trifluoromethyl)phenyl)ethan-1-one hydrochloride

To a stirred suspension of 2-bromo-1-(2-cyclopropoxy-5-(trifluoromethyl)phenyl)ethan-1-one (1.50 g, 4.66 mmol) in MeCN (15 mL) was added sodium diformylamide (0.53 g, 5.59 mmol) at rt. The mixture was heated at 80 °C for 1.5 h, then allowed to cool to rt and concentrated under reduced pressure. The residue was diluted with MeOH (15 mL) and *conc.* HCl (1.5 mL). The mixture was heated again at 80 °C for 1.5 h, then allowed to cool to rt. The mixture was concentrated under reduced pressure, the residue was stirred with diethyl ether (2 x 10 mL) to form a precipitate, which was collected by filtration under reduced pressure to afford 2-amino-1-(2-cyclopropoxy-5-(trifluoromethyl)phenyl)ethan-1-one HCl salt (0.91 g, 3.08 mmol, 66% yield). LCMS: Method C, 1.39 min, MS: ES+: 260.1.

### Step (iv)

### Ethyl 2-((2-(2-cyclopropoxy-5-(trifluoromethyl)phenyl)-2-oxoethyl)amino)-2-oxoacetate

To a stirred suspension of 2-amino-1-(2-cyclopropoxy-5-(trifluoromethyl)phenyl)ethan-1-one HCl salt (0.90 g, 3.04 mmol) in DCM (10 mL) was added potassium carbonate (1.68 g, 12.18 mmol) and ethyl oxalyl chloride (0.83 g, 6.09 mmol) dropwise at 0 °C. The mixture was stirred at 0 °C for 1 h, then poured into water (50 mL) and extracted with EtOAc (2 × 100 mL). The combined organic phases were dried over Na₂SO₄ and concentrated under reduced pressure to afford ethyl 2-((2-(2-cyclopropoxy-5-(trifluoromethyl)phenyl)-2-oxoethyl)amino)-2-oxoacetate (0.44 g, 1.24 mmol, 40% yield).

LCMS: Method C, 1.72 min, MS: ES+: 360.4.

### Step (v)

### Ethyl-5-(2-cyclopropoxy-5-(trifluoromethyl)phenyl)oxazole-2-carboxylate

A solution of ethyl 2-((2-(2-cyclopropoxy-5-(trifluoromethyl)phenyl)-2-oxoethyl)amino)-2-oxoacetate (0.44 g, 1.22 mmol) in POCl₃ (4 mL, 10 volumes) was heated at 100 °C for 2 h. The mixture was cooled to rt and poured into crushed ice and extracted with EtOAc (2 × 100 mL). The combined organic phases were dried over Na₂SO₄ and concentrated under reduced pressure. The residue were suspended in MeOH (10 mL) and stirred at -78 °C for 10 min. The solid was collected by filtration under reduced pressure to afford ethyl 5-(2-cyclopropoxy-5-(trifluoromethyl)phenyl)oxazole-2-carboxylate (0.14 g, 0.42 mmol, 34% yield).

LCMS: Method C, 1.96 min, MS: ES+: 342.0.

### Intermediate E

### Ethyl 5-(2-methoxy-5-(trifluoromethyl)phenyl)-1,3,4-oxadiazole-2-carboxylate

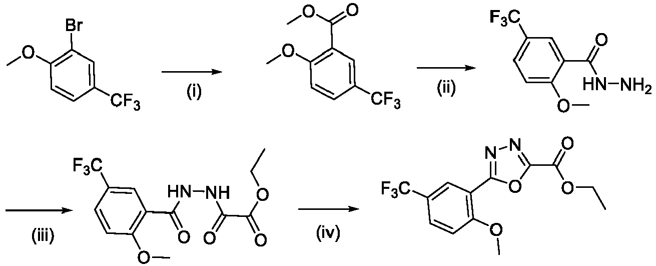

(i) CO gas, PdCl₂(dppf)DCM complex, MeOH, NaOAc, 70 °C 16 h; (ii) hydrazine hydrate, MeOH, 0 °C to rt, 16 h; (iii) ethyl oxalyl chloride, K₂CO₃, DCM, 0 °C to rt, 1 h;
(iv) TsCl, TEA, DCM, 0 °C to rt, 3 h.

### Step (i)

### Methyl 2-methoxy-5-(trifluoromethyl)benzoate

To a stirred solution of 2-bromo-1-methoxy-4-(trifluoromethyl)benzene (CAS 402-10-8, 3.0 g, 11.76 mmol) in MeOH, in autoclave, was added NaOAc (4.82 g, 58.82 mmol) and the mixture was degassed and purged with N₂ for 20 minutes.

[1,1'-Bis(diphenylphosphino)ferrocene]dichloropalladium(II), complex with DCM (1.91 g, 2.35 mmol) was added. The mixture was heated at 70 °C for 16 h under CO (g) pressure. The mixture was allowed to cool to rt and concentrated under reduced pressure. The residue was poured into water (50 mL) and extracted with ethyl acetate (3 x 50 mL). The combined organic phases were dried over Na₂SO₄, filtered and concentrated under reduced pressure. The residue was purified by flash column chromatography (silica gel, 10% EtOAc in n-hexanes) to yield methyl 2-methoxy-5-(trifluoromethyl)benzoate (0.63 g, 2.69 mmol, 22% yield).

LCMS: Method C, 1.31 min, MS: no mass ion consistent with product observed.

### Step (ii)

### 2-Methoxy-5-(trifluoromethyl)benzohydrazide

To a stirred solution of methyl 2-methoxy-5-(trifluoromethyl)benzoate (0.60 g, 2.56 mmol) in MeOH (6 mL) was added hydrazine hydrate (0.3 mL, 6.41 mmol) at 0 °C. The mixture was allowed to warm to rt and stirred for 16 h, then concentrated under reduced pressure to yield 2-methoxy-5-(trifluoromethyl)benzohydrazide (0.57 g, 2.43 mmol, 95% yield).

LCMS: Method J, 3.41 min, MS: ES+ 234.8.

### Step (iii)

### Ethyl 2-(2-(2-methoxy-5-(trifluoromethyl)benzoyl)hydrazineyl)-2-oxoacetate

To a stirred solution of 2-methoxy-5-(trifluoromethyl)benzohydrazide (0.54 g, 2.31 mmol) in DCM (5.4 mL) was added K₂CO₃ (0.63 g, 4.61 mmol) and followed by ethyl oxalyl chloride (0.52 mL, 4.61 mmol) dropwise at 0 °C. The mixture was allowed to warm to rt and stirred for 1 h, then poured into water (50 mL) and extracted with DCM (3 × 150 mL). The combined organic phases were dried over Na₂SO₄, filtred and concentrated to yield ethyl 2-(2-(2-methoxy-5-(trifluoromethyl)benzoyl)-hydrazineyl)-2-oxoacetate (0.62 g, 1.85 mmol, 80% yield).

LCMS: Method J, 3.98 min, MS: ES+ 335.0.

### Step (iv)

### Ethyl 5-(2-methoxy-5-(trifluoromethyl)phenyl)-1,3,4-oxadiazole-2-carboxylate

To a stirred solution of ethyl 2-(2-(2-methoxy-5-(trifluoromethyl)benzoyl)hydrazineyl)-2-oxoacetate (0.58 g, 1.73 mmol) in DCM (5.8 mL) was added TEA (0.75 mL, 5.21 mmol) and TsCl (0.33 g, 1.73 mmol) in portions at 0 °C. The mixture was allowed to warm to rt and stirred for 3 h, then poured into water (20 mL) and extracted with EtOAc (3 × 150 mL). The combined organic phases were dried over Na₂SO₄, and concentrated under reduced pressure, then the residue was purified using flash column chromatography (silica gel, 25% EtOAc in *n*-hexanes) to yield ethyl 5-(2-methoxy-5-(trifluoromethyl)phenyl)-1,3,4-oxadiazole-2-carboxylate (0.3 g, 0.95 mmol, 54% yield).

LCMS: Method J, 3.98 min, MS: ES+ 317.0.

### Intermediate F

### Ethyl 5-(2-chloro-5-(trifluoromethyl)phenyl)-1,3,4-oxadiazole-2-carboxylate

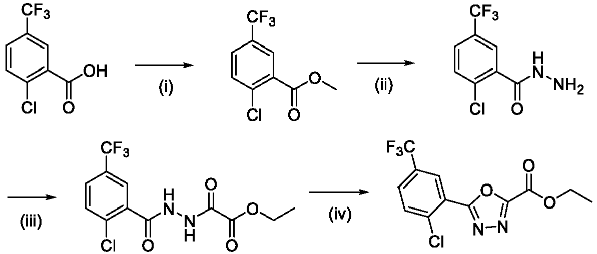

(i) SOCl₂, MeOH, 0 °C to 70 °C 5 h; (ii) hydrazine hydrate, MeOH, 0 °C to rt, 24 h; (iii) ethyl oxalyl chloride, K₂CO₃, DCM, 0 °C to rt, 1 h; (iv) TsCl, TEA, DCM, 0 °C to rt, 2 h.

### Step (i)

### Methyl 2-chloro-5-(trifluoromethyl)benzoate

To a stirred solution of 2-chloro-5-(trifluoromethyl)benzoic acid (CAS 657-06-7, from Combi-blocks, 2.0 g, 8.93 mmol) in MeOH (20 mL) was added SOCl₂ (1.58 g, 13.39 mmol) dropwise at 0 °C. The mixture was heated at 70 °C for 5 h, then allowed to cool to rt and poured into water (100 mL) and extracted with EtOAc (2 × 100 mL). The combined organic phases were dried over anhydrous Na₂SO₄ and concentrated under reduced pressure to yield methyl 2-chloro-5-(trifluoromethyl)benzoate (2.1 g, 8.82 mmol, 98% yield). This crude material was directly used in the next step. ¹H NMR (400 MHz, DMSO-*d₆*) δ ppm: 8.15 (s, 1H), 7.96 -7.98 (m, 1H), 7.84 - 7.86 (m, 1H), 3.90 (s, 3H).

### Step (ii)

### 2-Chloro-5-(trifluoromethyl)benzohydrazide

To a stirred solution of methyl 2-chloro-5-(trifluoromethyl)benzoate (1.5 g, 6.30 mmol) in MeOH (10 mL) was added hydrazine hydrate (3 mL, 2 vol) dropwise at 0 °C. The mixture was allowed to warm to rt and stirred for 24 h, then poured into water (100 mL) and extracted with DCM (2 x 100 mL). The combined organic phases were dried over anhydrous Na₂SO₄, filtered and concentrated under reduced pressure to yield 2-chloro-5-(trifluoromethyl)benzohydrazide (1.9 g, quantitative yield). LCMS: Method C, 1.33 min, MS: ES+ no mass ion consistent with product was observed.

### Step (iii)

### Ethyl 2-(2-(2-chloro-5-(trifluoromethyl)benzoyl)hydrazineyl)-2-oxoacetate

To a stirred solution of 2-chloro-5-(trifluoromethyl)benzohydrazide (1.9 g, 7.98 mmol) in DCM (15 mL) was added K₂CO₃ (4.41 g, 31.93 mmol) at 0 °C. Ethyl oxalyl chloride (2.18 g, 1.8 mL, 15.96 mmol) was added dropwise at 0 °C. The mixture was allowed to warm to rt, stirred for 1 h, then poured into water (100 mL) and extracted with DCM (2 x 100 mL). The combined organic phases were dried over Na₂SO₄ and concentrated under reduced pressure to afford ethyl 2-(2-(2-chloro-5-(trifluoromethyl)benzoyl)hydrazineyl)-2-oxoacetate (1.2 g, 3.55 mmol, 56% yield over two steps).

LCMS: Method C, 1.12 min, MS: ES+ 339.1.

### Step (iv)

### Ethyl 5-(2-chloro-5-(trifluoromethyl)phenyl)-1,3,4-oxadiazole-2-carboxylate

To a stirred solution of ethyl 2-(2-(2-chloro-5-(trifluoromethyl)benzoyl)hydrazineyl)-2-oxoacetate (1.1 g, 3.25 mmol) in DCM (10 mL) was added TEA (0.98 g, 1.36 mL, 9.75 mmol) at rt. TsCl (0.74 g, 3.90 mmol) was added at 0 °C. The mixture was allowed to warm to rt and stirred for 2 h, then poured into water (100 mL) and extracted with DCM (2 x 80 mL). The combined organic phases were dried over Na₂SO₄ and concentrated under reduced pressure. The residue was purified by flash column chromatography (silica gel, 26% EtOAc in *n*-hexanes) to afford ethyl 5-(2-chloro-5-(trifluoromethyl)phenyl)-1,3,4-oxadiazole-2-carboxylate (0.8 g, 2.50 mmol, 70% yield).

LCMS: Method C1, 1.33 min, MS: ES+ 321.2.

### Intermediate G

### tert-Butyl (2S,4R)-4-amino-2-((methylsulfonyl)methyl)pyrrolidine-1-carboxylate

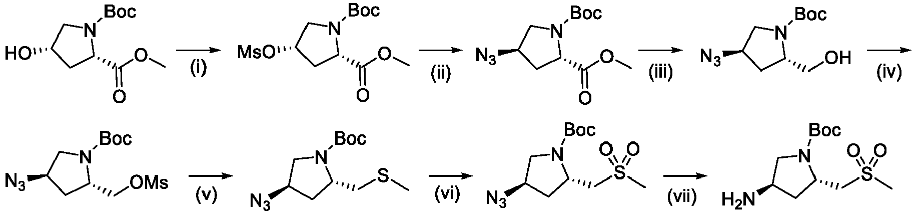

(i) MsCl, TEA, DCM, 0 °C, 1 h; (ii) sodium azide, DMF, 0°C to 80 °C, 4 h; (iii) 3 M LiBH₄ in THF, THF, -30 °C to rt, 3 h; (iv) MsCl, TEA, DCM, -10 °C to rt, 3 h; (v) NaSMe (15% in water), MeOH, rt, 16 h; (vi) Oxone, MeOH: water, 0 °C to rt, 1 h; (vii) 10% Pd/C (50% moist), MeOH, Hz, rt, 2 h.

### Step (i)

### 1-(tert-Butyl) 2-methyl (2S,4S)-4-((methylsulfonyl)ozη)pyrrolidine-1,2-dicarboxylate

This reaction was performed in duplicate. To a stirred solution of 1-(*tert*-butyl) 2-methyl (2*S*,4*S*)-4-hydroxypyrrolidine-1,2-dicarboxylate, (CAS 102195-79-9, from Pharmablock, 500 g, 2038.3 mmol, 1.0 eq) in DCM (5000 mL) was added TEA (411.7 g, 566 mL, 4076.6 mmol, 2.0 eq) followed by MsCl (302.1 g, 305 mL, 2649.8 mmol, 1.3 eq) dropwise at 0 °C and the mixture was stirred for 2 h. This was poured into water (2500 mL) and the organic phase was separated and the aqueous phase further extracted with DCM (3000 mL). The organic phases were combined and washed with dilute citric acid solution (800 mL) and brine (1500 mL), and dried over Na₂SO₄. The two batches were combined and concentrated under reduced pressure to afford 1-(*tert*-butyl) 2-methyl (2*S*,4*S*)-4-((methylsulfonyl)-oxy)pyrrolidine-1,2-dicarboxylate as a brown semi-solid (1200 g, 3715.2 mmol, 91% yield).

LCMS: Method C, 1.51 min, MS: [M-56] 268.1; ¹H NMR (400 MHz, DMSO-*d₆*) δ ppm: 5.26 - 5.27 (m, 1H), 4.42 - 4.56 (m, 1H), 3.78 - 3.83 (m, 5H), 3.04 (s, 3H), 2.51 - 2.58 (m, 2H), 1.51 (s, 4.5H, Boc rotamer 1), 1.46 (s, 4.5H, Boc rotamer 2).

### Step (ii)

### 1-(tert-Butyl) 2-methyl (2S,4R)-4-azidopyrrolidine-1,2-dicarboxylate

To a stirred solution of 1-(tert-butyl) 2-methyl (2S,4,S)-4-((methylsulfonyl)oxy)pyrrolidine-1,2-dicarboxylate (500 g, 1548.0 mmol, 1.0 eq) in DMF (5000 mL) was added NaN₃ (139.3 g, 2322.0 mmol, 1.5 eq) portionwise at 0 °C. The reaction mixture was gradually heated to 80 °C for 4 h. The reaction mixture was cooled to rt and poured into ice-cold water (3500 mL) and extracted with EtOAc (3 x 3000 mL). The organic phases were combined and washed with ice-cold water (4 × 1000 mL) and brine (2 × 1000 mL), dried over Na₂SO₄ and concentrated under reduced pressure to afford 1-(*tert*-butyl) 2-methyl (2*S*,4*R*)-4-azidopyrrolidine-1,2-dicarboxylate as a brown semi-solid (400 g, 1481.5 mmol, 95% yield).

LCMS: Method C, 1.65 min, MS: ES+ 271.2; ¹H NMR (400 MHz, DMSO-*d₆*) δ ppm: 4.36 - 4.39 (m, 1H), 4.20 - 4.27 (m, 1H), 3.65 - 3.68 (m, 3H), 3.51 - 3.58 (m, 1H), 3.38 - 3.41 (d, 1H), 2.29 - 2.38 (m, 1H), 2.10 - 2.19 (m, 1H), 1.40 (s, 4.5H, Boc rotamer 1), 1.34 (s, 4.5H, Boc rotamer 2).

### Step (iii)

### tert-Butyl (2S,4R)-4-azido-2-(hydroxymethyl)pyrrolidine-1-carboxylate

To a stirred solution of 1-(*tert*-butyl) 2-methyl (2S,4R)-4-azidopyrrolidine-1,2-dicarboxylate (300 g, 1111.1 mmol, 1.0 eq) in THF (3000 mL) was added LiBH₄ solution (3 M in THF, 47.2 g, 2166.7 mmol, 1.95 eq) dropwise at -30 °C. The mixture was allowed to warm to rt and stirred for 3 h. The mixture was quenched by dropwise addition of a saturated solution of NaHCO₃ (4000 mL) at -78 °C and stirred for 16 h at rt. The crude mixture was extracted with EtOAc (3 × 3000 mL) and washed with brine (1000 mL). The organic phase was dried over Na₂SO₄ and concentrated under reduced pressure to give a brown semi-solid (220 g). The crude semi-solid was purified by column chromatography (23% EtOAc in hexane) to obtain *tert*-butyl (2S,4R)-4-azido-2-(hydroxymethyl)pyrrolidine-1-carboxylate as a pale yellow liquid (202.5 g, 836.8 mmol, 75% yield).

LCMS: Method C, 1.51 min, MS: ES+ 243.2; ¹H NMR (400 MHz, DMSO-*d₆*) δ ppm: 4.77 (s, 1H), 4.24 (s, 1H), 3.77 (br s, 1H), 3.33 - 3.46 (m, 4H), 2.10 - 2.15 (m, 1H), 1.98 - 1.99 (m, 1H), 1.39 (s, 9H).

### Step (iv)

### (2S,4R)-4-Azido-2-(((methylsulfonyl)oxy)methyl)pyrrolidine-1-carboxylate

To a stirred solution of tert-butyl (2*S*,4*R*)-4-azido-2-(hydroxymethyl)pyrrolidine-1-carboxylate (440 g, 1818.2 mmol, 1.0 eq) in DCM (4400 mL) was added triethylamine (552 g, 760 mL, 5454.5 mmol, 3.0 eq) at rt. The mixture was stirred for 10 min before the dropwise addition of MsCl (312.4 g, 2727.3 mmol, 1.5 eq) at -10 °C. The mixture was slowly warmed to rt and stirred for 3 h, then poured into ice-cold water (4000 mL) and the organic phase was separated. The aqueous phase was extracted with DCM (2 × 2000 mL). The organic phases were combined and washed with dilute citric acid (2 × 1000 mL), dried over Na₂SO₄, filtered and concentrated under reduced pressure to afford tert-butyl (2*S*,4*R*)-4-azido-2-(((methylsulfonyl)oxy)methyl)pyrrolidine-1-carboxylate as a brown liquid (550 g, quantitative yield). This was used directly in the next step.

LCMS: Method M, 17.67 min, MS: [M-56] 265.0.

### Step (v)

### tert-Butyl (2S,4R)-4-azido-2-((methylthio)methyl)pyrrolidine-1-carboxylate

To a stirred solution of *tert*-butyl (2*S*,4*R*)-4-azido-2-(((methylsulfonyl)oxy)methyl)pyrrolidine-1-carboxylate (5.0 g, 15.62 mmol) in MeOH (55 mL) was added sodium thiomethoxide (15% in water) (4.4 g, 62.50 mmol) dropwise at rt. The mixture was stirred at rt for 16 h, then poured into water (100 mL) and extracted with EtOAc (3 × 70 mL). The combined organic phases were dried over anhydrous Na₂SO₄ and concentrated under reduced pressure to yield *tert*-butyl (2*S*,4*R*)-4-azido-2-((methylthio)methyl)pyrrolidine-1-carboxylate (4.07 g, 14.96 mmol, 96% yield).

LCMS: Method C, 1.94 min, MS: ES+ 173.1 (M-100).

### Step (vi)

### tert-Butyl (2S,4R)-4-azido-2-((methylsulfonyl)methyl)pyrrolidine-1-carboxylate

To a stirred solution of *tert*-butyl (2S,4R)-4-azido-2-((methylthio)methyl)pyrrolidine-1-carboxylate (4.0 g, 14.70 mmol) in MeOH (20 mL) was added solution of Oxone (potassium peroxymonosulfate) (13.6 g, 22.06 mmol) in water (20 mL) dropwise at 0 °C. The mixture was warmed to rt and stirred for 1 h, then poured into water (80 mL) and extracted with EtOAc (3 × 80 mL). The combined organic phases were dried over anhydrous Na₂SO₄ and concentrated under reduced pressure to yield *tert*-butyl (2S,4R)-4-azido-2-((methylsulfonyl)methyl)pyrrolidine-1-carboxylate (3.88 g, 12.76 mmol, 87% yield).

LCMS: Method C, 1.53 min, MS: ES+ 327.2 (M+23).

### Step (vii)

### tert-Butyl (2S,4R)-4-amino-2-((methylsulfonyl)methyl)pyrrolidine-1-carboxylate

To a stirred solution of *tert*-butyl (2*S*,4*R*)-4-azido-2-((methylsulfonyl)methyl)pyrrolidine-1-carboxylate (3.88 g, 12.76 mmol) in MeOH (40 mL) was added 10% Pd/C (50% moisture, 1.94 g, 50% w/w) and purged with H₂ gas for 2 h at rt. The mixture was filtered through Celite, washed with MeOH (70 mL) and concentrated under reduced pressure. The obtained crude material was dissolved in EtOAc (100 mL) and washed with 1N HCl (50 mL). The aquoues layer was basified with sodium bicarbonate up to pH ~8 and extracted with 10% MeOH in DCM (5 × 100 mL). The combined organic phases were dried over anhydrous Na₂SO₄ and concentrated under reduced pressure to yield *tert*-butyl (2S,4R)-4-amino-2-((methylsulfonyl)methyl)pyrrolidine-1-carboxylate (2.5 g, 8.99 mmol, 70% yield).

LCMS: Method C, 1.23 min, MS: ES+ 279.3.

### Intermediate H

### tert-Butyl (2S, 4R)-2-((1H-1, 2, 3-triazol-1-yl)methyl)-4-aminopyrrolidine-1-carboxylate

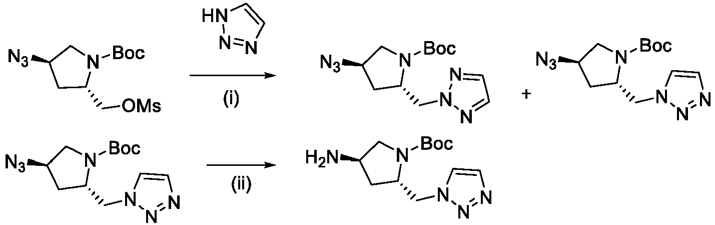

(i) K₂CO₃, DMF, 0 °C to rt, 48 h; (ii) 10% Pd/C, H₂, MeOH, rt, 1 h.

### Step (i)

### tert-Butyl (2S, 4R)-2-((1H-1, 2, 3-triazol-1-yl)methyl)-4-azidopyrrolidine-1-carboxylate

To a stirred solution of 1H-1,2,3-triazole (CAS 288-36-8, from Spectrochem, 0.76 g, 11.24 mmol) in DMF (30 mL) was added K₂CO₃ (3.88 g, 28.11 mmol) at 0°C. *tert*-Butyl (2*S*,4*R*)-4-azido-2-(((methyl sulfonyl)oxy)methyl)pyrrolidine-1-carboxylate (3.0 g, 9.37 mmol) was added at 0 °C. The mixture was allowed to warm to rt and stirred for 48 h, then poured into ice-cold water (700 mL) and extracted with EtOAc (5 × 100 mL). The combined organic phases were washed with ice-cold water (2 × 100 mL), dried over anhydrous Na₂SO₄ and concentrated under reduced pressure. The residue was purified by column chromatography (silica gel, 15-20% EtOAc in n-hexanes) to yield *tert*-butyl (2*S*,4*R*)-2-((2*H-*1,2,3-triazol-2-yl)methyl)-4-azidopyrrolidine-1-carboxylate (upper spot, undesired product, 1.0 g, 3.41 mmol, 36% yield) and subsequently (40-50% EtOAc in n-hexanes) *tert*-butyl (2*S*,4*R*)-2-((1*H-*1,2,3-triazol-1-yl)methyl)-4-azidopyrrolidine-1-carboxylate (lower spot, desired product, 1.0 g, 3.41 mmol, 36% yield).

For undesired product (upper spot, less polar): LCMS: Method C, 1.67 min, MS: ES+ 294.0; ¹H NMR (400 MHz, DMSO-*d₆*) δ ppm 7.83 (s, 2H), 4.63 - 4.65 (m, 2H), 4.02 - 4.19 (m, 2H), 3.41 - 3.44 (m, 1H), 3.04 - 3.14 (m, 1H), 2.00 - 2.17 (m, 2H), 1.43 (s, 9H).

For desired product (lower spot, more polar): LCMS: Method C, 1.52 min, MS: ES+ 294.2; ¹H NMR (400 MHz, DMSO-*d₆*) δ ppm 7.96 - 8.02 (m, 1H), 7.77 (s, 1H), 4.56 - 4.63 (m, 2H), 4.01 - 4.21 (m, 2H), 3.41 - 3.49 (m, 1H), 3.07 - 3.15 (m, 1H), 1.95 - 2.08 (m, 2H), 1.39 (s, 9H).

### Step (ii)

### tert-Butyl (2S, 4R)-2-((1H-1,2,3-triazol-1-yl)methyl)-4-aminopyrrolidine-1-carboxylate

To a stirred solution of *tert-*butyl (2*S*,4*R*)-2-((1H-1,2,3-triazol-1-yl)methyl)-4-azidopyrrolidine-1-carboxylate (lower spot, 1.0 g, 3.41 mmol) in MeOH (20 mL) was added 10% Pd/C (50% moisture) (0.50 g, 0.5 w/w). The mixture was purged with H₂ gas for 1 h, then filtered through Celite Hyflow^{®} and the filtrate was concentrated under reduced pressure to yield *tert-*butyl (2*S*,4*R*)-2-((1*H*-1,2,3-triazol-1-yl)methyl)-4-aminopyrrolidine-1-carboxylate (0.85 g, 3.18 mmol, 93% yield).

LCMS: Method C, 1.26 min, MS: ES+ 268.5.

### Example 1

### N-((3R,5S)-1-Cyano-5-(methoxymethyl)-pyrrolidin-3-yl)-5-(3-(trifluoromethyl)phenyl)oxazole-2-carboxamide

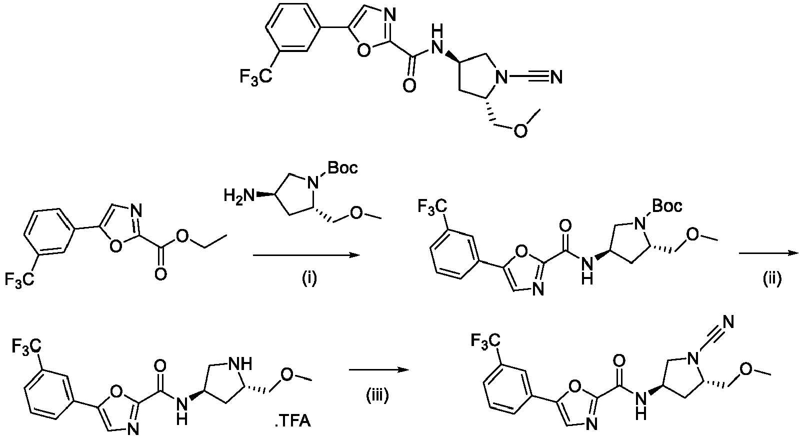

(i) TBD, THF, 0 °C to rt; (ii) TFA, DCM, 0 °C to rt; (iii) K₂CO₃, CNBr, THF, 0 °C to rt.

### Step (i)

### tert-Butyl (2S,4R)-2-(methoxymethyl)-4-(5-(3-(trifluoromethyl)phenyl)oxazole-2-carboxamido) pyrrolidine-1-carboxylate

To a stirred solution of ethyl 5-(3-(trifluoromethyl)phenyl)oxazole-2-carboxylate (70.0 g, 245.42 mmol) and *tert*-butyl (2S,4R)-4-amino-2-(methoxymethyl)pyrrolidine-1-carboxylate (CAS 1207853-53-9, 45.21 g, 196.33 mmol) in THF (700 mL) was added a solution of TBD (34.16 g, 245.42 mmol) in THF (350 mL) dropwise at 0 °C. The mixture was warmed to rt and stirred for 16 h. The mixture was poured into water (3500 mL) and extracted with EtOAc (2 × 2500 mL). The combined organic phases were dried over anhydrous Na₂SO₄ and concentrated under reduced pressure. The residue was purified by column chromatography (silica gel, 35% EtOAc in n-hexanes) to yield *tert*-butyl (2*S*,4*R*)-2-(methoxymethyl)-4-(5-(3-(trifluoromethyl)-phenyl)oxazole-2-carboxamido)pyrrolidine-1-carboxylate (50.0 g, 106.50 mmol, 43% yield).

LCMS: Method H, 3.68 min, MS: ES+ 470.1.

### Step (ii)

### N-((3R,5S)-5-(Methoxymethyl)pyrrolidin-3-yl)-5-(3-(trifluoromethyl)phenyl)oxazole-2-carboxamide

To a stirred solution of *tert*-butyl (2*S*,4*R*)-2-(methoxymethyl)-4-(5-(3-(trifluoromethyl)phenyl)-oxazole-2-carboxamido)pyrrolidine-1-carboxylate (60.0 g, 127.81 mmol) in DCM (600 mL, 10 vol) was added TFA (180 mL, 3 vol) dropwise at 0 °C. The mixture was allowed to warm to rt and stirred for 4 h, then concentrated under reduced pressure to yield *N*-((3*R*,5*S*)-5-(methoxymethyl)pyrrolidin-3-yl)-5-(3-(trifluoromethyl)phenyl) oxazole-2-carboxamide TFA salt (70.0 g, quantitative yield).

LCMS: Method H, 2.77 min, MS: ES- 368.0.

### Step (iii)

### N-((3R,5S)-1-Cyano-5-(methoxymethyl)pyrrolidin-3-yl)-5-(3-(trifluoromethyl)phenyl)oxazole-2-carboxamide

To a stirred solution of *N*-((3*R*,5*S*)-5-(methoxymethyl)pyrrolidin-3-yl)-5-(3-(trifluoromethyl) phenyl)oxazole-2-carboxamide TFA salt (70.0 g, 144.92 mmol) in THF (700 mL, 10 vol) was added K₂CO₃ (59.99 g, 434.78 mmol) portionwise at 0 °C. After 30 min, a solution of cyanogen bromide (18.43 g, 173.90 mmol) in THF (350 mL, 5 vol) was added dropwise at 0 °C and the mixture was stirred at rt for 4 h. The mixture was poured into water (2100 mL) and extracted with EtOAc (3 × 1400 mL). The combined organic phases were dried over Na₂SO₄ and concentrated under reduced pressure. The residue was triturated with diethyl ether (2 × 350 mL) to yield *N*-((3*R*,5*S*)-1-cyano-5-(methoxymethyl)pyrrolidin-3-yl)-5-(3-(trifluoromethyl)phenyl)-oxazole-2-carboxamide (24.0 g, 60.85 mmol, 47% yield over two steps).

LCMS: Method H, 3.09 min, MS: ES+ 395.2; ¹H NMR (400 MHz, DMSO-*d₆*) δ ppm: 9.37 (d, *J* = 6.8 Hz, 1H), 8.16 (s, 2H), 8.14 (d, *J* = 7.6 Hz, 1H), 7.76 - 7.83 (m, 2H), 4.48 - 4.52 (m, 1H), 3.99 - 4.05 (m, 1H), 3.67 - 3.71 (m, 1H), 3.40 - 3.49 (m, 3H), 3.32 (s, 3H), 2.09 - 2.15 (m, 1H), 1.94 - 2.00 (m, 1H); Chiral SFC: Method Y8, 4.69 min.

### Alternative synthesis of Example 1

### N-((3R,5S)-1-Cyano-5-(methoxymethyl)pyrrolidin-3-yl)-5-(3-(trifluoromethyl)phenyl)oxazole-2-carboxamide

Under N₂ atmosphere, to a stirred solution of *N*-((3*R*,5*S*)-5-(methoxymethyl)pyrrolidin-3-yl)-5-(3-(trifluoromethyl)phenyl)oxazole-2-carboxamide TFA salt (570.0 g, 1.1801 mol) in THF (5.7 L) was added K₂CO₃ (488.57 g, 3.5403 mol) in portions at 0 °C. A solution of cyanogen bromide (112.5 g, 1.0621 mol) in THF (225 mL) was added dropwise and stirred at 0 °C for 1 h. The mixture was allowed to warm to rt and stirred for 1 h. The progress of the reaction was monitored by TLC (50% EtOAc in *n*-hexanes, product R_{f} 0.05; 10% MeOH/DCM, product R_{f} 0.4). The mixture was quenched with water (2.8 L) and extracted with EtOAc (3 × 5 L). The combined organic phases were washed with brine solution (1.5 L) and then dried over anhydrous Na₂SO₄, filtered and concentrated under reduced pressure to yield crude product. The crude material was purified by column chromatography (silica gel, 35% EtOAc in *n*-hexanes). The pure fractions containing product were combined and distilled under reduced pressure below 45 °C to afford a white solid, which was further triturated from diethyl ether (2 × 1 L) to yield *N*-((3*R*,5*S*)-1-cyano-5-(methoxymethyl)pyrrolidin-3-yl)-5-(3-(trifluoromethyl)phenyl)oxazole-2-carboxamide as a white solid (340 g, 1.18 mol, 73% yield). LCMS: Method H3, 2.96 min, MS: ES+ 395.0; ¹H NMR (400 MHz, DMSO-*d₆*) *δ* ppm: 9.36 (d, *J* = 6.8 Hz, 1H), 8.15 (s, 2H), 8.12 (d, *J* = 7.6 Hz, 1H), 7.75 - 7.81 (m, 2H), 4.48 - 4.52 (m, 1H), 3.99 - 4.05 (m, 1H), 3.67 - 3.71 (m, 1H), 3.40 - 3.49 (m, 3H), 3.32 (s, 3H), 2.09 - 2.16 (m, 1H), 1.94 - 2.00 (m, 1H); chiral SFC: Method Y13, 4.64 min, >99.9% ee; HPLC: Method 13, 25.52 min; DSC melting temperature peak: 94.2 °C; high resolution MS: ES+ 395.1318 (calculated exact mass 394.1253).

### Example 2

### N-((3R,5R)-1-cyano-5-methylpyrrolidin-3-yl)-5-(3-(trifluoromethyl)phenyl)oxazole-2-carboxamide

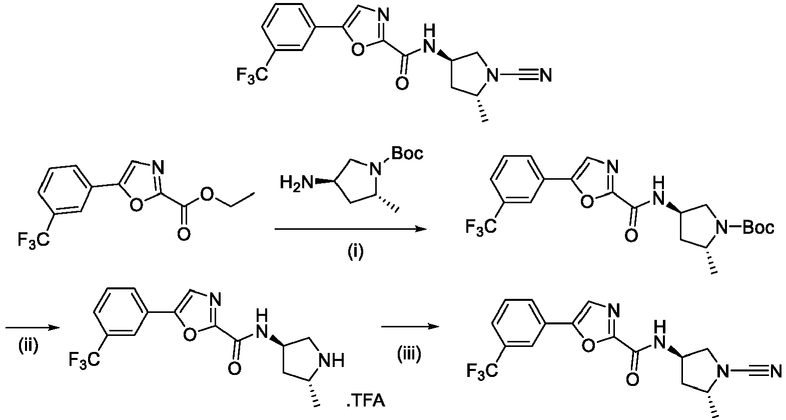

(i) TBD, THF, 0 °C to rt; (ii) TFA, DCM, 0 °C to rt; (iii) K₂CO₃, CNBr, THF, 0 °C to rt.

### Step (i)

### tert-Butyl (2R,4R)-2-methyl-4-(5-(3-(trifluoromethyl)phenyl)oxazole-2-carboxamido)pyrrolidine-1-carboxylate

To a stirred solution of ethyl 5-(3-(trifluoromethyl)phenyl)oxazole-2-carboxylate (1.0 g, 3.51 mmol) and *tert*-butyl (2R,4R)-4-amino-2-methylpyrrolidine-1-carboxylate (CAS 348165-63-9, 0.77 g, 3.86 mmol) in THF (10 mL) was added a solution of TBD (0.58 g, 4.21 mmol) in THF (5 mL) dropwise at 0 °C. The mixture was allowed to warm to rt and stirred for 1 h, then poured into cold water (150 mL) and extracted with EtOAc (3 × 70 mL). The combined organic phases were dried over anhydrous Na₂SO₄ and concentrated under reduced pressure. The residue was purified by flash column chromatography (silica gel, 50% EtOAc in *n*-hexanes) to yield *tert*-butyl (2*R*,4*R*)-2-methyl-4-(5-(3-(trifluoromethyl)-phenyl)oxazole-2-carboxamido)pyrrolidine-1-carboxylate (1.1 g, 2.50 mmol, 71% yield). LCMS: Method H, 3.76 min, MS: ES- 438.0.

### Step (ii)

### N-((3R,5R)-5-Methylpyrrolidin-3-yl)-5-(3-(trifluoromethyl)phenyl)oxazole-2-carboxamide

To a stirred solution of *tert*-butyl (2*R*,4*R*)-2-methyl-4-(5-(3-(trifluoromethyl)phenyl)oxazole-2-carboxamido)pyrrolidine-1-carboxylate (1.0 g, 2.28 mmol) in DCM (10 mL) was added TFA (4.0 mL, 4.0 vol) dropwise at 0 °C. The mixture was allowed to warm to rt and stirred for 1 h, then concentrated under reduced pressure to yield *N*-((3*R*,5*R*)-5-methylpyrrolidin-3-yl)-5-(3-(trifluoromethyl)phenyl)-oxazole-2-carboxamide TFA salt (1.60 g, quantitative yield).

LCMS: Method H, 2.78 min, MS: ES+ 340.20.

### Step (iii)

### N-((3R,5R)-1-Cyano-5-methylpyrrolidin-3-yl)-5-(3-(trifluoromethyl)phenyl)oxazole-2-carboxamide

To a stirred solution of *N-*((3*R*,5*R*)-5-methylpyrrolidin-3-yl)-5-(3-(trifluoromethyl)phenyl)oxazole-2-carboxamide TFA salt (1.60 g, 3.53 mmol) in THF (15 mL) was added K₂CO₃ (1.95 g, 14.13 mmol) and stirred at rt for 10 min. Cyanogen bromide (0.45 g, 4.24 mmol) was added at 0 °C and the reaction mixture was stirred at rt for 30 min. The mixture was poured into water (50 mL) and extracted with EtOAc (3 x 50 mL). The combined organic phases were dried over Na₂SO₄ and concentrated under reduced pressure. The residue was purified by flash column chromatography (silica gel, 50% EtOAc in n-hexanes) to yield *N*-((3*R*,5*R*)-1-cyano-5-methylpyrrolidin-3-yl)-5-(3-(trifluoromethyl)phenyl)-oxazole-2-carboxamide (0.46 g, 1.26 mmol, 55% yield over two steps).

LCMS: Method H, 3.15 min, MS: ES+ 365.1; ¹H NMR (400 MHz, DMSO-*d₆*) δ ppm: 9.40 (d, *J* = 6.8 Hz, 1H), 8.16 (s, 2H), 8.14 (d, *J* = 7.6 Hz, 1H), 7.77 - 7.83 (m, 2H), 4.46 - 4.54 (m, 1H), 3.89 - 3.93 (m, 1H), 3.77 - 3.79 (m, 1H), 3.43 (dd, *J* = 10.0, 3.2 Hz, 1H), 2.13 - 2.19 (m, 1H), 1.74 - 1.81 (m, 1H), 1.25 (d, *J* = 6.4 Hz, 3H); Chiral SFC: Method Y4, 3.91 min.

### Example 3

### N-((3R, 5S)-1-Cyano-5-((methylsulfonyl)methyl)pyrrolidin-3-yl)-5-(3-(trifluoromethyl)phenyl)-oxazole-2-carboxamide

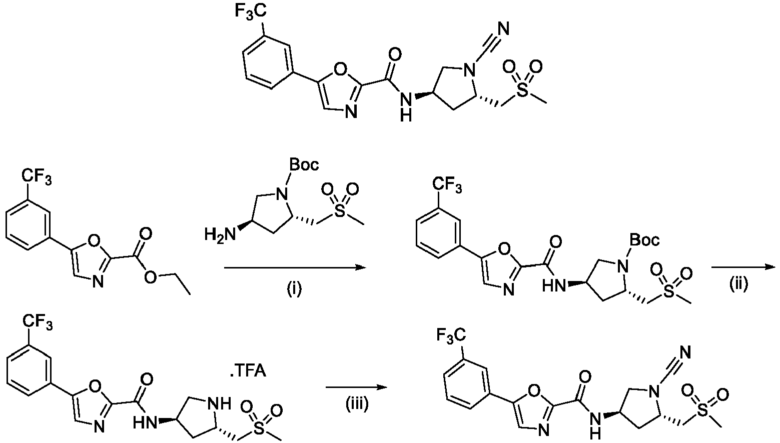

(i) TBD, THF, 0 °C to rt; (ii) TFA, DCM, 0 °C to rt; (iii) K₂CO₃, CNBr, THF, 0 °C to rt.

### Step (i)

### tert-Butyl (2S,4R)-2-((methylsulfonyl)methyl)-4-(5-(3-(trifluoromethyl)phenyl)oxazole-2-carboxamido)pyrrolidine-1-carboxylate

To a stirred solution of ethyl 5-(3-(trifluoromethyl)phenyl)oxazole-2-carboxylate (0.50 g, 1.75 mmol) and *tert*-butyl (2*S*,4*R*)-4-amino-2-((methylsulfonyl)methyl)pyrrolidine-1-carboxylate (CAS 2381441-33-2, 0.39 g, 1.40 mmol) in THF (2.5 mL) was added TBD (0.24 g, 1.75 mmol) in portions at 0 °C. The mixture was warmed to rt and stirred for 4 h, then poured into water (40 mL) and extracted with EtOAc (2 × 50 mL). The combined organic phases were dried over anhydrous Na₂SO₄ and concentrated under reduced pressure. The residue was purified by flash column chromatography (silica gel, 49.2% EtOAc in *n*-hexanes) to yield *tert*-butyl (2*S*,4*R*)-2-((methylsulfonyl)methyl)-4-(5-(3-(trifluoromethyl)phenyl) oxazole-2-carboxamido)pyrrolidine-1-carboxylate (0.09 g, 0.17 mmol, 10% yield).

LCMS: Method C, 1.77 min, MS: ES+ 462.4 (M-56).

### Step (ii)

### N-((3R,5S)-5-((Methylsulfonyl)methyl)pyrrolidin-3-yl)-5-(3-(trifluoromethyl)phenyl)oxazole-2-carboxamide TFA salt

To a stirred solution of *tert*-butyl (2*S*,4*R*)-2-((methylsulfonyl)methyl)-4-(5-(3-(trifluoromethyl)-phenyl)oxazole-2-carboxamido)pyrrolidine-1-carboxylate (0.09 g, 0.17 mmol) in DCM (3 mL) was added TFA (0.88 mL, 10 vol) dropwise at 0 °C. The mixture was allowed to warm to rt and stirred for 1 h, then concentrated under reduced pressure to yield *N*-((3*R*,5*S*)-5-((methylsulfonyl)methyl)-pyrrolidin-3-yl)-5-(3-(trifluoromethyl)phenyl)oxazole-2-carboxamide TFA salt (0.11 g, quantitative yield).

LCMS: Method C, 1.40 min, MS: ES+ 370.2.

### Step (iii)

### N-((3R,5S)-1-Cyano-5-((methylsulfonyl)methyl)pyrrolidin-3yl)-5-(3-(trifluoromethyl)phenyl)oxazole-2-carboxamide

To a stirred solution of *N*-((3*R*,5*S*)-5-((methylsulfonyl)methyl)pyrrolidin-3-yl)-5-(3-(trifluoromethyl) phenyl)oxazole-2-carboxamide TFA salt (0.10 g, 0.19 mmol) in THF (2 mL) was added K₂CO₃ (0.13 g, 0.94 mmol) at rt. After 10 min, cyanogen bromide (0.02 g, 0.19 mmol) was added at 0 °C. The mixture was allowed to warm to rt and stirred for 1 h, then poured into water (20 mL) and extracted with EtOAc (2 x 30 mL). The combined organic phases were dried over Na₂SO₄ and concentrated under reduced pressure. The residue was purified by flash column chromatography (silica gel, 90% EtOAc in *n*-hexanes) to yield *N*-((3*R,*5*S*)-1-cyano-5-((methylsulfonyl)methyl)pyrrolidin-3-yl)-5-(3-(trifluoromethyl)phenyl)oxazole-2-carboxamide (0.02 g, 0.05 mmol, 30% yield over two steps). LCMS: Method H, 2.79 min, MS: ES- 441.0; ¹H NMR (400 MHz, DMSO-*d₆*) δ ppm: 9.49 (d, *J* = 6.0 Hz, 1H), 8.18 (s, 2H), 8.15 (d, *J* = 6.8 Hz, 1H), 7.76 - 7.87 (m, 2H), 4.52 - 4.62 (m, 1H), 4.33 - 4.42 (m, 1H), 3.72 - 3.80 (m, 1H), 3.61 - 3.69 (m, 1H), 3.48 - 3.55 (m, 1H), 3.42 - 3.47 (m, 1H), 3.12 (s, 3H), 2.32 - 2.39 (m, 1H), 2.11 - 2.21 (m, 1H); Chiral SFC: Method Y5, 5.46 min.

### Example 4

### N-((3R,5S)-5-((1H-1,2,3-Triazol-1-yl)methyl)-1-cyanopyrrolidin-3-yl)-5-(3-(trifluoromethyl)phenyl) oxazole-2-carboxamide

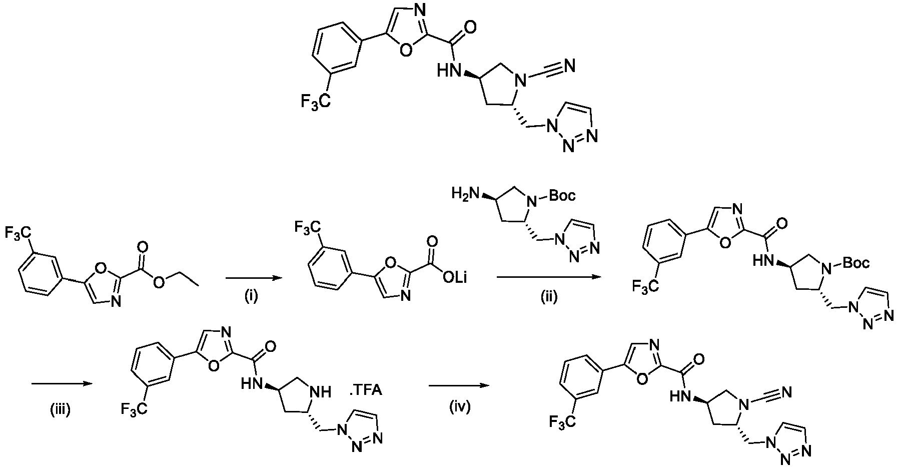

(i) LiOH.H₂O, H₂O, THF, 0 °C to rt; (ii) POCl₃, pyridine, 0 °C to rt; (iii) TFA, DCM, 0 °C to rt; (iv) K₂CO₃, CNBr, THF, 0 °C to rt.

### Step (i)

### Lithium 5-(3-(trifluoromethyl)phenyl)oxazole-2-carboxylate

To a stirred solution of ethyl 5-(3-(trifluoromethyl)phenyl)oxazole-2-carboxylate (0.3 g, 1.05 mmol) in THF (5 mL) was added a solution of LiOH.H₂O (0.13 g, 3.16 mmol) in water (2.5 mL) dropwise at 0 °C. The mixture was allowed to warm to rt and stirred for 1 h, then concentrated under reduced pressure. The obtained residue was co-concentrated with toluene (2 × 10 mL), then triturated with diethyl ether (10 mL) to afford lithium 5-(3-(trifluoromethyl)phenyl)oxazole-2-carboxylate (0.50 g, quantitative yield).

LCMS: Method C, 1.77 min, MS: ES+ 258.0.

### Step (ii)

### tert-Butyl (2S, 4R)-2-((1H-1,2,3-triazol-1-yl)methyl)-4-(5-(3-(trifluoromethyl)phenyl)oxazole-2-carboxamido)pyrrolidine-1-carboxylate

To a stirred solution of lithium 5-(3-(trifluoromethyl)phenyl)oxazole-2-carboxylate (0.30 g, 1.12 mmol) and *tert*-butyl (2*S*,4*R*)-2-((1*H*-1,2,3-triazol-1-yl)methyl)-4-aminopyrrolidine-1-carboxylate (0.20 g, 0.75 mmol) in pyridine (2 mL) was added POCl₃ (0.34 g, 0.21 mL, 2.25 mmol) dropwise at 0 °C. The mixture was allowed to warm to rt and stirred for 1 h, then poured into water (50 mL) and extracted with EtOAc (2 × 50 mL). The combined organic phases were dried over Na₂SO₄ and concentrated under reduced pressure. The residue was purified by flash column chromatography (silica gel, 5% MeOH in DCM) to yield *tert*-butyl (2*S*,4*R*)-2-((1*H*-1,2,3-triazol-1-yl)methyl)-4-(5-(3-(trifluoromethyl)phenyl) oxazole-2-carboxamido)pyrrolidine-1-carboxylate (0.06 g, 0.13 mmol, 17% yield).

LCMS: Method J, 4.70 min, MS: ES+ 507.2.

### Step (iii)

### N-((3R, 5S)-5-((1H-1,2,3-Triazol-1-yl)methyl)pyrrolidin-3-yl)-5-(3-(trifluoromethyl)phenyl)oxazole-2-carboxamide

To a stirred solution of *tert*-butyl (2*S*,4*R*)-2-((1*H*-1,2,3-triazol-1-yl)methyl)-4-(5-(3-(trifluoromethyl) phenyl)oxazole-2-carboxamido)pyrrolidine-1-carboxylate (0.06 g, 0.12 mmol) in DCM (4 mL) was added TFA (0.6 mL, 10 vol) dropwise at 0 °C. The mixture was allowed to warm to rt and stirred for 2 h, then concentrated under reduced pressure to yield *N*-((3*R*,5*S*)-5-((1*H*-1,2,3-triazol-1-yl)methyl) pyrrolidin-3-yl)-5-(3-(trifluoromethyl)phenyl)oxazole-2-carboxamide TFA salt (0.05 g, 0.1 mmol, 81% yield).

LCMS: Method C, 1.36 min, MS: ES+ 407.7.

### Step (iv)

### N-((3R,5S)-5-((1H-1,2,3-Triazol-1-yl)methyl)-1-cyanopyrrolidin-3-yl)-5-(3-(trifluoromethyl)phenyl) oxazole-2-carboxamide

To a stirred solution of *N*-((3*R,*5*S*)-5-((1*H*-1,2,3-triazol-1-yl)methyl)pyrrolidin-3-yl)-5-(3-(trifluoromethyl)phenyl)oxazole-2-carboxamide TFA salt (0.05 g, 0.1 mmol) in THF (2 mL) was added K₂CO₃ (0.04 g, 0.29 mmol) and the mixture was stirred for 10 min at 0 °C. Cyanogen bromide (0.01 g, 0.1 mmol) was added at 0 °C. The mixture was allowed to warm to rt and stirred for 2 h, then poured into water (50 mL) and extracted with EtOAc (2 × 60 mL). The combined organic phases were dried over anhydrous Na₂SO₄, filtered and concentrated under reduced pressure. The residue was purified by flash column chromatography (silica gel, 3.5% MeOH in DCM) to yield *N*-((3*R*,5*S*)-5-((1*H*-1,2,3-triazol-1-yl)methyl)-1-cyanopyrrolidin-3-yl)-5-(3-(trifluoromethyl)phenyl)-oxazole-2-carboxamide (0.02 g, 0.04 mmol, 45% yield).

LCMS: Method H, 2.82 min, MS: ES-430.0; ¹H NMR (400 MHz, DMSO-*d₆*) *δ* ppm 9.42 (d, *J* = 6.4 Hz, 1H), 8.20 (s, 1H), 8.17 (s, 2H), 8.13 (d, *J* =7.2 Hz, 1H), 7.78 - 7.83 (m, 3H), 4.62 (d, *J* = 5.6 Hz, 2H), 4.35 - 4.38 (m, 2H), 3.67 (m, 1H), 3.39 - 3.48 (m, 1H), 2.17 - 2.24 (m, 1H), 1.96 - 2.02 (m, 1H); chiral HPLC: Method Y4, 4.03 min.

### Example 5

### N-((3R,5S)-1-Cyano-5-(methoxymethyl)pyrrolidin-3-yl)-5-(2-cyclopropoxy-5-(trifluoromethyl)phenyl)-1,3,4-oxadiazole-2-carboxamide

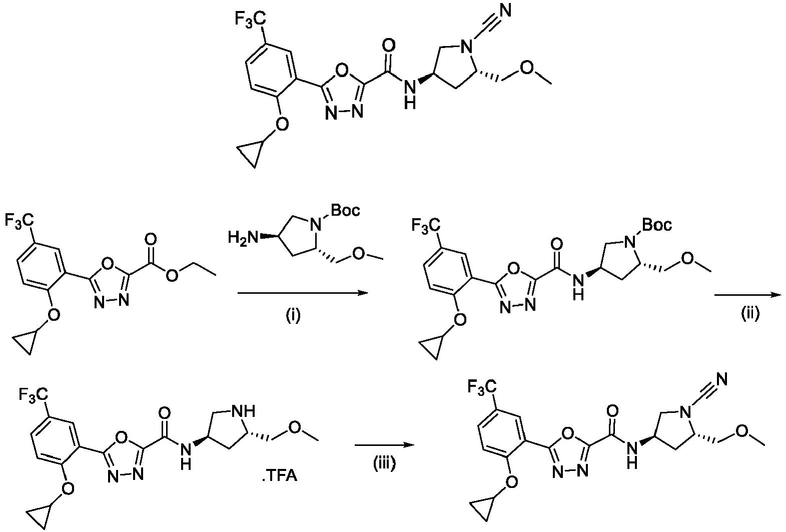

(i) TBD, THF, 0 °C to rt; (ii) TFA, DCM, 0 °C to rt; (iii) K₂CO₃, CNBr, THF, 0 °C to rt.

### Step (i)

### tert-Butyl (2S,4R)-4-(5-(2-cyclopropoxy-5-(trifluoromethyl)phenyl)-1, 3, 4-oxadiazole-2-carboxamido)-2-(methoxymethyl)pyrrolidine-1-carboxylate

To a stirred solution of ethyl 5-(2-cyclopropoxy-5-(trifluoromethyl)phenyl)-1,3,4-oxadiazole-2-carboxylate (0.2 g, 0.58 mmol) and *tert*-butyl (2S,4R)-4-amino-2-(methoxymethyl)pyrrolidine-1-carboxylate (CAS 1207853-53-9, 0.13 g, 0.58 mmol) in THF (4 mL) was added TBD (0.12 g, 0.88 mmol) in portions at 0 °C. The mixture was warmed to rt and stirred for 1 h, then poured into water (50 mL) and extracted with EtOAc (2 × 50 mL). The combined organic phases were dried over anhydrous Na₂SO₄ and concentrated under reduced pressure. The residue was purified by flash column chromatography (silica gel, 25% EtOAc in *n*-hexanes) to yield *tert*-butyl (2S,4R)-4-(5-(2-cyclopropoxy-5-(trifluoromethyl)phenyl)-1,3,4-oxadiazole-2-carboxamido)-2-(methoxymethyl)pyrrolidine-1-carboxylate (0.12 g, 0.24 mmol, 40% yield).

LCMS: Method H, 3.76 min, MS: ES- 525.1.

### Step (ii)

### 5-(2-Cyclopropoxy-5-(trifluoromethyl)phenyl)-N-((3R,5S)-5-(methoxymethyl)pyrrolidin-3-yl)-1,3,4-oxadiazole-2-carboxamide

To a stirred solution of *tert*-butyl (2S,4R)-4-(5-(2-cyclopropoxy-5-(trifluoromethyl)phenyl)-1,3,4-oxadiazole-2-carboxamido)-2-(methoxymethyl)pyrrolidine-1-carboxylate (0.12 g, 0.23 mmol) in DCM (5 mL) was added TFA (0.4 mL, 3 vol) dropwise at 0 °C. The mixture was allowed to warm to rt and stirred for 1 h, then concentrated under reduced pressure to yield 5-(2-cyclopropoxy-5-(trifluoromethyl)phenyl)-*N*-((3*R*,5*S*)-5-(methoxymethyl)pyrrolidin-3-yl)-1,3,4-oxadiazole-2-carboxamide TFA salt (0.18 g, quantitative yield).

LCMS: Method H, 2.87 min, MS: ES- 425.0.

### Step (iii)

### N-((3R,5S)-1-Cyano-5-(methoxymethyl)pyrrolidin-3-yl)-5-(2-cyclopropoxy-5-(trifluoromethyl)phenyl)-1,3,4-oxadiazole-2-carboxamide

To a stirred solution of 5-(2-cyclopropoxy-5-(trifluoromethyl)phenyl)-*N*-((3*R*,5*S*)-5-(methoxymethyl)-pyrrolidin-3-yl)-1,3,4-oxadiazole-2-carboxamide TFA salt (0.18 g, 0.33 mmol) in THF (5 mL) was added K₂CO₃ (0.14 g, 0.99 mmol) at rt and the mixture was stirred for 10 min. Cyanogen bromide (0.03 g, 0.33 mmol) was added at 0 °C. The mixture was stirred at rt for 1 h, then poured into water (50 mL) and extracted with EtOAc (2 × 50 mL). The combined organic phases were dried over Na₂SO₄ and concentrated under reduced pressure. The residue was purified by flash column chromatography (silica gel, 60% EtOAc in *n*-hexanes) to yield *N*-((3*R*,5*S*)-1-cyano-5-(methoxymethyl)pyrrolidin-3-yl)-5-(2-cyclopropoxy-5-(trifluoromethyl)phenyl)-1,3,4-oxadiazole-2-carboxamide (0.04 g, 0.08 mmol, 37% yield over two steps).

LCMS: Method H, 3.19 min, MS: ES+ 452.2; ¹H NMR (400 MHz, DMSO-*d₆*) δ ppm: 9.77 (d, *J* = 6.8 Hz, 1H), 8.20 (s, 1H), 8.08 (d, *J* = 8.8 Hz, 1H), 7.79 (d, *J* = 8.8 Hz, 1H), 4.50 - 4.53 (m, 1H), 4.15 - 4.23 (m, 1H), 3.99 - 4.08 (m, 1H), 3.69 - 3.73 (m, 1H), 3.41 - 3.50 (m, 3H), 3.33 (s, 3H), 2.09 - 2.16 (m, 1H), 1.95 - 2.02 (m, 1H), 0.89 - 0.91 (m, 2H), 0.78 (m, 2H); Chiral SFC: Method Y4, 3.67 min.

### Example 6

### N-((3R, SR)-1-Cyano-5-methylpyrrolidin-3yl)-5-(2-cyclopropoxy-5-(trifluoromethyl)phenyl)-1,3,4-oxadiazole-2-carboxamide

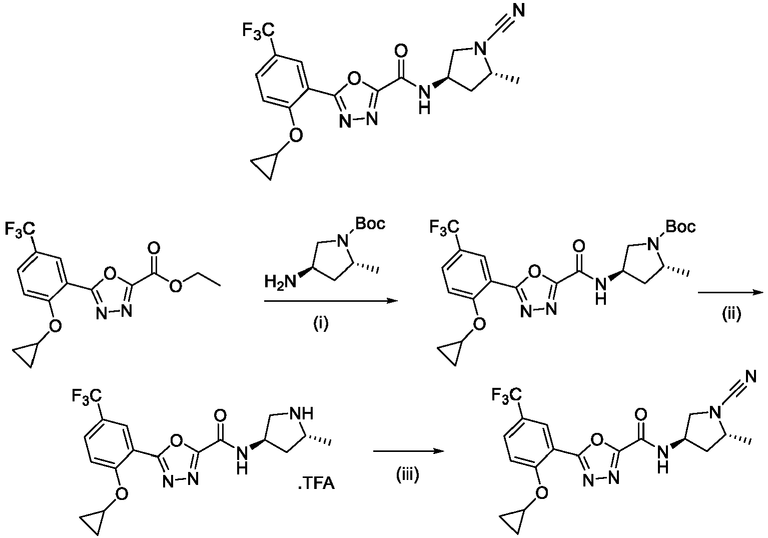

(i) TBD, THF, 0 °C to rt; (ii) TFA, DCM, 0 °C to rt; (iii) K₂CO₃, CNBr, THF, 0 °C to rt.

### Step (i)

### tert-Butyl (2R,4R)-4-(5-(2-cyclopropoxy-5-(trifluoromethyl)phenyl)-1,3, 4-oxadiazole-2-carboxamido)-2-methylpyrrolidine-1-carboxylate

To a stirred solution of ethyl 5-(2-cyclopropoxy-5-(trifluoromethyl)phenyl)-1,3,4-oxadiazole-2-carboxylate (0.23 g, 0.67 mmol) and *tert*-butyl (2R,4R)-4-amino-2-methylpyrrolidine-1-carboxylate (CAS 348165-63-9, 0.13 g, 0.67 mmol) in THF (5 mL) was added TBD (0.14 g, 1.00 mmol) in portions at 0 °C. The mixture was warmed to rt and stirred for 1 h, then poured into water (50 mL) and extracted with EtOAc (2 × 50 mL). The combined organic phases were dried over anhydrous Na₂SO₄ and concentrated under reduced pressure. The residue was purified by flash column chromatography (silica gel, 45% EtOAc in *n*-hexanes) to yield *tert*-butyl (2*R*,4*R*)-4-(5-(2-cyclopropoxy-5-(trifluoromethyl)-phenyl)-1,3,4-oxadiazole-2-carboxamido)-2-methylpyrrolidine-1-carboxylate (0.07 g, 0.14 mmol, 21% yield).

LCMS: Method H, 4.02 min, MS: ES- 495.4.

### Step (ii)

### 5-(2-Cyclopropoxy-5-(trifluoromethyl)phenyl)-N-((3R, 5R)-5-methylpyrrolidin-3-yl)-], 3, 4-oxadiazole-2-carboxamide

To a stirred solution of *tert*-butyl (2*R*,4*R*)-4-(5-(2-cyclopropoxy-5-(trifluoromethyl)phenyl)-1,3,4-oxadiazole-2-carboxamido)-2-methylpyrrolidine-1-carboxylate (0.06 g, 0.13 mmol) in DCM (3 mL) was added TFA (0.2 mL, 3 vol) dropwise at 0 °C. The mixture was allowed to warm to rt and stirred for 1 h, then concentrated under reduced pressure to yield 5-(2-cyclopropoxy-5-(trifluoromethyl)-phenyl)-*N-*((3*R*,5*R*)-5-methylpyrrolidin-3-yl)-1,3,4-oxadiazole-2-carboxamide TFA salt (0.1 g, quantitative yield). LCMS: Method H, 3.05 min, MS: ES+ 397.2.

### Step (iii)

### N-((3R,5R)-1-Cyano-5-methylpyrrolidin-3-yl)-5-(2-cyclopropoxy-5-(trifluoromethyl)phenyl)-1,3,4-oxadiazole-2-carboxamide

To a stirred solution of 5-(2-cyclopropoxy-5-(trifluoromethyl)phenyl)-*N*-((3*R*,5*R*)-5-methylpyrrolidin-3-yl)-1,3,4-oxadiazole-2-carboxamide TFA salt (0.1 g, 0.19 mmol) in THF (3 mL) was added K₂CO₃ (0.08 g, 0.59 mmol) at rt. After 10 min, cyanogen bromide (0.02 g, 0.19 mmol) was added at 0 °C. The mixture was stirred at rt for 1 h, then poured into water (50 mL) and extracted with EtOAc (2 x 50 mL). The combined organic phases were dried over Na₂SO₄ and concentrated under reduced pressure. The residue was purified by flash column chromatography (silica gel, 70% EtOAc in n-hexanes) to yield N ((3R,5R)-1-cyano-5-methylpyrrolidin-3-yl)-5-(2-cyclopropoxy-5-(trifluoromethyl)phenyl)-1,3,4-oxadiazole-2-carboxamide (0.03 g, 0.07 mmol, 56% yield over two steps).

LCMS: Method H, 3.43 min, MS: ES- 420.2; ¹H NMR (400 MHz, DMSO-*d₆*) δ ppm: 9.80 (d, *J* = 6.4 Hz, 1H), 8.20 (s, 1H), 8.08 (d, *J* = 8.8 Hz, 1H), 7.79 (d, *J* = 8.8 Hz, 1H), 4.48 - 4.57 (m, 1H), 4.15 - 4.25 (m, 1H), 3.88 - 3.93 (m, 1H), 3.78 (m, 1H), 3.42- 3.60 (m, 1H), 2.15 - 2.18 (m, 1H), 1.74 - 1.81 (m, 1H), 1.25 (d, *J* = 6.0 Hz, 3H), 0.86 - 0.92 (m, 2H), 0.76 - 0.79 (m, 2H); Chiral SFC: Method Y4, 3.99 min.

### Example 7

### N-((3R,5R)-1-Cyano-5-methylpyrrolidin-3-yl)-5-(3-(trifluoromethyl)phenyl)-1,3,4-oxadiazole-2-carboxamide

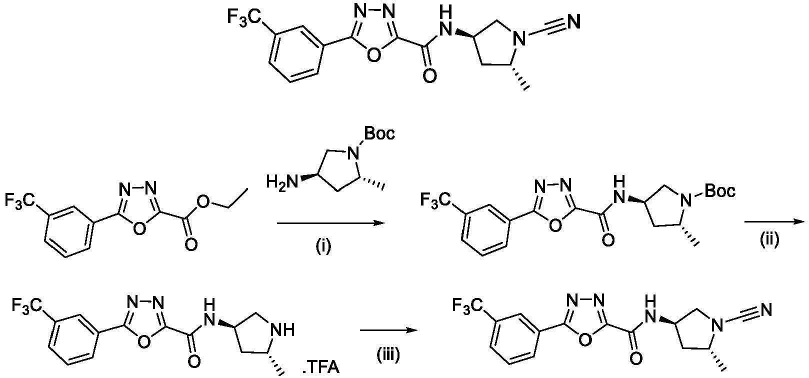

(i) TBD, THF, 0 °C to rt; (ii) TFA, DCM, 0 °C to rt; (iii) K₂CO₃, CNBr, THF, 0 °C to rt.

### Step (i)

### tert-Butyl (2R, 4R)-2-methyl-4-(5-(3-(trifluoromethyl)phenyl)-1, 3, 4-oxadiazole-2-carboxamido) pyrrolidine-1-carboxylate

To a stirred solution of ethyl 5-(3-(trifluoromethyl)phenyl)-1,3,4-oxadiazole-2-carboxylate (0.30 g, 1.05 mmol) and *tert*-butyl (2R,4R)-4-amino-2-methylpyrrolidine-1-carboxylate (CAS 348165-63-9, 0.25 g, 1.26 mmol) in THF (6 mL) was added TBD (0.22 g, 1.57 mmol) in portions at 0 °C. The mixture was warmed to rt and stirred for 1 h, then poured into water (100 mL) and extracted with EtOAc (2 x 80 mL). The combined organic phases were dried over anhydrous Na₂SO₄ and concentrated under reduced pressure. The residue was purified by flash column chromatography (silica gel, 30% EtOAc in *n*-hexanes) to yield *tert*-butyl (2*R*,4*R*)-2-methyl-4-(5-(3-(trifluoromethyl)phenyl)-1,3,4-oxadiazole-2-carboxamido)pyrrolidine-1-carboxylate (0.21 g, 0.48 mmol, 45% yield).

LCMS: Method C, 1.82 min, MS: ES+ 441.0.

### Step (ii)

### N-((3R,5R)-5-Methylpyrrolidin-3yl)-5-(3-(trifluoromethyl)phenyl)-1,3,4-oxadiazole-2-carboxamide

To a stirred solution of *tert*-butyl (2*R*,4*R*)-2-methyl-4-(5-(3-(trifluoromethyl)phenyl)-1,3,4-oxadiazole-2-carboxamido)pyrrolidine-1-carboxylate (0.21 g, 0.48 mmol) in DCM (5 mL) was added TFA (0.6 mL, 3 vol) dropwise at 0 °C. The mixture was allowed to warm to rt and stirred for 1 h, then concentrated under reduced pressure to yield *N*-((3*R*,5*R*)-5-methylpyrrolidin-3-yl)-5-(3-(trifluoromethyl)phenyl)-1,3,4-oxadiazole-2-carboxamide TFA salt (0.20 g, 0.44 mmol, 92% yield). LCMS: Method C, 1.33 min, MS: ES+ 341.0.

### Step (iii)

### N-((3R,5R)-1-Cyano-5-methylpyrrolidin-3-yl)-5-(3-(trifluoromethyl)phenyl)-1,3,4-oxadiazole-2-carboxamide

To a stirred solution of *N*-((3*R*,5*R*)-5-methylpyrrolidin-3-yl)-5-(3-(trifluoromethyl)phenyl)-1,3,4-oxadiazole-2-carboxamide TFA salt (0.20 g, 0.44 mmol) in THF (5 mL) was added K₂CO₃ (0.18 g, 1.32 mmol) at rt and the mixture was stirred for 10 min. Cyanogen bromide (0.05 g, 0.44 mmol) was added at 0 °C. The mixture was stirred at rt for 45 min, then poured into water (50 mL) and extracted with EtOAc (2 x 50 mL). The combined organic phases were dried over Na₂SO₄ and concentrated under reduced pressure. The residue was purified by flash column chromatography (silica gel, 60% EtOAc in *n*-hexanes) to yield *N*-((3*R*,5*R*)-1-cyano-5-methylpyrrolidin-3-yl)-5-(3-(trifluoromethyl) phenyl)-1,3,4-oxadiazole-2-carboxamide (0.12 g, 0.34 mmol, 77% yield).

LCMS: Method H, 3.06 min, MS: ES- 364.0; ¹H NMR (400 MHz, DMSO-*d₆*) *δ* ppm 9.82 (d, *J* = 6.8 Hz, 1H), 8.41 (d, *J* = 7.6 Hz, 1H), 8.33 (s, 1H), 8.10 (d, *J* = 7.6 Hz, 1H), 7.92 (t, *J* = 8.0 Hz, 1H), 4.53 - 4.55 (m, 1H), 3.90 - 3.95 (m, 1H), 3.78 - 3.82 (m, 1H), 3.45 - 3.48 (m, 1H), 2.17 - 2.19 (m, 1H), 1.77 - 1.84 (m, 1H), 1.27 (d, *J* = 6.4 Hz, 3H); Chiral SFC: Method Y3, 2.65 min.

### Example 8

### N-((3R, 5S)-1-Cyano-5-(methoxymethyl)pyrrolidin-3-yl)-5-(3-(trifluoromethyl)phenyl)-1,3,4-oxadiazole-2-carboxamide

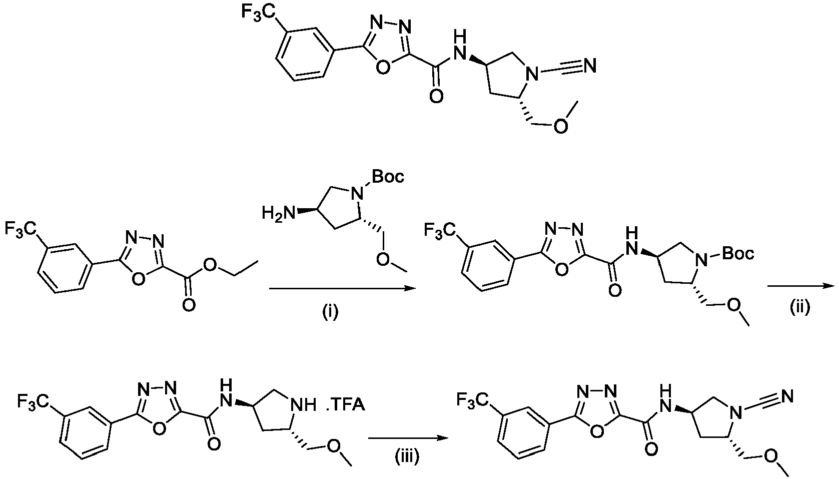

(i) TBD, THF, 0 °C to rt; (ii) TFA, DCM, 0 °C to rt; (iii) K₂CO₃, CNBr, THF, 0 °C to rt.

### Step (i)

### tert-Butyl (2S,4R)-2-(methoxymethyl)-4-(5-(3-(trifluoromethyl)phenyl)-1,3,4-oxadiazole-2-carboxamido)pyrrolidine-1-carboxylate

To a stirred solution of ethyl 5-(3-(trifluoromethyl)phenyl)-1,3,4-oxadiazole-2-carboxylate (0.30 g, 1.05 mmol) and *tert*-butyl (2*S*,4*R*)-4-amino-2-(methoxymethyl)pyrrolidine-1-carboxylate (CAS 1207853-53-9, 0.29 g, 1.26 mmol) in THF (6 mL) was added TBD (0.22 g, 1.57 mmol) in portions at 0 °C. The mixture was warmed to rt and stirred for 1 h, then poured into water (100 mL) and extracted with EtOAc (2 × 80 mL). The combined organic phases were dried over anhydrous Na₂SO₄ and concentrated under reduced pressure. The residue was purified by flash column chromatography (silica gel, 40% EtOAc in *n*-hexanes) to yield *tert*-butyl (2*S*,4*R*)-2-(methoxymethyl)-4-(5-(3-(trifluoromethyl)phenyl)-1,3,4-oxadiazole-2-carboxamido)pyrrolidine-1-carboxylate (0.19 g, 0.40 mmol, 39% yield). LCMS: Method C, 1.79 min, MS: ES+ 471.1.

### Step (ii)

### N-((3R,5S)-5-(Methoxymethyl)pyrrolidin-3-yl)-5-(3-(trifluoromethyl)phenyl)-1,3,4-oxadiazole-2-carboxamide

To a stirred solution of *tert*-butyl (2*S*,4*R*)-2-(methoxymethyl)-4-(5-(3-(trifluoromethyl)phenyl)-1,3,4-oxadiazole-2-carboxamido)pyrrolidine-1-carboxylate (0.19 g, 0.40 mmol) in DCM (5 mL) was added TFA (0.6 mL, 3 vol) dropwise at 0 °C. The mixture was allowed to warm to rt and stirred for 1 h, then concentrated under reduced pressure to yield *N*-((3*R*,5*S*)-5-(methoxymethyl)pyrrolidin-3-yl)-5-(3-(trifluoromethyl)phenyl)-1,3,4-oxadiazole-2-carboxamide TFA salt (0.18 g, 0.37 mmol, 92% yield). LCMS: Method C, 1.34 min, MS: ES+ 371.0.

### Step (iii)

### N-((3R,5S)-1-Cyano-5-(methoxymethyl)pyrrolidin-3-yl)-5-(3-(trifluoromethyl)phenyl)-1,3,4-oxadiazole-2-carboxamide

To a stirred solution of *N*-((3*R*,5*S*)-5-(methoxymethyl)pyrrolidin-3-yl)-5-(3-(trifluoromethyl)phenyl)-1,3,4-oxadiazole-2-carboxamide TFA salt (0.18 g, 0.37 mmol) in THF (5 mL) was added K₂CO₃ (0.15 g, 1.12 mmol) at rt and the mixture was stirred for 10 min. Cyanogen bromide (0.04 g, 0.37 mmol) was added at 0 °C. The mixture was stirred at rt for 45 min, then poured into water (50 mL) and extracted with EtOAc (2 × 50 mL). The combined organic phases were dried over Na₂SO₄ and concentrated under reduced pressure. The residue was purified by flash column chromatography (silica gel, 70% EtOAc in n-hexanes) to yield *N*-((3*R,*5S)-1-cyano-5-(methoxymethyl)pyrrolidin-3-yl)-5-(3-(trifluoromethyl)-phenyl)-1,3,4-oxadiazole-2-carboxamide (0.10 g, 0.26 mmol, 69% yield).

LCMS: Method H, 2.99 min, MS: ES- 394.0; ¹H NMR (400 MHz, DMSO-*d₆*) *δ* ppm 9.80 (d, *J* = 6.4 Hz, 1H), 8.41 (d, *J* = 7.6 Hz, 1H), 8.33 (s, 1H), 8.10 (d, *J* = 8.0 Hz, 1H), 7.92 (t, *J* = 8.0 Hz, 1H), 4.48 - 4.59 (m, 1H), 4.0 - 4.1 (m, 1H), 3.71 - 3.75 (m, 1H), 3.42 - 3.51 (m, 3H), 3.36 (s, 3H), 2.11 - 2.18 (m, 1H), 1.99 - 2.04 (m, 1H); Chiral SFC: Method Y3, 2.87 min.

### Example 9

### N-((3R,5S)-1-cyano-5-(methoxymethyl)pyrrolidin-3-yl)-5-(2-cyclopropoxy-5-(trifluoromethyl)phenyl)-oxazole-2-carboxamide

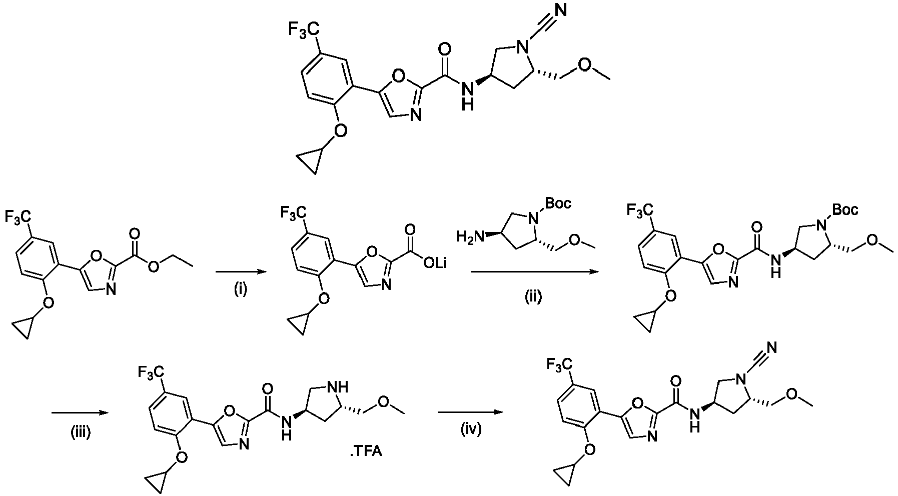

(i) LiOH.H₂O, H₂O, THF, 0 °C to rt; (ii) POCl₃, pyridine, 0 °C to rt; (iii) TFA, DCM, 0 °C to rt; (iv) K₂CO₃, CNBr, THF, 0 °C to rt.

### Step (i)

### Lithium 5-(2-cyclopropoxy-5-(trifluoromethyl)phenyl)oxazole-2-carboxylate

To a stirred solution of ethyl 5-(2-cyclopropoxy-5-(trifluoromethyl)phenyl)oxazole-2-carboxylate (0.13 g, 0.38 mmol) in THF (1.5 mL) was added a solution of LiOH.HzO (0.02 g, 0.57 mmol) in water (1.5 mL) dropwise at 0 °C. The mixture was allowed to warm to rt and stirred for 1 h, then concentrated under reduced pressure to yield lithium 5-(2-cyclopropoxy-5-(trifluoromethyl)phenyl)oxazole-2-carboxylate (0.14 g, quantitative yield).

LCMS: Method J, 4.16 min, MS: ES+ 313.4.

### Step (ii)

### tert-Butyl (2S,4R)-4-(5-(2-cyclopropoxy-5-(trifluoromethyl)phenyl)oxazole-2-carboxamido)-2-(methoxymethyl)pyrrolidine-1-carboxylate

To a stirred solution of lithium 5-(2-cyclopropoxy-5-(trifluoromethyl)phenyl)oxazole-2-carboxylate (0.13 g, 0.41 mmol) and *tert*-butyl (2S,4R)-4-amino-2-(methoxymethyl)pyrrolidine-1-carboxylate (CAS 1207853-53-9, 0.09 g, 0.41 mmol) in pyridine (4 mL) was added POCl₃ (0.19 g, 1.23 mmol) dropwise at 0 °C. The mixture was stirred at 0 °C for 0.5 h, then poured into water (50 mL) and extracted with EtOAc (2 × 50 mL). The combined organic phases were dried over Na₂SO₄ and concentrated under reduced pressure. The residue was purified by flash column chromatography (silica gel, 33.8% EtOAc in n-hexanes) to yield *tert*-butyl (2*S*,4*R*)-4-(5-(2-cyclopropoxy-5-(trifluoromethyl) phenyl)oxazole-2-carboxamido)-2-(methoxymethyl)pyrrolidine-1-carboxylate (0.09 g, 0.17 mmol, 44% yield over two steps).

LCMS: Method C, 1.98 min, MS: ES+ 526.2.

### Step (iii)

### 5-(2-cyclopropoxy-5-(trifluoromethyl)phenyl)-N-((3R, 5S)-5-(methoxymethyl)pyrrolidin-3-yl)oxazole-2-carboxamide

To a stirred solution of tert-butyl (2S,4R)-4-(5-(2-cyclopropoxy-5-(trifluoromethyl)phenyl)oxazole-2-carboxamido)-2-(methoxymethyl)pyrrolidine-1-carboxylate (0.09 g, 0.17 mmol) in DCM (3 mL) was added TFA (0.9 mL, 10 vol) dropwise at 0 °C. The mixture was allowed to warm to rt and stirred for 1 h, then concentrated under reduced pressure to yield 5-(2-cyclopropoxy-5-(trifluoromethyl)phenyl)-*N*-((3*R*,5*S*)-5-(methoxymethyl)pyrrolidin-3-yl)oxazole-2-carboxarnide TFA salt (0.13 g, quantitative yield).

LCMS: Method C, 1.44 min, MS: ES+ 426.1.

### Step (iv)

### N-((3R,5S)-1-cyano-5-(methoxymethyl)pyrrolidin-3-yl)-5-(2-cyclopropoxy-5-(trifluoromethyl)phenyl)oxazole-2-carboxamide

To a stirred solution of 5-(2-cyclopropoxy-5-(trifluoromethyl)phenyl)-*N*-((3*R*,5*S*)-5-(methoxymethyl) pyrrolidin-3-yl)oxazole-2-carboxamide TFA salt (0.12 g, 0.23 mmol) in THF (5 mL) was added K₂CO₃ (0.10 g, 0.69 mmol) at rt and the mixture was stirred for 10 min. Cyanogen bromide (0.02 g, 0.23 mmol) was added at 0 °C. The mixture was allowed to warm to rt and stirred at rt for 1 h, then poured into water (30 mL) and extracted with EtOAc (2 × 40 mL). The combined organic phases were dried over anhydrous Na₂SO₄, filtered and concentrated under reduced pressure. The residue was purified by flash column chromatography (silica gel, 58.6% EtOAc in n-hexanes) to yield *N*-((3*R*,5*S*)-1-cyano-5-(methoxymethyl)pyrrolidin-3-yl)-5-(2-cyclopropoxy-5-(trifluoromethyl)phenyl)oxazole-2-carboxamide (0.01 g, 0.03 mmol, 15% yield over two steps).

LCMS: Method H, 3.42 min, MS: ES+ 451.2; ¹H NMR (400 MHz, DMSO-*d₆*) δ ppm: 9.38 (d, *J* = 6.0 Hz, 1H), 8.05 (s, 1H), 7.86 (d, *J* = 8.4 Hz, 1H), 7.68 - 7.72 (m, 2H), 4.45 - 4.53 (m, 1H), 4.27 - 4.33 (m, 1H), 3.97 - 4.06 (m, 1H), 3.67 - 3.71 (m, 1H), 3.40 - 3.46 (m, 3H), 3.32 (s, 3H), 2.08 - 2.19 (m, 1H), 1.91 - 2.01 (m, 1H), 0.91 (br s, 4H); chiral HPLC: Method Y10, 7.84 min.

### Example 10

### N-((3R,5S)-1-Cyano-5-(methoxymethyl)pyrrolidin-3-yl)-5-(2-methoxy-5-(trifluoromethyl)phenyl)-1,3,4-oxadiazole-2-carboxamide

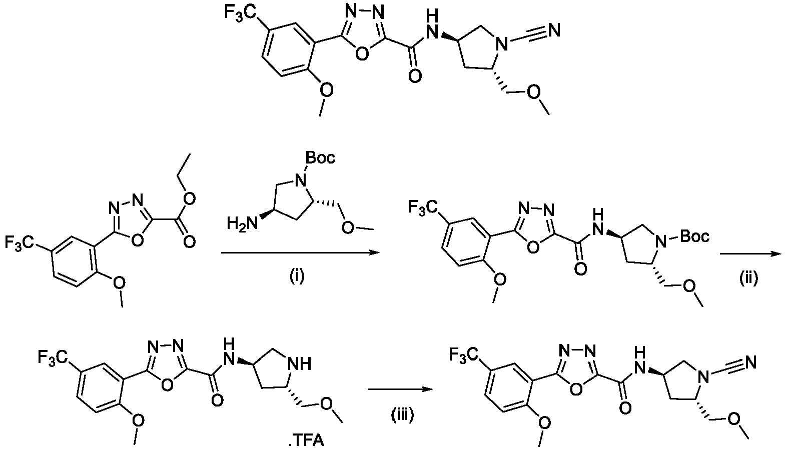

(i) TBD, THF, 0 °C to rt; (ii) TFA, DCM, 0 °C to rt; (iii) K₂CO₃, CNBr, THF, 0 °C to rt.

### Step (i)

### tert-Butyl (2S,4R)-4-(5-(2-methoxy-5-(trifluoromethyl)phenyl)-1,3,4-oxadiazole-2-carboxamido)-2-(methoxymethyl)pyrrolidine-1-carboxylate

To a stirred solution of ethyl 5-(2-methoxy-5-(trifluoromethyl)phenyl)-1,3,4-oxadiazole-2-carboxylate (0.28 g, 0.88 mmol) and *tert*-butyl (2*S*,4*R*)-4-amino-2-(methoxymethyl)pyrrolidine-1-carboxylate (CAS 1207853-53-9, 0.224 g, 0.97 mmol) in THF (2.8 mL) was added TBD (0.147 g, 0.106 mmol) at 0 °C. The mixture was warmed to rt and stirred for 1 h, then poured into water (25 mL) and extracted with EtOAc (3 × 100 mL). The combined organic phases were dried over anhydrous Na₂SO₄ and concentrated under reduced pressure. The residue was purified by flash column chromatography (silica gel, 40% EtOAc in *n*-hexanes) to yield *tert*-butyl (2*S*,4*R*)-4-(5-(2-methoxy-5-(trifluoromethyl) phenyl)-1,3,4-oxadiazole-2-carboxamido)-2-(methoxymethyl)pyrrolidine-1-carboxylate (0.19 g, 0.38 mmol, 42% yield). LCMS: Method J, 4.85 min, MS: ES+ 445.0 (M-56).

### Step (ii)

### 5-(2-Methoxy-5-(trifluoromethyl)phenyl)-N-((3R,5S)-5-(methoxymethyl)pyrrolidin-3-yl)-1,3,4-oxadiazole-2-carboxamide

To a stirred solution of *tert*-butyl (2*S*,4*R*)-4-(5-(2-methoxy-5-(trifluoromethyl)phenyl)-1,3,4-oxadiazole-2-carboxamido)-2-(methoxymethyl)pyrrolidine-1-carboxylate (0.19 g, 0.38 mmol) in DCM (1.9 mL) was added TFA (1.9 mL, 10 vol) dropwise at 0 °C. The mixture was allowed to warm to rt and stirred for 1 h, then concentrated under reduced pressure to yield 5-(2-methoxy-5-(trifluoromethyl)phenyl)-*N*-((3R,5*S*)-5-(methoxymethyl)pyrrolidin-3-yl)-1,3,4-oxadiazole-2-carboxamide TFA salt (0.17 g, 0.33 mmol, 87% yield). The crude was forwarded for next step.

### Step (iii)

### N-((3R,5S)-1-cyano-5-(methoxymethyl)pyrrolidin-3-yl)-5-(2-methoxy-5-(trifluoromethyl)phenyl)-1,3,4-oxadiazole-2-carboxamide

To a stirred solution of 5-(2-methoxy-5-(trifluoromethyl)phenyl)-*N*-3*R*,5*S*)-5-(methoxymethyl) pyrrolidin-3-yl)-1,3,4-oxadiazole-2-carboxamide TFA salt (0.17 g, 0.33 mmol) in THF (1.7 mL) was added K₂CO₃ (0.14 g, 0.99 mmol) at rt and the mixture was stirred for10 min. Cyanogen bromide (0.04 g, 0.39 mmol) was added at 0 °C and the mixture was then stirred at rt for 1 h. The mixture was poured into water (15 mL) and extracted with EtOAc (3 x 100 mL). The combined organic phases were dried over Na₂SO₄ and concentrated under reduced pressure. The residue was purified by flash column chromatography (silica gel, 70% EtOAc in n-hexanes) to yield *N*-((*3R,* 5*S*)-1-cyano-5-(methoxymethyl)pyrrolidin-3-yl)-5-(2-methoxy-5-(trifluoromethyl)phenyl)-1,3,4-oxadiazole-2-carboxamide (0.009 g, 0.02 mmol, 6% yield).

LCMS: Method H, 2.89 min, MS: ES+ 426.0; ¹H NMR (400 MHz, DMSO-*d₆*) δ ppm 9.79 (d, *J* = 6.4 Hz, 1H), 8.18 (s, 1H), 8.05 (d, *J* = 8.4 Hz, 1H), 7.53 (d, *J* = 8.8 Hz, 1H), 4.54 - 4.48 (m, 1H), 4.01 (m, 4H), 3.72 - 3.68 (m, 1H), 3.49 - 3.40 (m, 3H), 3.32 (s, 3H), 2.16 - 2.09 (m, 1H), 2.01 - 1.94 (m, 1H), chiral SFC: Method Y9, 4.9 min.

### Example 11

### 5-(2-Chloro-5-(trifluoromethyl)phenyl)-N-((3R,5S)-1-cyano-5-(methoxymethyl)pyrrolidin-3-yl)-1,3,4-oxadiazole-2-carboxamide

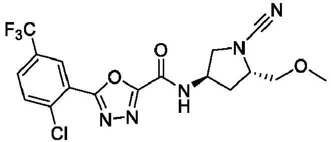

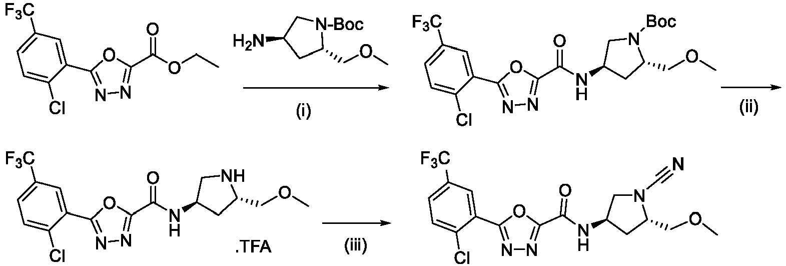

(i) TBD, THF, 0 °C to rt; (ii) TFA, DCM, 0 °C to rt; (iii) K₂CO₃, CNBr, THF, 0 °C to rt.

### Step (i)

### tert-Butyl (2S,4R)-4-(5-(2-chloro-5-(trifluoromethyl)phenyl)-1,3,4-oxadiazole-2-carboxamido)-2-(methoxymethyl)pyrrolidine-1-carboxylate

To a stirred solution of ethyl 5-(2-chloro-5-(trifluoromethyl)phenyl)-1,3,4-oxadiazole-2-carboxylate (0.30 g, 1.09 mmol) and *tert*-butyl (2*S*,4*R*)-4-amino-2-(methoxymethyl)pyrrolidine-1-carboxylate (CAS 1207853-53-9, 0.25 g, 1.09 mmol) in THF (5 mL) was added TBD (0.18 g, 1.31 mmol) at 0 °C. The mixture was allowed to warm to rt and stirred for 2.5 h, then poured into water (50 mL) and extracted with EtOAc (2 × 50 mL). The combined organic phases were dried over anhydrous Na₂SO₄, filtered and concentrated under reduced pressure. The residue was purified by flash column chromatography (silica gel, 37% EtOAc in *n*-hexanes) to yield *tert*-butyl (2*S*,4*R*)-4-(5-(2-chloro-5-(trifluoromethyl)phenyl)-1,3,4-oxadiazole-2-carboxamido)-2-(methoxymethyl)pyrrolidine-1-carboxylate (0.11 g, 0.22 mmol, 20% yield). LCMS: Method C1, 1.38 min, MS: ES- 503.3.

### Step (ii)

### 5-(2-Chloro-5-(trifluoromethyl)phenyl)-N-((3R,5S)-5-(methoxymethyl)pyrrolidin-3-yl)-1,3,4-oxadiazole-2-carboxamide

To a stirred solution of *tert*-butyl (2*S*,4*R*)-4-(5-(2-chloro-5-(trifluoromethyl)phenyl)-1,3,4-oxadiazole-2-carboxamido)-2-(methoxymethyl)pyrrolidine-1-carboxylate (0.11 g, 0.22 mmol) in DCM (5 mL) was added TFA (1.1 mL, 10 vol) dropwise at 0 °C. The mixture was allowed to warm to rt and stirred for 1 h, then concentrated under reduced pressure to yield 5-(2-chloro-5-(trifluoromethyl)phenyl)-*N*-((3*R*, 5*S*)-5-(methoxymethyl)pyrrolidin-3-yl)-1,3,4-oxadiazole-2-carboxamide TFA salt (0.12 g, quantitative yield). LCMS: Method C1, 1.06 min, MS: ES+ 405.3.

### Step (iii)

### 5-(2-Chloro-5-(trifluoromethyl)phenyl)-N-((3R,5S)-1-cyano-5-(methoxymethyl)pyrrolidin-3-yl)-1,3,4-oxadiazole-2-carboxamide

To a stirred solution of 5-(2-chloro-5-(trifluoromethyl)phenyl)-*N*-(3*R*,5*S*)-5-(methoxymethyl) pyrrolidin-3-yl)-1,3,4-oxadiazole-2-carboxamide TFA salt (0.12 g, 0.23 mmol) in THF (5 mL) was added K₂CO₃ (0.10 g, 0.69 mmol) at rt and the mixture was stirred for 10 min. Cyanogen bromide (0.02 g, 0.23 mmol) was added at 0 °C. The mixture was allowed to warm to rt and stirred for 0.5 h, then poured into water (30 mL) and extracted with EtOAc (2 x 30 mL). The combined organic phases were dried over Na₂SO₄ and concentrated under reduced pressure. The residue was purified by flash column chromatography (silica gel, 48% EtOAc in n-hexanes) to yield 5-(2-chloro-5-(trifluoromethyl)phenyl)-*N*-((3*R*, 5*S*)-1-cyano-5-(methoxymethyl)pyrrolidin-3-yl)-1,3,4-oxadiazole-2-carboxamide (0.02 g, 0.05 mmol, 21% yield over two steps).

LCMS: Method H, 3.10 min, MS: ES+ 447.1 (M + 18)⁺; ¹H NMR (400 MHz, DMSO-*d₆*) δ ppm: 9.86 (m, 1H), 8.34 (s, 1H), 8.01 - 8.10 (m, 2H), 4.53 (s, 1H), 4.03 (s, 1H), 3.71 - 3.72 (m, 1H), 3.43 - 3.46 (m, 3H), 3.34 (s, 3H), 2.12 - 2.13 (m, 1H), 1.99 - 2.00 (m, 1H), chiral SFC: Method Y4, 3.7 min.

### Example 12

### N-((3R,5R)-1-Cyano-5-(methoxymethyl)-pyrrolidin-3-yl)-5-(3-(trifluoromethyl)phenyl)oxazole-2-carboxamide

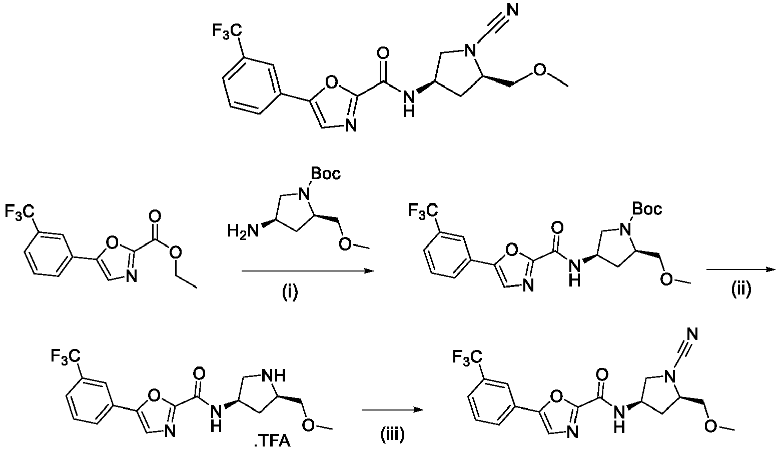

### Step (i)

### tert-Butyl (2R,4R)-2-(methoxymethyl)-4-(5-(3-(trifluoromethyl)phenyl)oxazole-2-carboxamido) pyrrolidine-1-carboxylate

To a stirred solution of ethyl 5-(3-(trifluoromethyl)phenyl)oxazole-2-carboxylate (0.2 g, 0.70 mmol) and *tert*-butyl (2*R*,4*R*)-4-amino-2-(methoxymethyl)pyrrolidine-1-carboxylate (CAS 1123305-98-5, 0.13 g, 0.56 mmol) in THF (5 mL) was added TBD (0.15 g, 1.05 mmol) in portions at 0 °C. The mixture was allowed to warm to rt and stirred for 1 h, then poured into water (50 mL) and extracted with EtOAc (2 × 50 mL). The combined organic phases were dried over anhydrous Na₂SO₄, filtered and concentrated under reduced pressure. The residue was purified by flash column chromatography (silica gel, 30% EtOAc in *n*-hexanes) to yield *tert*-butyl (2*R*,4*R*)-2-(methoxymethyl)-4-(5-(3-(trifluoromethyl)phenyl)oxazole-2-carboxamido)pyrrolidine-1-carboxylate (0.13 g, 0.27 mmol, 39% yield).

LCMS: Method C1, 1.42 min, MS: ES+ 470.2.

### Step (ii)

### N-((3R,5R)-5-(Methoxymethyl)pyrrolidin-3-yl)-5-(3-(trifluoromethyl)phenyl)oxazole-2-carboxamide

To a stirred solution of *tert*-butyl (2*R*,4*R*)-2-(methoxymethyl)-4-(5-(3-(trifluoromethyl)phenyl) oxazole-2-carboxamido)pyrrolidine-1-carboxylate (0.13 g, 0.27 mmol) in DCM (5 mL) was added TFA (0.39 mL, 3 vol) dropwise at 0 °C. The mixture was allowed to warm to rt and stirred for 1 h, then concentrated under reduced pressure to yield *N*-((3*R*,5*R*)-5-(methoxymethyl)pyrrolidin-3-yl)-5-(3-(trifluoromethyl)phenyl)oxazole-2-carboxamide TFA salt (0.12 g, 0.25 mmol, 89% yield).

LCMS: Method C1, 1.08 min, MS: ES+ 370.3.

### Step (iii)

### N-((3R, 5R)-1-Cyano-5-(methoxymethyl)pyrrolidin-3-yl)-5-(3-(trifluoromethyl)phenyl)oxazole-2-carboxamide

To a stirred solution of *N*-((3*R*,5*R*)-5-(methoxymethyl)pyrrolidin-3-yl)-5-(3-(trifluoromethyl)phenyl) oxazole-2-carboxamide TFA salt (0.12 g, 0.25 mmol) in THF (6 mL) was added K₂CO₃ (0.1 g, 0.74 mmol) at rt and stirred for 5 min. Cyanogen bromide (0.03 g, 0.25 mmol) was added at 0 °C. The mixture was stirred at rt for 1 h, then poured into water (50 mL) and extracted with EtOAc (2 × 50 mL). The combined organic phases were dried over Na₂SO₄ and concentrated under reduced pressure. The residue was purified by flash column chromatography (silica gel, 30% EtOAc in n-hexanes) to yield *N*-((3*R*,5*R*)-1-cyano-5-(methoxymethyl)pyrrolidin-3-yl)-5-(3-(trifluoromethyl)phenyl)oxazole-2-carboxamide (0.04 g, 0.10 mmol, 40% yield).

LCMS: Method H1, 3.12 min, MS: ES+ 395.0; ¹H NMR (400 MHz, DMSO-*d₆*) δ ppm: 9.20 (d, *J* = 7.6 Hz, 1H), 8.12 - 8.16 (m, 3H), 7.76 - 7.83 (m, 2H), 4.51 - 4.54 (m, 1H), 3.91 - 3.93 (m, 1H), 3.63 - 3.67 (m, 1H), 3.46 - 3.55 (m, 2H), 3.37 - 3.41 (m, 4H), 2.29 - 2.36 (m, 1H), 1.83 - 1.88 (m, 1H); Chiral SFC: Method Y13, 4.93 min.

### Example 13

### N-((3R,5S)-1-cyano-5-methylpyrrolidin-3-yl)-5-(3-(trifluoromethyl)phenyl)oxazole-2-carboxamide

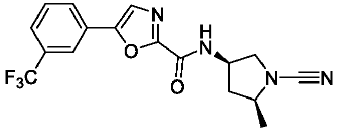

The title compound may be prepared by an analogous method to Example 2 using *tert*-butyl (2*S*,4*R*)-4-amino-2-methylpyrrolidine-1-carboxylate (CAS 708274-46-8) in Step (i).

### Example 14

### N-((3R,5R)-1-Cyano-5-(methoxymethyl)pyrrolidin-3-yl)-5-(2-cyclopropoxy-5-(trifluoromethyl)phenyl)-1,3,4-oxadiazole-2-carboxamide

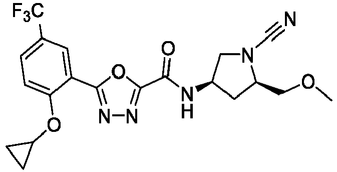

The title compound may be prepared by an analogous method to Example 5 using *tert*-butyl (2*R*,4*R*)-4-amino-2-(methoxymethyl)pyrrolidine-1-carboxylate (CAS 1123305-98-5) in Step (i).

### Example 15

### N-((3R,5S)-1-Cyano-5-methylpyrrolidin-3-yl)-5-(2-cyclopropoxy-5-(trifluoromethyl)phenyl)-1,3,4-oxadiazole-2-carboxamide

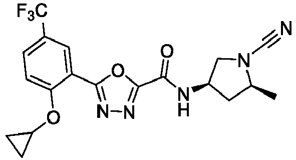

The title compound may be prepared by an analogous method to Example 6 using tert-butyl (2S,4R)-4-amino-2-methylpyrrolidine-1-carboxylate (CAS 708274-46-8) in Step (i).

### Example 16

### N-((3R,SS)-1-Cyano-5-methylpyrrolidin-3yl)-5-(3-(trifluoromethyl)phenyl)-1,3,4-oxadiazole-2-carboxamide

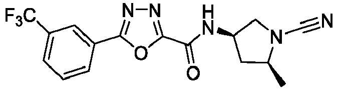

The title compound may be prepared by an analogous method to Example 7 using *tert*-butyl (2*S*,4*R*)-4-amino-2-methylpyrrolidine-1-carboxylate (CAS 708274-46-8) in Step (i).

### Example 17

### N-((3R,SR)-1-Cyano-5-(methoxymethyl)pyrrolidin-3-yl)-5-(3-(trifluoromethyl)phenyl)-1,3,4-oxadiazole-2-carboxamide

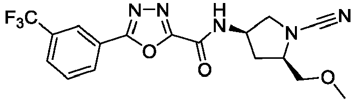

The title compound may be prepared by an analogous method to Example 8 using *tert*-butyl (*2R,4R*)-4-amino-2-(methoxymethyl)pyrrolidine-1-carboxylate (CAS 1123305-98-5) in Step (i).

### Example 18

### N-((3R,5R)-1-cyano-5-(methoxymethyl)pyrrolidin-3-yl)-5-(2-cyclopropoxy-5-(trifluoromethyl)phenyl)-oxazole-2-carboxamide

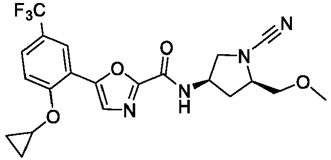

The title compound may be prepared by an analogous method to Example 9 using *tert*-butyl (*2R,4R*)-4-amino-2-(methoxymethyl)pyrrolidine-1-carboxylate (CAS 1123305-98-5) in Step (i).

### Example 19

### N-((3R,5R)-1-Cyano-5-(methoxymethyl)pyrrolidin-3-yl)-5-(2-methoxy-5-(trifluoromethyl)phenyl)-1,3,4-oxadiazole-2-carboxamide

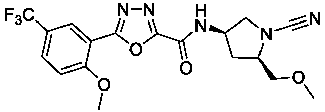

The title compound may be prepared by an analogous method to Example 10 using *tert*-butyl (2*R*,4*R*)-4-amino-2-(methoxymethyl)pyrrolidine-1-carboxylate (CAS 1123305-98-5) in Step (i).

### Example 20

### 5-(2-Chloro-5-(trifluoromethyl)phenyl)-N-((3R,5R)-1-cyano-5-(methoxymethyl)pyrrolidin-3-yl)-1,3,4-oxadiazole-2-carboxamide

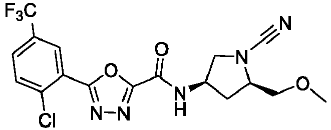

The title compound may be prepared by an analogous method to Example 11 using *tert*-butyl (2*R*,4*R*)-4-amino-2-(methoxymethyl)pyrrolidine-1-carboxylate (CAS 1123305-98-5) in Step (i).

### Biological Activity of Compounds of the Invention

### Abbreviations:

- TAMRA: carboxytetramethylrhodamine
- PCR: polymerase chain reaction
- PBS: phosphate buffered saline
- EDTA: ethylenediaminetetraacetic acid
- Tris: 2-amino-2-(hydroxymethyl)-1,3-propanediol
- NP-40: Nonidet P-40, octylphenoxypolyethoxyethanol
- BSA: bovine serum albumin
- PNS: peripheral nervous system
- BH3: Bcl-2 homology domain 3
- PTEN: phosphatase and tensin homologue
- SDS-PAGE: Sodium dodecyl sulfate polyacrylamide gel electrophoresis
- DMSO: Dimethyl sulfoxide
- YFP: Yellow fluorescent protein
- VME: Vinyl methyl ester
- HA: Hemagglutinin
- Ahx: Aminohexanoic acid

### USP30 biochemical IC₅₀ assay

Dilution plates were prepared at 21 times the final concentration (2100µM for a final concentration of 100µM) in 50% DMSO in a 96-well polypropylene V-bottom plate (Greiner #651201). A typical 8-point dilution series would be 100, 30, 10, 3, 1, 0.3, 0.1, 0.03µM final. Reactions were performed in duplicate in black 384 well plates (small volume, Greiner 784076) in a final reaction volume of 21 µl. Either 1µl of 50% DMSO or diluted compound was added to the plate. USP30 (Boston Biochem #E582) was diluted in reaction buffer (40mM Tris, pH 7.5, 0.005% Tween 20, 0.5mg/ml BSA, 5 mM beta-mercaptoethanol) to achieve a final assay concentration of 4 nM, and 10µl of diluted USP30 was added to the compound. Enzyme and compound were incubated for 30 min at room temp. Reactions were initiated by the addition of 50nM of TAMRA labelled peptide linked to ubiquitin via an iso-peptide bond as fluorescence polarisation substrate. Reactions were read immediately after addition of substrate and following a 2-hour incubation at room temperature. Readings were performed on a Pherastar Plus (BMG Labtech). λ Excitation 540 nm; λ Emission 590 nm.

Activity of exemplary compounds in USP30 biochemical IC₅₀ assay:

| **Example** | **IC₅₀ (nM)** | | **Example** | **IC₅₀ (nM)** | | **Example** | **IC₅₀ (nM)** |
|---|---|---|---|---|---|---|---|
| 1 | 9 | | 5 | 41 | | 9 | 4 |
| 2 | 8 | | 6 | 73 | | 10 | 41 |
| 3 | 3 | | 7 | 33 | | 11 | 22 |
| 4 | 4 | | 8 | 38 | | 12 | 15 |

### Reference Examples

Activity of exemplary compounds in USP30 biochemical IC₅₀ assay:

| **Reference Example** | **Structure** | **Origin** | **USP30 IC₅₀ (nM)** |
|---|---|---|---|
| A | 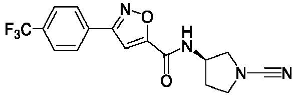 | WO 2016/156816 Example 219 | 120 |
| B | 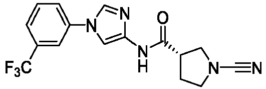 | WO 2016/046530 Example 154 | 300 |
| C | 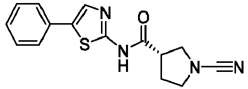 | WO 2016/046530 Example 1 | 310 |
| D | 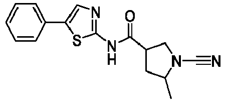 | WO 2016/046530 Example 88 | 4400 |

### Off-Target Pharmacology

Example 1 was subject to pharmacological profiling in the Eurofins CEREP SafetyScreen44 panel. At a single concentration of 10 µM, less than 50% inhibition of binding or enzyme activity was observed against all targets in the panel. Example 1 has a low probability for off-target interactions due to the low affinity for targets in this assay.

### TOM20-ubiquitylation assay

Human cell lines can be challenged with mitochondrial depolarizing agents (ionophores (eg. CCCP, valinomycin), mitochondrial complex inhibitors (oligomycin, antimycin A)) to induce ubiquitylation of TOM20, which is then further promoted in the presence of USP30 inhibitors. TOM20 ubiquitylation is subsequently assessed through western blotting of the cell lysates, with TOM20 ubiquitylation adduct detection possible due to an 8 kDa molecule weight increase for each molecule of ubiquitin added, resulting in laddering of a TOM20 immunoreactive band. TOM20-ubiquitylation levels can be quantified using chemiluminescence densitometry of laddered immunoreactive bands.

### In Vitro Cytotoxicity (Cell Tox):

Measured in HCT116 human colorectal carcinoma cells using alamarBlue^{™} as the assay endpoint. Compound cytotoxicity was measured over a period of 96-hour continual compound exposure.

### Further Studies

log P: partition coefficient; lipophilicity measurement.
log D: distribution co-efficient; lipophilicity measurement.
TPSA: topological polar surface area.
Turbidimetric solubility: Test compound solution prepared in DMSO diluted into aqueous buffer. Turbidimetry is used as the end-point by measuring absorbance at 620 nm.
FaSSIF: simulated intestinal fluid in fasted state measured at pH 6.5.
Hep Cl mouse: in vitro hepatocyte clearance in mouse cells.
Hep Cl human: in vitro hepatocyte clearance in human cells.
Plasma f_{u,p}: The free fraction of a compound in plasma preparation determined by in vitro equilibrium dialysis. It is understood that only unbound (free) compound is capable of engaging with the target. Brain f_{u,br}: The free fraction of a compound in brain homogenate preparation determined by in vitro equilibrium dialysis. It is understood that only unbound (free) compound is capable of engaging with the target.
Clᵤ: in vitro clearance. Clᵤ as defined here is the scaled clearance, in turn calculated from the intrinsic clearance. The intrinsic clearance is the predicted clearance due to hepatic metabolic reactions, determined from incubation of a compound in a hepatocyte preparation. The lower the value in mL/min/kg, the more stable the compound.
Cl in vivo clearance: Pharmacokinetic measurement of the volume of plasma (or any matrix) from which a substance is completely removed per unit time. The lower the value in mL/min/kg, the more stable the compound.
Oral F: Oral bioavailability.
MDR1-MDCK (Madin-Darby Canine Kidney cell monolayer) (in vitro) flux assay.
WT-MDCK (wild-type) in vitro flux.
Kpᵤᵤ is the ratio of unbound drug in brain to unbound drug in plasma and may be indicative of potential for treating peripheral and/or CNS indications.

| **Studies** | | **Example 1** |
|---|---|---|
| Cell Tox | HCT116 EC₅₀ (µM) | > 30 |
| Physicochemical | Log D measured at pH 7.4 | 2.6 |
| | TPSA | 92 |
| | Turbidimetric solubility (µM) | > 100 |
| | FaSSIF (µM) measured at pH 6.5 | 200 |
| Hepatocyte Cl mouse | Scaled Clᵤ (mL/min/kg) | 81 |
| Hepatocyte Cl human | Scaled Clᵤ (mL/min/kg) | 10.3 |
| Stability | Mouse plasma t_{½} (min) | 359 |
| MDR1-MDCK | Effective efflux ratio | 1.2 |
| WT-MDCK | Efflux Ratio (A-B (Pₐₚₚ flux (10⁻⁶ cm/s)) | 0.8 (11.8) |
| Binding Mouse | Plasma f_{u,p} / Brain f_{u,br} | 0.12 / 0.05 |
| PK mouse 2 mg/kg iv | Cl plasma (mL/min/kg) | 19 |
| PK mouse 10 mg/kg | Oral F (%) | 82 |
| TOM20-Ub 1.5-fold gain | Antimycin A/oligomycin mitophagy trigger EC1.5x (µM) | 0.020 |
| Unbound plasma Cmax/TOM20-Ub cell potency (10 mg/kg PO dose-mouse) | | 41-fold |
| Kpᵤᵤ | Brain microdialysis PK 30 mg/kg PO - mouse | 1.2 |
| Brain microdialysis PK 30 mg/kg PO mouse: pre-frontal cortex C_{max,u}/cell TOM20-Ub | | 23-fold |

The examples possess beneficial properties demonstrating potential superiority over other compounds. For instance, for Example 1 the observed IV plasma clearance of 19 mL/min/kg, as measured in the mouse, is low, demonstrating valuable plasma stability, and the compound has very high oral bioavailability of 82%.

| **Compound** | | **Ex. 1** | **Ex. 2** | **Ex. 8** | **Ex. 9** | **Ex. 12** |
|---|---|---|---|---|---|---|
| DUB IC₅₀ (µM) | USP30 | 0.009 | 0.008 | 0.038 | 0.004 | 0.015 |
| DUB IC₅₀ (µM) | USP2, USP6, USP10, USP16, USP21, USP22, USP25, USP28 | 16.0 - 97.9 | 17.2 - 107.9 | 4.6 - 78.9 | 2.9 - 135 | > 300 |
| DUB selectivity preference for USP30 v 8 DUBs | | > 1777 | > 2150 | > 121 | > 725 | > 20000 |

| **Compound** | | **Ex. 1** | **Ex. 2** | **Ex. 8** | **Ex. 9** | **Ex. 12** |
|---|---|---|---|---|---|---|
| Cathepsin IC₅₀ (µM) | Cathepsin B | 122.1 | 79.0 | 115.8 | > 300 | > 300 |
| | Cathepsin K | 10.2 | 11.2 | 11.9 | 9.9 | > 30 |
| | Cathepsin L | 30.4 | 7.9 | >30 | 18.2 | > 30 |
| | Cathepsin S | 137.7 | 146.2 | >30 | 164.1 | > 30 |
| | Cathepsin V | 105.3 | 56.0 | >30 | 100.7 | > 30 |
| | B, K, L, S, V | 10.2 - 137.7 | 7.9-146.2 | 11.9-115.8 | 9.9 - > 300 | > 30 |
| Selectivity preference for USP30 v cathepsins | | > 1133 | > 988 | > 313 | > 2475 | > 2000 |

| **Compound** | | **Ref. Ex. A** | **Ref. Ex. B** |
|---|---|---|---|
| DUB IC₅₀ (µM) | USP30 | 0.12 | 0.30 |
| DUB IC₅₀ (µM) | USP2, USP6, USP10, USP16, USP21, USP22, USP25, USP28 | 0.81 - 3.0 (exc. USP6) | 3.8 - 59.7 |
| DUB selectivity preference for USP30 v 7/8 DUBs | | 6.8 - 25 | 12.7 - 199 |

| **Compound** | | **Ref. Ex. A** | **Ref. Ex. B** |
|---|---|---|---|
| Cathepsin IC₅₀ (µM) | Cathepsin B | 0.85 | 4.6 |
| | Cathepsin K | 0.73 | 0.91 |
| | Cathepsin L | 2.1 | 1.1 |
| | Cathepsin S | > 30 | 6.4 |
| | Cathepsin V | 7.2 | 2.5 |
| | B, K, L, S, V | 0.73 - > 30 | 0.91 - 6.4 |
| Selectivity preference for USP30 v cathepsins | | 6.1 - 60 (Cat S > 250 | 3.0 - 21 |

| **Compound** | | **Exs. 1, 7, 8, 9, 11** | **Ex. 2** | **Ex. 12** | **Ref. Ex. B** | **Ref. Ex. C** | **Ref. Ex. D** |
|---|---|---|---|---|---|---|---|
| DUB IC₅₀ (µM) | USP30 | 0.004 - 0.033 | 0.008 | 0.015 | 0.30 | 0.31 | 4.4 |
| | UCHL1 | >300 | 280 | 149 | 0.15 | 0.25 | 6.8 |
| DUB selectivity preference for USP30 v UCHL1 | | > 9000 | 35000 | 9933 | 0.5 | 0.8 | 1.5 |

| **Compound** | | **Ex. 1** | **Ref. Ex. A** | **Ref. Ex. B** |
|---|---|---|---|---|
| Hep Cl mouse | scaled Clᵤ (mL/min/kg) | 81 | 118 | > 121 |
| Stability | Mouse plasma t½ (min) | 359 | > 120 | 10.4 |

### Comparative Data

Reference Examples A, B, C and D are known DUB inhibitors that have been identified as active as inhibitors of USP30 and share some structural similarity with the compounds of the present invention, possessing the cyanamide structural feature. Reference Examples B, C and D are disclosed in WO 2016/046530 as having UCHL1 inhibitory activity.

Example 1 shows significantly improved hepatocyte metabolic stability (as measured in mouse hepatocytes) compared to Reference Examples A and B.

Example 1 shows significantly improved plasma stability compared to Reference Example B.

### Potency for USP30

Examples 1 to 12 of the present invention are significantly more potent against USP30 than Reference Examples A, B, C and D, as measured in the biochemical assay. For instance, Examples 1, 2, 3, 4 and 9 are 13 to 40-fold more potent than Reference Example A, 33 to 100-fold more potent than Reference Examples B and C, and at least 490-fold more potent than Reference Example D.

### Selectivity for USP30 over USP2, USP6, USP10, USP16, USP21, USP22, USP25 and USP28

The data provided demonstrates that Examples 1, 2, 8, 9 and 12 are significantly more selective for USP30 over eight DUBs (USP2, USP6, USP10, USP16, USP21, USP22, USP25 and USP28) compared to Reference Example A. These examples are, at a minimum, between 121 and 20000-fold more potent against USP30 than against each of the eight DUBs. This is a significant selectivity advantage over Reference Examples A and B, which are as low as 6.5-fold and 12.7-fold more potent, respectively ('A' not tested against USP6).

### Selectivity for USP30 over UCHL1

The data provided demonstrates that Examples 1, 2, 7, 8, 9, 11 and 12 are significantly more selective for USP30 over UCHL1 compared to Reference Examples B, C and D. These examples are greater than 9000-fold more potent against USP30 than UCHL1, whereas Reference Examples B, C and D are only 0.5, 0.8 and 1.5-fold more potent, respectively; 'B' and 'C' are more selective for UCHL1.

### Selectivity for USP30 over cathepsins B, K, L, S and V

The data provided demonstrates that Examples 1, 2, 8, 9 and 12 are significantly more selective for USP30 over the cathepsins (B, K, L, S and V) compared to Reference Examples A and B. These examples are all greater than 313-fold more potent against USP30 than against each of the cathepsins. This is a significant selectivity advantage over Reference Examples A and B, which against cathepsin K are only 6.1 and 3-fold more potent, respectively.

The above-identified advantages of the compounds of the invention over the reference examples of the prior art are both significant and unexpected. On their own, and in particular in combination, this superiority makes the compounds of the invention particularly suitable for use in the treatment or prevention of diseases linked to USP30 activity.

### Preclinical in vivo models

Compounds of the invention may be tested for efficacy in representative in vivo disease models, using standard study procedures from the published literature, including, for example:
(a) Bleomycin-induced lung fibrosis model, which is a leading preclinical in vivo model of Idiopathic Pulmonary Fibrosis. [Kobayashi et al, 2016, J Immunol, 197(2):504-516].
(b) Diet-induced model of NAFLD and glucose homeostasis. [Nishida et al, 2013, Lab Invest; Feb;93(2):230-41].
(c) MPTP Model of Parkinson's Disease, which is a commonly used paradigm for looking at neurodegeneration in the dopaminergic system of the brain which is triggered by chemically-induced mitochondrial dysfunction. [Karuppagouner et al, 2014, Sci Rep. 2014 May 2;4:4874].
(d) Ndufs4KO Leigh syndrome model. [Kruse et al, 2008, Cell Metab. Apr;7(4):312-20].
(e) Aged rodent model: effects on hippocampal, cognitive and motor function. [Kobilo et al, 2014, Learn Mem. Jan 17;21(2): 119-26; Creed et al, 2019, Neuroscience. Jun 15;409: 169-179; Van Skike et al, 2020, Aging Cell. 19; e13057]. Example 1 was assessed in this model.
(f) The unilateral ureteral obstructive kidney disease model (UUO).

UUO causes renal injury characterised by tubular cell injury, interstitial inflammation and fibrosis. It serves as a model of irreversible post-renal acute kidney injury (AKI). Experimental UUO has illustrated the molecular mechanisms of apoptosis, inflammation and fibrosis, all of which are key processes in renal injury, regardless of the primary insult. Consequently, the UUO model provides investigators information beyond obstruction (Chevalier et al, 2009, Kidney Int 75(11): 1145-1152).

Example 1 was assessed in the UUO model to determine the ability of the compound to attenuate progressive tubulointerstitial fibrosis and chronic kidney disease (CKD).

On day 1 of the study, adult C57BL/6 mice were dosed by oral gavage according to one of the following dosing regimens; Vehicle or 15 mg/kg Example 1 (p.o.) BID. Two hours post dosing on day 1 study mice underwent surgery to ligate the left ureter at two points. Successful UUO surgery was later confirmed by observation of dilation of renal pelvis due to hydronephrosis. The animals were dosed according to their prescribed regimen for 10 days at which point kidneys were harvested, or histopathology assessment and for protein/RNA assessment. Picrosirius Red staining was performed to assess the extent of collagen deposition and IHC was employed to assess relative α-Smooth Muscle Actin (α-SMA) expression.

Results demonstrated that 15 mg/kg Example 1 (p.o.) dosed BID, statistically reduced collagen deposition as evidenced by reduced picrosirius red staining in ligated kidneys. Assessment of α-SMA staining, revealed that oral dosing of 15 mg/kg Example 1 BID resulted in a -statistical reduction in α-SMA levels in UUO injured kidneys when compared to vehicle treated controls.
(g) AKI can be induced by bilateral renal pedical clamping resulting in ischemia reperfusion injury (IRI) resulting in severe loss of renal function tubular damage and inflammation [Lu et al. 2012. J Nephrol. 25 (5): 738-45].

## Claims

1. A compound of formula (I), which is selected from formula (I)(i) and formula (I)(ii): 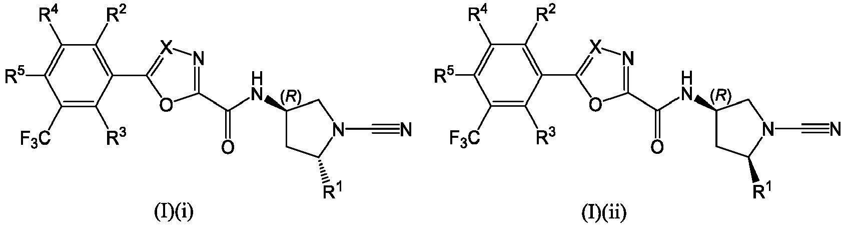 a tautomer thereof, or a pharmaceutically acceptable salt of said compound or tautomer, wherein:
X is CH or N;
R¹ is selected from (C₁-C₄)alkyl, (C₁-C₄)fluoroalkyl, CH₂OCH₃, CH₂SO₂CH₃ and CH₂-N-linked triazole;
R² is selected from hydrogen, halogen, (C₁-C₄)alkoxy and cyclopropoxy; and
R³, R⁴ and R⁵ are each independently selected from hydrogen and halogen.

2. The compound according to claim 1, wherein X is CH.

3. The compound according to claim 1, wherein X is N.

4. The compound according to any one of claims 1 to 3, wherein R¹ is selected from methyl, CH₂F, CHF₂, CF₃, CH₂OCH₃, CH₂SO₂CH₃ and CH₂-N-linked triazole.

5. The compound according to claim 4, wherein R¹ is CH₂OCH₃.

6. The compound according to any one of claims 1 to 5, wherein R² is selected from hydrogen, chlorine, fluorine, methoxy and cyclopropoxy.

7. The compound according to claim 6, wherein R² is hydrogen.

8. The compound according to any one of claims 1 to 7, wherein R³, R⁴ and R⁵ are each independently selected from hydrogen, chlorine and fluorine.

9. The compound according to claim 8, wherein R³, R⁴ and R⁵ are each hydrogen.

10. The compound according to any one of claims 1 to 9, having the formula (I)(i): 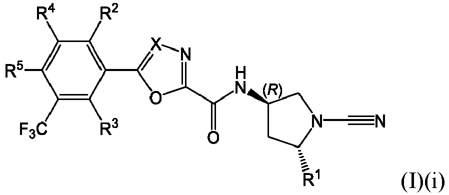 a tautomer thereof, or a pharmaceutically acceptable salt of said compound or tautomer.

11. The compound according to claim 10, which is selected from:
N-((3*R*,5*S*)-1-cyano-5-(methoxymethyl)-pyrrolidin-3-yl)-5-(3-(trifluoromethyl)phenyl)oxazole-2-carboxamide;
N-((3*R*,5*R*)-1-cyano-5-methylpyrrolidin-3-yl)-5-(3-(trifluoromethyl)phenyl)oxazole-2-carboxamide;
N-((3*R*,5*S*)-1-cyano-5-((methylsulfonyl)methyl)pyrrolidin-3-yl)-5-(3-(trifluoromethyl)phenyl)-oxazole-2-carboxamide;
N-((3*R*,5*S*)-5-((1H-1,2,3-triazol-1-yl)methyl)-1-cyanopyrrolidin-3-yl)-5-(3-(trifluoromethyl)phenyl) oxazole-2-carboxamide;
N-((3*R*,5*S*)-1-cyano-5-(methoxymethyl)pyrrolidin-3-yl)-5-(2-cyclopropoxy-5-(trifluoromethyl)phenyl)-1,3,4-oxadiazole-2-carboxamide;
N-((3*R*,5*R*)-1-cyano-5-methylpyrrolidin-3-yl)-5-(2-cyclopropoxy-5-(trifluoromethyl)phenyl)-1,3,4-oxadiazole-2-carboxamide;
N-((3*R*,5*R*)-1-cyano-5-methylpyrrolidin-3-yl)-5-(3-(trifluoromethyl)phenyl)-1,3,4-oxadiazole-2-carboxamide;
N-((3*R*,5*S*)-1-cyano-5-(methoxymethyl)pyrrolidin-3-yl)-5-(3-(trifluoromethyl)phenyl)-1,3,4-oxadiazole-2-carboxamide;
N-((3*R*,5*S*)-1-cyano-5-(methoxymethyl)pyrrolidin-3-yl)-5-(2-cyclopropoxy-5-(trifluoromethyl)phenyl)-oxazole-2-carboxamide;
N-((3*R*,5*S*)-1-cyano-5-(methoxymethyl)pyrrolidin-3-yl)-5-(2-methoxy-5-(trifluoromethyl)phenyl)-1,3,4-oxadiazole-2-carboxamide; and
5-(2-chloro-5-(trifluoromethyl)phenyl)-N-((3*R*,5*S*)-1-cyano-5-(methoxymethyl)pyrrolidin-3-yl)-1,3,4-oxadiazole-2-carboxamide;
or a pharmaceutically acceptable salt thereof.

12. The compound according to any one of claims 1 to 9, having the formula (I)(ii): 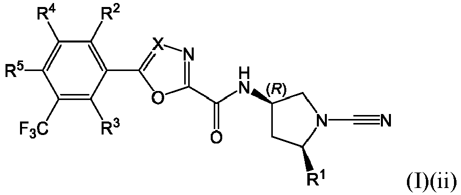 a tautomer thereof, or a pharmaceutically acceptable salt of said compound or tautomer.

13. The compound according to claim 12, which is selected from:
N-((3*R*,5*R*)-1-cyano-5-(methoxymethyl)-pyrrolidin-3-yl)-5-(3-(trifluoromethyl)phenyl)oxazole-2-carboxamide;
N-((3*R*,5*S*)-1-cyano-5-methylpyrrolidin-3-yl)-5-(3-(trifluoromethyl)phenyl)oxazole-2-carboxamide;
N-((3*R*,5*R*)-1-cyano-5-((methylsulfonyl)methyl)pyrrolidin-3-yl)-5-(3-(trifluoromethyl)phenyl)-oxazole-2-carboxamide;
N-((3*R*,5*R*)-5-((1H-1,2,3-triazol-1-yl)methyl)-1-cyanopyrrolidin-3-yl)-5-(3-(trifluoromethyl)phenyl) oxazole-2-carboxamide;
*N*-((3*R*,5*R*)-1-cyano-5 -(methoxymethyl)pyrrolidin-3 -yl)-5 -(2-cyclopropoxy-5 - (trifluoromethyl)phenyl)-1,3,4-oxadiazole-2-carboxamide;
N-((3*R*,5*S*)-1-cyano-5-methylpyrrolidin-3-yl)-5-(2-cyclopropoxy-5-(trifluoromethyl)phenyl)-1,3,4-oxadiazole-2-carboxamide;
N-((3*R*,5*S*)-1-cyano-5-methylpyrrolidin-3-yl)-5-(3-(trifluoromethyl)phenyl)-1,3,4-oxadiazole-2-carboxamide;
N-((3*R*,5*R*)-1-cyano-5-(methoxymethyl)pyrrolidin-3-yl)-5-(3-(trifluoromethyl)phenyl)-1,3,4-oxadiazole-2-carboxamide;
N-((3*R*,5*R*)-1-cyano-5 -(methoxymethyl)pyrrolidin-3 -yl)-5 -(2-cyclopropoxy-5 - (trifluoromethyl)phenyl) -oxazole -2-carboxamide;
N-((3*R*,5*R*)-1-cyano-5-(methoxymethyl)pyrrolidin-3-yl)-5-(2-methoxy-5-(trifluoromethyl)phenyl)-1,3,4-oxadiazole-2-carboxamide; and
5-(2-chloro-5-(trifluoromethyl)phenyl)-N-((3*R*,5*R*)-1-cyano-5-(methoxymethyl)pyrrolidin-3-yl)-1,3,4-oxadiazole-2-carboxamide;
or a pharmaceutically acceptable salt thereof.

14. A compound according to any one of claims 1 to 13, a tautomer thereof, or a pharmaceutically acceptable salt of said compound or tautomer, for use as a medicament.

15. A compound according to any one of claims 1 to 13, a tautomer thereof, or a pharmaceutically acceptable salt of said compound or tautomer, for use in the treatment or prevention of a condition involving mitochondrial dysfunction, a cancer, or fibrosis.

16. A compound for use according to claim 15,
wherein the condition involving mitochondrial dysfunction is selected from a CNS disorder; neurodegenerative disease; Parkinson's disease; Alzheimer's disease; amyotrophic lateral sclerosis; Huntington's disease; ischemia; stroke; dementia with Lewy bodies; frontotemporal dementia; multiple sclerosis; mitochondrial encephalopathy, lactic acidosis and stroke-like episodes syndrome; materially-inherited diabetes and deafness; Leber's hereditary optic neuropathy; neuropathy, ataxia, retinitis pigmentosa-maternally inherited Leigh syndrome; Danon disease; diabetes; diabetic nephropathy; metabolic disorders; heart failure; ischemic heart disease leading to myocardial infarction; psychiatric diseases, schizophrenia; multiple sulfatase deficiency; mucolipidosis II; mucolipidosis III; mucolipidosis IV; GMl-gangliosidosis; neuronal ceroid-lipofuscinoses; Alpers disease; Barth syndrome; beta-oxidation defects; carnitine-acyl-carnitine deficiency; carnitine deficiency; creatine deficiency syndromes; co-enzyme Q 10 deficiency; complex I deficiency; complex II deficiency; complex III deficiency; complex IV deficiency; complex V deficiency; COX deficiency; chronic progressive external ophthalmoplegia syndrome; CPT I deficiency; CPT II deficiency; glutaric aciduria type II; Kearns-Sayre syndrome; lactic acidosis; long-chain acyl-CoA dehydrogenase deficiency; Leigh disease or syndrome; X-linked Leigh's disease; Leigh Syndrome French Canadian variant; the symptoms associated with Leigh's disease; lethal infantile cardiomyopathy; Luft disease; medium-chain acyl-CoA dehydrogenase deficiency; myoclonic epilepsy and ragged-red fiber syndrome; mitochondrial cytopathy; mitochondrial recessive ataxia syndrome; mitochondrial DNA depletion syndrome; myoneurogastrointestinal disorder and encephalopathy; Pearson syndrome; pyruvate dehydrogenase deficiency; pyruvate carboxylase deficiency; POLG mutations; medium/short-chain 3-hydroxyacyl-CoA dehydrogenase deficiency; very long-chain acyl-CoA dehydrogenase deficiency; peroxisomal disorders; methylmalonic acidemia; mevalonate kinase deficiency; age-dependent decline in cognitive function and muscle strength; cognitive impairment associated with neurodegenerative and neuropsychiatric disorders; multiple system atrophy; progressive supranuclear palsy; corticobasal degeneration; Parkinson's disease related to mutations in α-synuclein, parkin, PINK1, GBA, and LRRK2; and autosomal recessive juvenile Parkinson's disease or early onset Parkinson's disease (EOPD), where parkin or PINK1 is mutated, truncated or deleted;
wherein the cancer is selected from breast, ovarian, prostate, lung, kidney, gastric, colon, testicular, head and neck, pancreas, brain, melanoma, bone, liver, soft tissue, cancers of tissue organs, cancers of the blood cells, CML, AML, mantle cell lymphoma, neuroblastoma, melanoma, soft tissue sarcoma, liposarcoma, fibroblastic sarcoma, leiomyosarcoma, hepatocellular carcinoma, osteosarcoma, oesophageal cancer, leukaemia, lymphoma, multiple myeloma, metastatic carcinoma, osteosarcoma, chondosarcoma, Ewing's sarcoma, nasopharyngeal carcinoma, colorectal cancer, colorectal cancer, non-small cell lung carcinoma, cancer where apoptotic pathways are dysregulated, and cancer where proteins of the BCL-2 family are mutated, or over or under expressed; and
wherein the fibrosis is selected from:
fibrosis or a fibrotic disorder associated with the accumulation of extracellular matrix constituents that occurs following trauma, inflammation, tissue repair, immunological reactions, cellular hyperplasia, and neoplasia;
fibrosis or a fibrotic disorder associated with major organ diseases, fibroproliferative disorders, and scarring associated with trauma;
fibrosis or a fibrotic disorder associated with interstitial lung disease, liver cirrhosis, non-alcoholic fatty liver disease, non-alcoholic steatohepatitis, kidney disease, acute kidney disease, acute kidney injury, chronic kidney disease, delayed kidney graft function, heart or vascular disease, diseases of the eye, systemic and local scleroderma, keloids, hypertrophic scars, atherosclerosis, restenosis, Dupuytren's contracture, surgical complications, chemotherapeutics drug-induced fibrosis, radiation-induced fibrosis, accidental injury and burns, retroperitoneal fibrosis, and peritoneal fibrosis/peritoneal scarring; and
fibrosis associated with interstitial lung disease selected from sarcoidosis, silicosis, drug reactions, infections, collagen vascular diseases, rheumatoid arthritis, systemic sclerosis, scleroderma, pulmonary fibrosis, idiopathic pulmonary fibrosis, usual interstitial pneumonitis, interstitial lung disease, cryptogenic fibrosing alveolitis, bronchiolitis obliterans, and bronchiectasis.

17. A pharmaceutical composition comprising a compound of formula (I) as defined in any one of claims 1 to 13, a tautomer thereof, or a pharmaceutically acceptable salt of said compound or tautomer, together with one or more pharmaceutically acceptable excipients.

18. A compound, which is selected from formulae (II)(i), (III)(i), (II)(i) and (III)(ii): 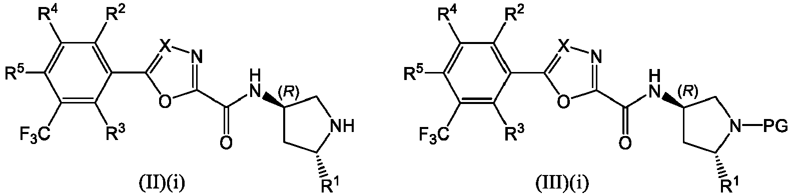 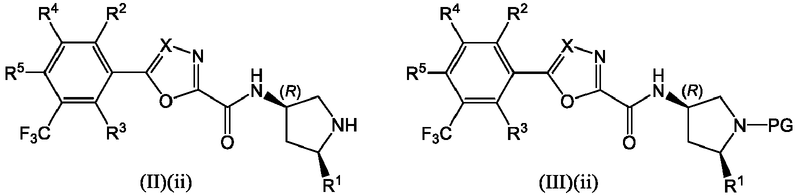
a tautomer thereof, or a salt of said compound or tautomer;
wherein R¹, R², R³, R⁴ and R⁵ are as defined for the compound of formula (I) in any one of claims 1 to 13; and PG is a protecting group.

19. The compound according to claim 18, wherein the protecting group is selected from tert-butyloxycarbonyl, benzyloxycarbonyl, p-methoxybenzyl carbonyl, 9-fluorenylmethyloxycarbonyl, acetyl, benzoyl, benzyl, carbamate, p-methoxybenzyl, 3,4-dimethoxybenzyl, p-methoxyphenyl, tosyl, trichloroethoxycarbonyl, 4-nitrobenzenesulfonyl and 2-nitrophenylsulfenyl.

## Patentansprüche

1. Verbindung aus Formel (I), die ausgewählt ist aus Formel (I)(i) und Formel (I)(ii): 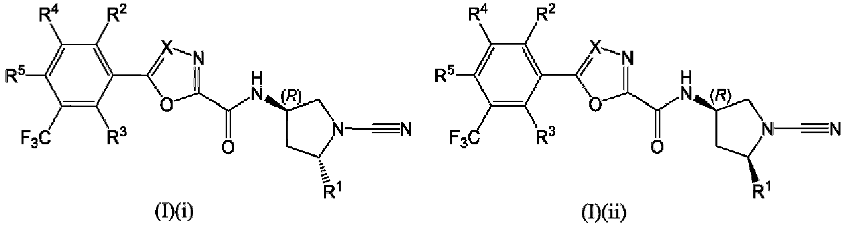 ein Tautomer daraus oder ein pharmazeutisch akzeptables Salz der Verbindung oder des Tautomers, wobei:
X CH oder N ist;
R¹ ausgewählt ist aus (C₁-C₄)Alkyl, (C₁-C₄)Fluoralkyl, CH₂OCH₃, CH₂SO₂CH₃ und CH₂-N-verknüpftem Triazol;
R² ausgewählt ist aus Wasserstoff, Halogen, (C₁-C₄)Alkoxy und Cyclopropoxy; und
R³, R⁴ und R⁵ jeweils unabhängig ausgewählt sind aus Wasserstoff und Halogen.

2. Verbindung nach Anspruch 1, wobei X CH ist.

3. Verbindung nach Anspruch 1, wobei X N ist.

4. Verbindung nach einem der Ansprüche 1 bis 3, wobei R¹ ausgewählt ist aus Methyl, CH₂F, CHF₂, CF₃, CH₂OCH₃, CH₂SO₂CH₃ und CH₂-N-verknüpftem Triazol.

5. Verbindung nach Anspruch 4, wobei R¹ CH₂OCH₃ ist.

6. Verbindung nach einem der Ansprüche 1 bis 5, wobei R² ausgewählt ist aus Wasserstoff, Chlor, Fluor, Methoxy und Cyclopropoxy.

7. Verbindung nach Anspruch 6, wobei R² Wasserstoff ist.

8. Verbindung nach einem der Ansprüche 1 bis 7, wobei R³, R⁴ und R⁵ jeweils unabhängig ausgewählt sind aus Wasserstoff, Chlor und Fluor.

9. Verbindung nach Anspruch 8, wobei R³, R⁴ und R⁵ jeweils Wasserstoff sind.

10. Verbindung nach einem der Ansprüche 1 bis 9, welche die Formel (I)(i) aufweist: 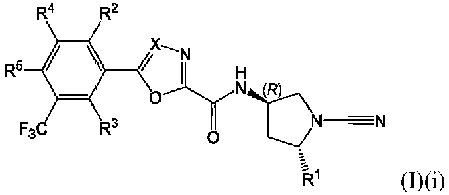 ein Tautomer daraus oder ein pharmazeutisch akzeptables Salz der Verbindung oder des Tautomers.

11. Verbindung nach Anspruch 10, die ausgewählt ist aus:
N-((3*R*,5*S*)-1-Cyano-5-(methoxymethyl)-pyrrolidin-3-yl)-5-(3-(trifluormethyl)phenyl)oxazol-2-carboxamid;
N-((3*R*,5*R*)-1-Cyano-5-methylpyrrolidin-3-yl)-5-(3-(trifluormethyl)phenyl)oxazol-2-carboxamid;
N-((3*R*,5*S*)-1-Cyano-5-((methylsulfonyl)methyl)pyrrolidin-3-yl)-5-(3-(trifluormethyl)phenyl)-oxazol-2-carboxamid;
N-((3*R*,5*S*)-5-((1H-1,2,3-Triazol-1-yl)methyl)-1-cyanopyrrolidin-3-yl)-5-(3-(trifluormethyl)phenyl) oxazol-2-carboxamid;
N-((3*R*,5*S*)-1-Cyano-5-(methoxymethyl)pyrrolidin-3-yl)-5-(2-cyclopropoxy-5-(trifluormethyl)phenyl)-1,3,4-oxadiazol-2-carboxamid;
N-((3*R*,5*R*)-1-Cyano-5-methylpyrrolidin-3-yl)-5-(2-cyclopropoxy-5-(trifluormethyl)phenyl)-1,3,4-oxadiazol-2-carboxam id;
N-((3*R*,5*R*)-1-Cyano-5-methylpyrrolidin-3-yl)-5-(3-(trifluormethyl)phenyl)-1,3,4-oxadiazol-2-carboxamid;
N-((3*R*,5*S*)-1-Cyano-5-(methoxymethyl)pyrrolidin-3-yl)-5-(3-(trifluormethyl)phenyl)-1,3,4-oxadiazol-2-carboxam id;
N-((3*R*,5*S*)-1-Cyano-5-(methoxymethyl)pyrrolidin-3-yl)-5-(2-cyclopropoxy-5-(trifluormethyl)phenyl)-oxazol-2-carboxamid;
N-((3*R*,5*S*)-1-Cyano-5-(methoxymethyl)pyrrolidin-3-yl)-5-(2-methoxy-5-(trifluormethyl)phenyl)-1,3,4-oxadiazol-2-carboxamid; und
5-(2-Chlor-5-(trifluormethyl)phenyl)-N-((3*R*,5*S*)-1-cyano-5-(methoxymethyl)pyrrolidin-3-yl)-1,3,4-oxadiazol-2-carboxam id;
oder ein pharmazeutisch akzeptables Salz daraus.

12. Verbindung nach einem der Ansprüche 1 bis 9, welche die Formel (I)(ii) aufweist: 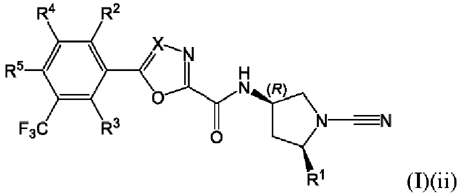 ein Tautomer daraus oder ein pharmazeutisch akzeptables Salz der Verbindung oder des Tautomers.

13. Verbindung nach Anspruch 12, die ausgewählt ist aus:
N-((3*R*,5*R*)-1-Cyano-5-(methoxymethyl)-pyrrolidin-3-yl)-5-(3-(trifluormethyl)phenyl)oxazol-2-carboxamid;
N-((3*R*,5*S*)-1-Cyano-5-methylpyrrolidin-3-yl)-5-(3-(trifluormethyl)phenyl)oxazol-2-carboxamid;
N-((3*R*,5*R*)-1-Cyano-5-((methylsulfonyl)methyl)pyrrolidin-3-yl)-5-(3-(trifluormethyl)phenyl)-oxazol-2-carboxamid;
N-((3*R*,5*R*)-5-((1H-1,2,3-Triazol-1-yl(methyl)-1-cyanopyrrolidin-3-yl)-5-(3-(trifluormethyl)phenyl) oxazol-2-carboxamid;
N-((3*R*,5*R*)-1-Cyano-5-(methoxymethyl)pyrrolidin-3-yl)-5-(2-cyclopropoxy-5-(trifluormethyl)phenyl)-1,3,4-oxadiazol-2-carboxamid;
N-((3*R*,5*S*)-1-Cyano-5-methylpyrrolidin-3-yl)-5-(2-cyclopropoxy-5-(trifluormethyl)phenyl)-1,3,4-oxadiazol-2-carboxam id;
N-((3*R*,5*S*)-1-Cyano-5-methylpyrrolidin-3-yl)-5-(3-(trifluormethyl)phenyl)-1,3,4-oxadiazol-2-carboxamid;
N-((3*R*,5*R*)-1-Cyano-5-(methoxymethyl)pyrrolidin-3-yl)-5-(3-(trifluormethyl)phenyl)-1,3,4-oxadiazol-2-carboxam id;
N-((3*R*,5*R*)-1-Cyano-5-(methoxymethyl)pyrrolidin-3-yl)-5-(2-cyclopropoxy-5-(trifluormethyl)phenyl)-oxazol-2-carboxamid;
N-((3*R*,5*R*)-1-Cyano-5-(methoxymethyl)pyrrolidin-3-yl)-5-(2-methoxy-5-(trifluormethyl)phenyl)-1,3,4-oxadiazol-2-carboxamid; und
5-(2-Chlor-5-(trifluormethyl) phenyl)-N-((*3R*,5*R*)-1-cyano-5-(methoxymethyl)pyrrolidin-3-yl)-1,3,4-oxadiazol-2-carboxam id;
oder ein pharmazeutisch akzeptables Salz daraus.

14. Verbindung nach einem der Ansprüche 1 bis 13, ein Tautomer daraus oder ein pharmazeutisch akzeptables Salz der Verbindung oder des Tautomers zur Verwendung als ein Medikament.

15. Verbindung nach einem der Ansprüche 1 bis 13, ein Tautomer daraus oder ein pharmazeutisch akzeptables Salz der Verbindung oder des Tautomers zur Verwendung in der Behandlung oder Prävention eines Leidens unter Beteiligung von mitochondrialer Dysfunktion, einer Krebserkrankung oder Fibrose.

16. Verbindung zur Verwendung nach Anspruch 15,
wobei das Leiden unter Beteiligung von mitochondrialer Dysfunktion ausgewählt ist aus: eine CNS-Erkrankung; neurodegenerative Krankheit; Parkinson-Krankheit; Alzheimer-Krankheit; amyotrophe laterale Sklerose; Huntington-Krankheit; Ischämie; Schlaganfall; Lewy-Körperchen-Demenz; frontotemporale Demenz; Multiple Sklerose; MELAS-Syndrom (mitochondriale Enzelopathie, Laktatazidose und schlaganfallähnliche Episoden); maternal vererbter Diabetes und Taubheit; Lebersche hereditäre Optikusneuropathie; maternal vererbtes Leigh-Syndrom (Neuropathie, Ataxie mit Retinitis pigmentosa); Danon-Krankheit; Diabetes; diabetische Nephropathie; Stoffwechselerkrankungen; Herzinsuffizienz; ischämische Herzkrankheit, die zu einem Myokardinfarkt führt; psychische Krankheiten; Schizophrenie; multipler Sulfatase-Mangel; Mucolipidose II; Mucolipidose III; Mucolipidose IV; GM1-Gangliosidose; neuronale Ceroid-Lipofuszinosen; Alpers-Krankheit; Barth-Syndrom; Beta-Oxidation-Defekte; Carnitin-Acylcarnitin-Mangel; Carnitin-Mangel; Kreatinmangelsyndrome; Coenzym Q10-Mangel; Komplex I-Mangel; Komplex II-Mangel; Komplex III-Mangel; Komplex IV-Mangel; Komplex V-Mangel, COX-Mangel; Syndrom der chronisch-progressiven externen Ophthalmoplegie; CPT I-Mangel; CPT II-Mangel; Glutarazidurie Typ 2; Kearns-Sayre-Syndrom; Laktatazidose; langkettiger-Acyl-CoA-Dehydrogenase-Mangel; Leigh-Krankheit oder -Syndrom; X-verknüpfte Leigh-Krankheit; Leigh-Syndrom, französisch-kanadischer Typ; mit der Leigh-Krankheit verbundene Symptome; letale infantile Kardiomyopathie; Luft-Syndrom; mittelkettiger-Acyl-CoA-Dehydrogenase-Mangel; Syndrom der myoklonischen Epilepsie mit rissigen roten Fasern; mitochondriale Cytopathie; mitochondriales rezessives Ataxie-Syndrom; mitochondriales DNA-Depletionssyndrom; myoneurogastrointestinale Erkrankung und Enzephalopathie; Pearson-Syndrom; Pyruvatdehydrogenase-Mangel; Pyruvatcarboxylase-Mangel; POLG-Mutationen; mittel-/kurzkettiger-3-Hydroxyacyl-CoA-Dehydrogenase-Mangel; sehr langkettiger-Acyl-CoA-Dehydrogenase-Mangel; peroxisomale Erkrankungen; methylmalonische Acidurie; Mevalonatkinase-Mangel; altersabhängige Abnahme kognitiver Funktionen und Muskelstärke; kognitive Beeinträchtigung in Verbindung mit neurodegenerativen und neuropsychiartrischen Erkrankungen; multiple Systematrophie; progressive supranukleäre Blickparese; kortikobasale Degeneration; Parkinson-Krankheit in Zusammenhang mit Mutationen im α-Synuclein, Parkin, PINK1, GBA und LRRK2; sowie autosomal-rezessive, juvenile Parkinson-Krankheit oder früh beginnende Parkinson-Krankheit (EOPD), bei der Parkin oder PINK1 mutiert, verkürzt oder gelöscht ist;
wobei die Krebserkrankung ausgewählt ist aus Brust, Eierstock, Prostata, Lungen, Nieren, Magen, Darm, Hoden, Kopf und Hals, Bauchspeicheldrüsen, Gehirn, Melanom, Knochen, Leber, Weichgewebe, Krebserkrankungen der Gewebsorgane, Krebserkrankungen der Blutzellen, CML, AML, Mantelzell-Lyphom, Neuroblastom, Melanom, Weichgewebesarkom, Liposarkom, fibroblastisches Sarkom, Leiomyosarkom, Leberzellkarzinom, Osteosarkom, Speiseröhrenkrebserkrankung, Leukämie, Lymphom, multiplem Myelom, metastasierendem Karzinom, Osteosarkom, Chondosarkom, Ewing-Sarkom, Nasopharynxkarzinom, kolorektalem Karzinom, nicht kleinzelligem Lungenkarzinom, Krebserkrankungen, bei denen apoptotische Signalwege dysreguliert sind, und Krebserkrankungen, bei denen Proteine der BCL-2-Familie mutiert oder über- oder unterexprimiert sind; und
wobei die Fibrose ausgewählt ist aus:
Fibrose oder eine fibrotische Erkrankung in Verbindung mit der Ansammlung extrazellulärer Matrixbestandteile, die als Folge von Trauma, Entzündung, Gewebereparatur, immunologischen Reaktionen, zellulärer Hyperplasie und Neoplasie auftritt;
Fibrose oder eine fibrotische Erkrankung in Verbindung mit wesentlichen Organkrankheiten, fibroproliferativen Erkrankungen und Vernarbung in Verbindung mit Trauma;
Fibrose oder eine fibrotische Erkrankung in Verbindung mit interstitieller Lungenkrankheit, Leberzirrhose, nicht alkoholische Fettleberkrankheit, nicht alkoholische Steatohepatitis, Nierenkrankheit, akute Nierenkrankheit, akute Nierenverletzung, chronische Nierenkrankheit, verzögerte Nierentransplantatfunktion, Herz- oder Gefäßkrankheit, Krankheiten des Auges, systemische und lokale Sklerodermie, Keloide, hypertrophe Narben, Arteriosklerose, Restenose, Dupuytren-Kontraktur, chirurgische Komplikationen, durch Chemotherapeutika induzierte Fibrose, strahlungsinduzierte Fibrose, unfallbedingte Verletzung und Verbrennungen, retroperitoneale Fibrose und peritoneale Fibrose/peritoneale Vernarbung; und
Fibrose in Verbindung mit interstitieller Lungenkrankheit ausgewählt aus Sarkoidose, Silikose, Arzneimittelreaktionen, Infektionen, Kollagen-Gefäßkrankheiten, rheumatoide Arthritis, systemische Sklerose, Sklerodermie, pulmonäre Fibrose, idiopathische Lungenfibrose, gewöhnliche interstitielle Pneumonie, interstitielle Lungenkrankheit, kryptogenetische fibrosierende Alveolitis, Bronchiolitis obliterans und Bronchiektasie.

17. Pharmazeutische Zusammensetzung, umfassend eine Verbindung aus Formel (I), wie in einem der Ansprüche 1 bis 13 definiert, ein Tautomer daraus oder ein pharmazeutisch akzeptables Salz der Verbindung oder des Tautomers, zusammen mit einem oder mehreren pharmazeutisch akzeptablen Hilfsstoffen.

18. Verbindung, die ausgewählt ist aus den Formeln (II)(i), (III)(i), (II)(i) und (III)(ii): 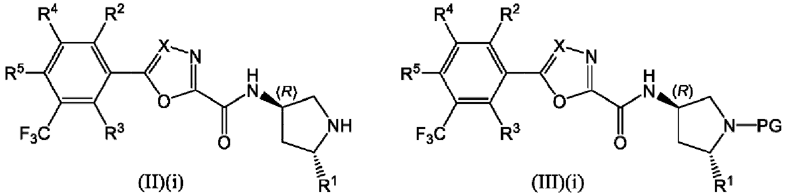 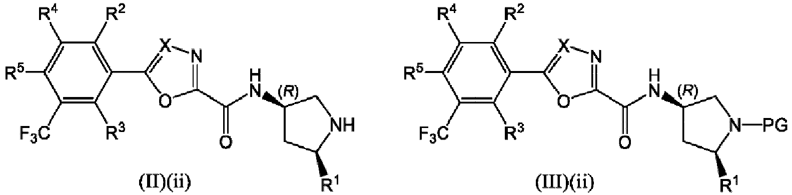
ein Tautomer daraus oder ein Salz der Verbindung oder des Tautomers;
wobei R¹, R², R³, R⁴ und R⁵ der Definition für die Verbindung von Formel (I) in einem der Ansprüche 1 bis 13 entsprechen; und PG eine Schutzgruppe ist.

19. Verbindung nach Anspruch 18, wobei die Schutzgruppe ausgewählt ist aus Tert-butyloxycarbonyl, Benzyloxycarbonyl, p-Methoxybenzylcarbonyl, 9-Fluorenylmethyloxycarbonyl, Acetyl, Benzoyl, Benzyl, Carbamat, p-Methoxybenzyl, 3,4-Dimethoxybenzyl, p-Methoxyphenyl, Tosyl, Trichlorethoxycarbonyl, 4-Nitrobenzensulfonyl und 2-Nitrophenylsulfenyl.

## Revendications

1. Composé de formule (I), qui est choisi parmi la formule (I)(i) et la formule (I)(ii) : 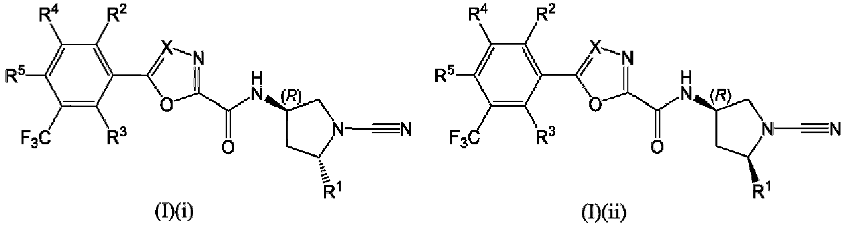 un tautomère de celui-ci, ou un sel pharmaceutiquement acceptable dudit composé ou tautomère, dans lequel :
X est CH ou N ;
R¹ est choisi parmi (C₁-C₄)alkyle, (C₁-C₄)fluoroalkyle, CH₂OCH₃, CH₂SO₂CH₃ et CH₂-triazole N-lié ;
R² est choisi parmi hydrogène, halogène, (C₁-C₄)alcoxy et cyclopropoxy ; et
R³, R⁴ et R⁵ sont chacun indépendamment choisis parmi hydrogène et halogène.

2. Composé selon la revendication 1, dans lequel X est CH.

3. Composé selon la revendication 1, dans lequel X est N.

4. Composé selon l'une quelconque des revendications 1 à 3, dans lequel R¹ est choisi parmi méthyle, CH₂F, CHF₂, CF₃, CH₂OCH₃, CH₂SO₂CH₃ et CH₂-triazole N-lié.

5. Composé selon la revendication 4, dans lequel R¹ est CH₂OCH₃.

6. Composé selon l'une quelconque des revendications 1 à 5, dans lequel R² est choisi parmi hydrogène, chlore, fluor, méthoxy et cyclopropoxy.

7. Composé selon la revendication 6, dans lequel R² est hydrogène.

8. Composé selon l'une quelconque des revendications 1 à 7, dans lequel R³, R⁴ et R⁵ sont chacun indépendamment choisis parmi hydrogène, chlore et fluor.

9. Composé selon la revendication 8, dans lequel R³, R⁴ et R⁵ sont chacun hydrogène.

10. Composé selon l'une quelconque des revendications 1 à 9, ayant la formule (I)(i) : 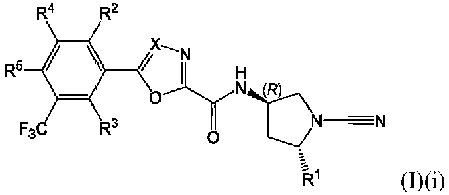 un tautomère de celui-ci, ou un sel pharmaceutiquement acceptable dudit composé ou tautomère.

11. Composé selon la revendication 10, qui est choisi parmi :
N-((3*R*,5*S*)-1-cyano-5-(méthoxyméthyl)-pyrrolidin-3-yl)-5-(3-(trifluorométhyl)phényl)oxazole-2-carboxamide ;
N-((3*R*,5*R*)-1-cyano-5-méthylpyrrolidin-3-yl)-5-(3-(trifluorométhyl)phényl)oxazole-2-carboxamide ;
N-((3*R*,5*S*)-1-cyano-5-((méthylsulfonyl)méthyl)pyrrolidin-3-yl)-5-(3-(trifluorométhyl)phényl)-oxazole-2-carboxamide ;
N-((3*R*,5*S*)-5-((1H-1,2,3-triazol-1-yl)méthyl)-1-cyanopyrrolidin-3-yl)-5-(3-(trifluorométhyl)phényl)oxazole-2-carboxamide ;
N-((3*R*,5*S*)-1-cyano-5-(méthoxyméthyl)pyrrolidin-3-yl)-5-(2-cyclopropoxy-5-(trifluorométhyl)phényl)-1,3,4-oxadiazole-2-carboxamide ;
N-((3*R*,5*R*)-1-cyano-5-méthylpyrrolidin-3-yl)-5-(2-cyclopropoxy-5-(trifluorométhyl)phényl)-1,3,4-oxadiazole-2-carboxamide ;
N-((3*R*,5*R*)-1-cyano-5-méthylpyrrolidin-3-yl)-5-(3-(trifluorométhyl)phényl)-1,3,4-oxadiazole-2-carboxamide ;
N-((3*R*,5*S*)-1-cyano-5-(méthoxyméthyl)pyrrolidin-3-yl)-5-(3-(trifluorométhyl)phényl)-1,3,4-oxadiazole-2-carboxamide ;
N-((3*R*,5*S*)-1-cyano-5-(méthoxyméthyl)pyrrolidin-3-yl)-5-(2-cyclopropoxy-5-(trifluorométhyl)phényl)-oxazole-2-carboxamide ;
N-((3*R*,5*S*)-1-cyano-5-(méthoxyméthyl)pyrrolidin-3-yl)-5-(2-méthoxy-5-(trifluorométhyl)phényl)-1,3,4-oxadiazole-2-carboxamide ; et
5-(2-chloro-5-(trifluorométhyl)phényl)-N-((*3R*,*5S*)-1-cyano-5-(méthoxyméthyl)pyrrolidin-3-yl)-1,3,4-oxadiazole-2-carboxamide ;
ou un sel pharmaceutiquement acceptable de celui-ci.

12. Composé selon l'une quelconque des revendications 1 à 9, ayant la formule (I)(ii) : 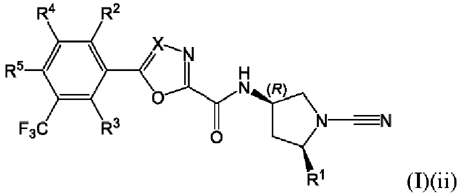 un tautomère de celui-ci, ou un sel pharmaceutiquement acceptable dudit composé ou tautomère.

13. Composé selon la revendication 12, qui est choisi parmi :
N-((3*R*,5*R*)-1-cyano-5-(méthoxyméthyl)-pyrrolidin-3-yl)-5-(3-(trifluorométhyl)phényl)oxazole-2-carboxamide ;
N-((3*R*,5*S*)-1-cyano-5-méthylpyrrolidin-3-yl)-5-(3-(trifluorométhyl)phényl)oxazole-2-carboxamide ;
N-((3*R*,5*R*)-1-cyano-5-((méthylsulfonyl)méthyl)pyrrolidin-3-yl)-5-(3-(trifluorométhyl)phényl)-oxazole-2-carboxamide ;
N-((3*R*,5*R*)-5-((1H-1,2,3-triazol-1-yl)méthyl)-1-cyanopyrrolidin-3-yl)-5-(3-(trifluorométhyl)phényl)oxazole-2-carboxamide ;
N-((3*R*,5*R*)-1-cyano-5-(méthoxyméthyl)pyrrolidin-3-yl)-5-(2-cyclopropoxy-5-(trifluorométhyl)phényl)-1 ,3,4-oxadiazole-2-carboxamide ;
N-((3*R*,5*S*)-1-cyano-5-méthylpyrrolidin-3-yl)-5-(2-cyclopropoxy-5-(trifluorométhyl)phényl)-1,3,4-oxadiazole-2-carboxamide ;
N-((3*R*,5*S*)-1-cyano-5-méthylpyrrolidin-3-yl)-5-(3-(trifluorométhyl)phényl)-1,3,4-oxadiazole-2-carboxamide ;
N-((3*R*,5*R*)-1-cyano-5-(méthoxyméthyl)pyrrolidin-3-yl)-5-(3-(trifluorométhyl)phényl)-1,3,4-oxadiazole-2-carboxamide ;
N-((3*R*,5*R*)-1-cyano-5-(méthoxyméthyl)pyrrolidin-3-yl)-5-(2-cyclopropoxy-5-(trifluorométhyl)phényl)-oxazole-2-carboxamide ;
N-((3*R*,5*R*)-1-cyano-5-(méthoxyméthyl)pyrrolidin-3-yl)-5-(2-méthoxy-5-(trifluorométhyl)phényl)-1,3,4-oxadiazole-2-carboxamide ; et
5-(2-chloro-5-(trifluorométhyl)phényl)-N-((*3R*,*5R*)-1-cyano-5-(méthoxyméthyl)pyrrolidin-3-yl)-1,3,4-oxadiazole-2-carboxamide ;
ou un sel pharmaceutiquement acceptable de celui-ci.

14. Composé selon l'une quelconque des revendications 1 à 13, un tautomère de celui-ci ou un sel pharmaceutiquement acceptable dudit composé ou tautomère, destiné à être utilisé en tant médicament.

15. Composé selon l'une quelconque des revendications 1 à 13, un tautomère de celui-ci ou un sel pharmaceutiquement acceptable dudit composé ou tautomère, destiné à être utilisé dans le traitement ou la prévention d'un état impliquant un dysfonctionnement mitochondrial, un cancer ou une fibrose.

16. Composé destiné à une utilisation selon la revendication 15,
dans lequel l'état impliquant un dysfonctionnement mitochondrial est choisi parmi un trouble du SNC ; une maladie neurodégénérative ; la maladie de Parkinson ; la maladie d'Alzheimer ; la sclérose latérale amyotrophique ; la maladie de Huntington ; l'ischémie ; un accident vasculaire cérébral ; la démence à corps de Lewy ; la démence frontotemporale ; la sclérose en plaques ; l'encéphalopathie mitochondriale, l'acidose lactique et le syndrome de pseudo-épisodes vasculaires cérébraux ; le diabète et la surdité héréditaires ; la neuropathie optique héréditaire de Leber ; la neuropathie, l'ataxie, la rétinite pigmentaire-syndrome de Leigh à hérédité maternelle ; la maladie de Danon ; le diabète ; la néphropathie diabétique ; les troubles métaboliques ; l'insuffisance cardiaque ; la cardiopathie ischémique conduisant à un infarctus du myocarde ; les maladies psychiatriques, la schizophrénie ; le déficit multiple en sulfatases ; la mucolipidose II ; la mucolipidose III ; la mucolipidose IV ; la gangliosidose à GM1 ; les céroïdes-lipofuscinoses neuronales ; la maladie d'Alpers ; le syndrome de Barth ; les défauts de la bêta-oxydation ; le déficit en carnitine-acyl-carnitine ; le déficit en carnitine ; les syndromes de carence en créatine ; le déficit en coenzyme Q10 ; le déficit en complexe I ; le déficit en complexe II ; le déficit en complexe III ; le déficit en complexe IV ; le déficit en complexe V ; le déficit en COX ; le syndrome d'ophtalmoplégie externe chronique progressive ; le déficit en CPT I ; le déficit en CPT II ; l'acidurie glutarique de type II ; le syndrome de Kearns-Sayre ; l'acidose lactique ; le déficit en acyl-CoA déshydrogénase à longue chaîne ; la maladie ou le syndrome de Leigh ; la maladie de Leigh liée à l'X; la variante franco-canadienne du syndrome de Leigh ; les symptômes associés à la maladie de Leigh ; la cardiomyopathie infantile mortelle ; la maladie de Luft ; le déficit en acyl-CoA déshydrogénase à chaîne moyenne ; l'épilepsie myoclonique et le syndrome des fibres rouges irrégulières ; la cytopathie mitochondriale ; le syndrome d'ataxie mitochondriale récessive ; le syndrome de déplétion de l'ADN mitochondrial ; les troubles myoneurogastro-intestinaux et l'encéphalopathie ; le syndrome de Pearson ; le déficit en pyruvate déshydrogénase ; le déficit en pyruvate carboxylase ; les mutations POLG ; le déficit en 3-hydroxyacyl-CoA déshydrogénase à chaîne moyenne/courte ; le déficit en acyl-CoA déshydrogénase à très longue chaîne ; les troubles peroxysomaux ; l'acidémie méthylmalonique ; le déficit en mévalonate kinase ; le déclin de la fonction cognitive et de la force musculaire lié à l'âge ; les troubles cognitifs associés à des troubles neurodégénératifs et neuropsychiatriques ; l'atrophie multisystémique ; la paralysie supranucléaire progressive ; la dégénérescence corticobasale ; la maladie de Parkinson liée à des mutations de α-synucléine, la parkine, PINK1, GBA et LRRK2 ; et la maladie de Parkinson juvénile autosomique récessive ou la maladie de Parkinson à apparition précoce (EOPD), où la parkine ou PINK1 est mutée, tronquée ou supprimée ;
dans lequel le cancer est choisi parmi le cancer du sein, de l'ovaire, de la prostate, du poumon, des reins, de l'estomac, du côlon, des testicules, de la tête et du cou, du pancréas, du cerveau, un mélanome, le cancer des os, du foie, des tissus mous, les cancers des organes tissulaires, les cancers des cellules sanguines, la LMC, la LMA, le lymphome à cellules du manteau, un neuroblastome, un mélanome, un sarcome des tissus mous, un liposarcome, un sarcome fibroblastique, un léiomyosarcome, un carcinome hépatocellulaire, un ostéosarcome, le cancer de l'oesophage, la leucémie, un lymphome, un myélome multiple, un carcinome métastatique, un ostéosarcome, un chondosarcome, un sarcome d'Ewing, un carcinome du nasopharynx, le cancer colorectal, le cancer colorectal, un carcinome pulmonaire non à petites cellules, un cancer dans lequel les voies apoptotiques sont dérégulées et un cancer dans lequel les protéines de la famille BCL-2 sont mutées, ou sur ou sous-exprimées ; et
dans lequel la fibrose est choisie parmi :
fibrose ou un trouble fibrotique associé à l'accumulation de constituants de la matrice extracellulaire qui survient à la suite d'un traumatisme, d'une inflammation, d'une réparation tissulaire, de réactions immunologiques, d'une hyperplasie cellulaire et d'une néoplasie ;
fibrose ou un trouble fibrotique associé à des maladies organiques majeures, des troubles fibroprolifératifs et des cicatrices associées à un traumatisme ;
fibrose ou un trouble fibrotique associé à une maladie pulmonaire interstitielle, une cirrhose du foie, une stéatose hépatique non alcoolique, une stéatohépatite non alcoolique, une maladie rénale, une maladie rénale aiguë, une lésion rénale aiguë, une maladie rénale chronique, un retard de reprise de fonction du greffon rénal, une maladie cardiaque ou vasculaire, des maladies de l'œil, une sclérodermie systémique et locale, des chéloïdes, des cicatrices hypertrophiques, l'athérosclérose, la resténose, la maladie de Dupuytren, les complications chirurgicales, la fibrose induite par les médicaments chimiothérapeutiques, la fibrose radio-induite, les blessures et brûlures accidentelles, la fibrose rétropéritonéale et la fibrose péritonéale/les cicatrices péritonéales ; et
fibrose associée à une maladie pulmonaire interstitielle choisie parmi la sarcoïdose, la silicose, les réactions médicamenteuses, les infections, les maladies vasculaires du collagène, la polyarthrite rhumatoïde, la sclérose systémique, la sclérodermie, la fibrose pulmonaire, la fibrose pulmonaire idiopathique, la pneumopathie interstitielle habituelle, la maladie pulmonaire interstitielle, l'alvéolite fibrosante cryptogénique, la bronchiolite oblitérante et la bronchectasie.

17. Composition pharmaceutique comprenant un composé de formule (I) tel que défini dans l'une quelconque des revendications 1 à 13, un tautomère de celui-ci, ou un sel pharmaceutiquement acceptable dudit composé ou tautomère, conjointement avec un ou plusieurs excipients pharmaceutiquement acceptables.

18. Composé, qui est choisi parmi les formules (II)(i), (III)(i), (II)(i) et (III)(ii) : 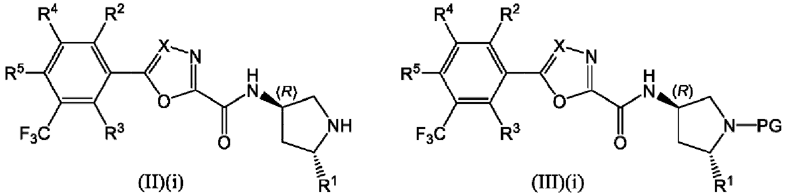 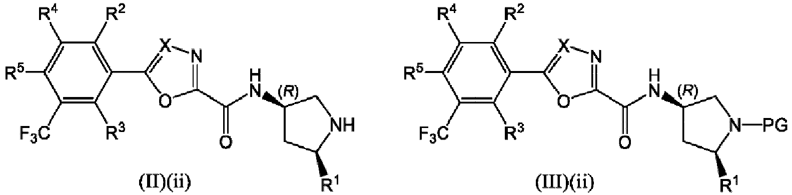
un tautomère de celui-ci, ou un sel dudit composé ou tautomère ;
dans lequel R¹, R², R³, R⁴ et R⁵ sont tels que définis pour le composé de formule (I) dans l'une quelconque des revendications 1 à 13 ; et PG est un groupe protecteur.

19. Composé selon la revendication 18, dans lequel le groupe protecteur est choisi parmi tert-butyloxycarbonyle, benzyloxycarbonyle, p-méthoxybenzylcarbonyle, 9-fluorénylméthyloxycarbonyle, acétyle, benzoyle, benzyle, carbamate, p-méthoxybenzyle, 3,4-diméthoxybenzyle, p-méthoxyphényle, tosyle, trichloroéthoxycarbonyle, 4-nitrobenzènesulfonyle et 2-nitrophénylsulfényle.
